# EUROPEAN PATENT APPLICATION

(11) **EP 4 046 996 A1**
(43) Date of publication of application: **24.08.2022**
(21) Application number: 21158130.1
(22) Date of filing: 19.02.2021
(51) Int. Cl.: C07D 413/06, C07D 417/14, A61P 35/00, A61K 31/395

(54) **CRYPTOPHYCIN COMPOUNDS AND CONJUGATES THEREOF**

(71) Applicant: Universität Bielefeld, 33615 Bielefeld (DE)
(72) Inventor: DESSIN, Cedric, 33790 Halle (DE); SCHACHTSIEK, Thomas, 33602 Bielefeld (DE); BLANCHARD NERIN, Guillermo, 50011 Zaragoza (ES); SEWALD, Norbert, 33739 Bielefeld (DE)
(74) Representative: Viering, Jentschura & Partner mbB Patent- und Rechtsanwälte

(57) **Abstract**

The present invention relates to cryptophycin compounds, to new cryptophycin payloads, to new cryptophycin conjugates, to compositions containing them and to their therapeutic use, especially as anticancer agents.

## Description

The present invention relates to cryptophycin compounds, to new cryptophycin payloads, to new cryptophycin conjugates, to compositions containing them and to their therapeutic use, especially as anticancer agents.

Cryptophycins are naturally occurring cyclic depsipeptides that were first isolated as secondary metabolites from cyanobacteria. They target tubulin and block the microtubule formation, leading to high cytotoxicity against many cancer cell lines. Moreover, as they are a weak target for the P-gp efflux pump, the cytotoxicity is only slightly reduced in multidrug-resistant (MDR) cancer cells. Due to these characteristics, several cryptophycin analogues were investigated as chemotherapeutics and cryptophycin-52 was even brought to the clinics. However, these were discontinued in phase II because of side effects and insufficient efficacy (27,28). Subsequent research focused on several structure-activity relationship studies with special emphasis on the introduction of a functional group, enabling the conjugation to a homing device for targeted tumor therapy (34-40).

Certain cryptophycin derivatives were developed as payloads in the ADC (antibody-drug conjugate) field (41-43). In particular, cryptophycin that is modified in the para position of the phenyl ring in unit A has been used in this context, as described for example in international patent publication WO 2011/001052 A1. However, the use of these conjugates in preclinical development of new ADCs was hampered by their instability in murine plasma. Stability problems in the macrocycle could be subsequently overcome by applying modifications in the payload, as reported in WO 2017/076998 A1, or changing the antibody anchoring point (Su et al., Bioconj Chem 2018, 29, 1155-1167).

To date, there are only few ADCs approved for cancer therapy and higher diversity is desirable to compensate for emerging resistances. In addition, there is also need in the art for novel, highly potent toxins, with no cryptophycin-based ADCs being approved so far. The development of cryptophycin-based ADCs is further complicated by the high efforts needed for their synthesis. In addition, ADCs sometimes lack efficacy against solid tumors. In those circumstances, the use of low molecular ligands as targeting moieties may overcome these drawbacks.

There is thus still need in the art for new targeted anti-tumor drugs based on potent cytotoxins. The present invention meets this need by providing a new class of cryptophycin compounds, cryptophycin payloads, and cryptophycin conjugates as well as novel processes for their preparation.

### SUMMARY OF THE INVENTION

In a first aspect, the present invention relates to a cryptophycin compound of formula (I) 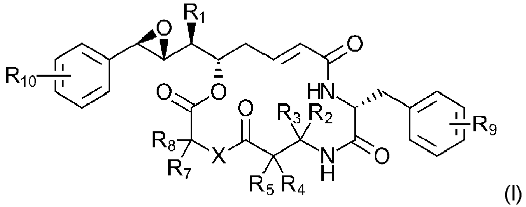 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, Rs, R₆, R₇ and R₈ represent, independently of each other, a hydrogen atom or a (C₁-C₆)alkyl group or a (C₁-C₆)alkylene-N(R₁₁)₂ group or a (C₁-C₆)alkylene-N⁺(R₁₁)₃ group or a (C₁-C₆)alkylene-OR₁₁ group or a (C₁-C₆)alkylene-SR₁₁ group or a (C₁-C₆)alkylene-S⁺(R₁₁)₂ group or a (C₁-C₆)alkylene-S(=O)R₁₁ group or a (C₁-C₆)alkylene-S⁺(=O)(R₁₁)₂ group or a (C₁-C₆)alkylene-S-SR₁₁ group or a (C₁-C₆)alkylene-COOR₁₁ group; or alternatively R₄ and Rs or R₇ and R₈ form together with the carbon atom to which they are attached a (C₃-C₆)cycloalkyl or a (C₃-C₆)heterocycloalkyl group; with the proviso that one of R₄, Rs, R₆, R₇ and R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)alkylene-SR₁₁, (C₁-C₆)alkylene-S⁺(R₁₁)₂, (C₁-C₆)alkylene-S(=O)R₁₁, (C₁-C₆)alkylene-S⁺(=O)(R₁₁)₂, (C₁-C₆)alkylene-S-SR₁₁, and (C₁-C₆)alkylene-COOR₁₁, and the others represent a hydrogen atom or a (C₁-C₆)alkyl group, preferably a hydrogen or (C₁-C₄)alkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₁ independently represents a hydrogen atom or a (C₁-C₆)alk(en)yl group; and
each R₁₂ independently represents a hydrogen atom, a (C₁-C₆)alkyl group, a (C₃-C₆)cycloalkyl group or a (C₃-C₆)heterocycloalkyl group.

In various embodiments, the compound of formula (I) is a compound of formula (1.1) 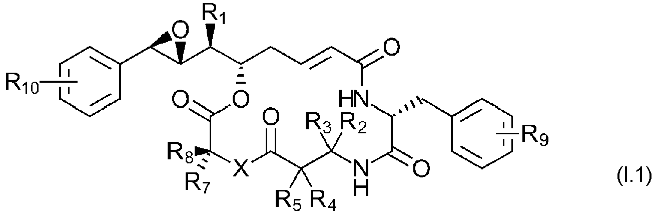 wherein the definitions of R₁-R₁₀ are as set forth above.

In the compound of formula (I) or (1.1), R₁ may be methyl.

In various embodiments of these compounds, each of R₂ and R₃ represents a hydrogen atom or one of R₂ and R₃ represents a hydrogen atom and the other one represents a methyl group or R₂ and R₃ form together with the carbon atom to which they are attached a cyclopropyl group.

In various embodiments, each of R₄ and R₅ represents a methyl or ethyl group, preferably methyl group, or one represents hydrogen and the other represents methyl or ethyl or both represent hydrogen or both combine to form together with the carbon atom to which they are attached a C₃-cycloalkyl group.

In various embodiments, X is O or NR₆, wherein R₆ represents a hydrogen atom.

R₇ may represent a hydrogen atom.

In various embodiments, R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)alkylene-SR₁₁, (C₁-C₆)alkylene-S⁺(R₁₁)₂, (C₁-C₆)alkyleneS(=O)R₁₁, (C₁-C₆)alkylene-S⁺(=O)(R₁₁)₂, (C₁-C₆)alkylene-S-SR₁₁, and (C₁-C₆)alkylene-COOR₁₁.

In various embodiments, R₉ represents at least two substituents, one being selected from a methoxy group or a N((C₁-C₆)alkyl)₂ or -N⁺((C₁-C₆)alkyl)₃ group, preferably being in the 4-position, and the other being selected from a halogen, preferably chlorine, atom, preferably being in the 3-position.

In some embodiments, R₁₀ represents a hydrogen atom.

All of the above described embodiments of R₁-R₁₀ and X may be realized individually or in combination.

Accordingly, in various embodiments, R₁ is methyl, each of R₂ and R₃ represents a hydrogen atom, R₆ represents a hydrogen atom, R₇ represents a hydrogen atom, R₉ represents two substituents selected from a methoxy group and a halogen, preferably chlorine, atom, more preferably 3-chloro-4-methoxy (relative to the phenyl ring to which these are attached), and R₁₀ represents a hydrogen atom. In such embodiments, R³, R⁴, R⁸ and X may be as defined above.

In various embodiments listed above, R₈ represents -(CH₂)ₚ-N(R₁₃)₂ or -(CH₂)ₚ-SR₁₃ wherein p is 1, 2, 3 or 4 and R₁₃ is preferably hydrogen or methyl.

In another aspect, the present invention relates to cryptophycin derivatives of formula (II) 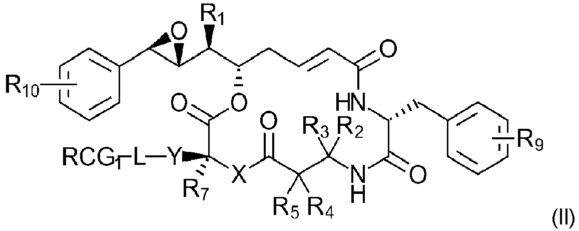 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, and R₇ represent, independently of each other, a hydrogen atom or a (C₁-C₆)alkyl group, preferably a hydrogen or (C₁-C₄)alkyl group; or alternatively R₄ and R₅ form together with the carbon atom to which they are attached a (C₃-C₆)cycloalkyl or a (C₃-C₆)heterocycloalkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-RCG₁ represents a group of formula: -(C₁-C₆)alkylene-NR₁₃-L-RCG₁, -(C₁-C₆)alkylene-N⁺(R₁₃)₂-L-RCG₁, -(C₁-C₆)alkylene-O-L-RCG₁, -(C₁-C₆)alkylene-S(=O)-L-RCG₁, -(C₁-C₆)alkylene-S⁺(=O)(R₁₃)-L-RCG₁, -(C₁-C₆)alkylene-S-L-RCG₁, -(C₁-C₆)alkylene-S⁺(R₁₃)-L-RCG₁, or -(C₁-C₆)alkylene-S-S-L-RCG₁; R₁₃ represents a (C₁-C₆)alkyl group;
L represents a linker group; and
RCG₁ represents a reactive group.

All embodiments for R₁ to R₇ and R₉ to R₁₀ and X disclosed above in relation to the compounds of formulae (I) and (1.1) also apply to the compounds of formula (II).

In various embodiments, of these cryptophycin derivatives L is a linker of the formula Str-Pep-Sp, wherein Str is a stretcher unit, Pep is a peptide or non-peptide linker unit, and Sp is a spacer unit.

Str may be a -(C₁-C₁₀)alkylene-C(=O)- group, a -(CH₂)ₐ-(O-CH₂CH₂)ₙ-(CH₂)_{b}-C(=O)- group, or a -CH₂)ₐ-(CH₂CH₂-O)ₙ-(CH₂)_{b}-C(=O)- group, wherein a and b are independently 0 or an integer of 1 to 4, and n is an integer of 1 to 20.

In various embodiments, Sp may be a spacer unit of formula

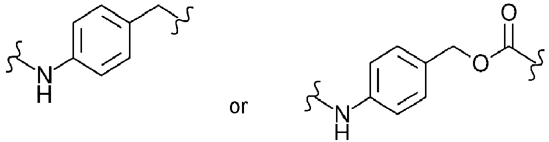

Pep may be a bond, a peptidyl moiety, or a non-peptide chemical moiety selected from the group consisting of: 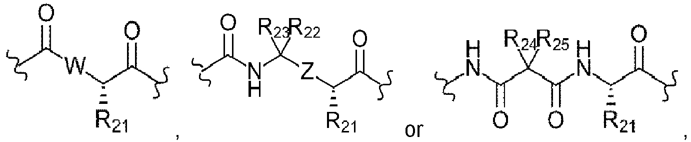 wherein
W is -NH-heterocycloalkylene- or heterocycloalkylene;
Z is bivalent heteroaryl, aryl, -C(=O)(C₁-C₆)alkylene, (C₂-C₆)alkenyl, (C₁-C₆)alkylenyl or (C₁-C₆)alkylene-NH-;
each R₂₁ is independently (C₁-C₁₀)alkyl, (C₂-C₁₀)alkenyl, (C₁-C₁₀)alkylNHC(=NH)NH₂, (C₁-C₁₀)alkylNHC(=O)NH₂ or (OCH₂CH₂)ₙ-OH or (CH₂CH₂O)ₙ-H with n = 3 to 50;
R₂₂ and R₂₃ are each independently H, (C₁-C₁₀)alkyl, (C₂-C₁₀)alkenyl, arylalkyl or heteroarylalkyl, or (OCH₂CH₂)ₙ-OH or (CH₂CH₂O)ₙ-H with n = 3 to 20, or R₂₂ and R₂₃ together with the carbon atom to which they are attached form (C₃-C₇)cycloalkyl; and
R₂₄ and R₂₅ are each independently (C₁-C₁₀)alkyl, (C₂-C₁₀)alkenyl, arylalkyl, or heteroarylalkyl, -CH₂-O-(C₁-C₁₀)alkyl, or R₂₂ and R₂₃ together with the carbon atom to which they are attached form (C₃-C₇)cycloalkyl.

In various embodiments, Pep is a peptidyl moiety and comprises or consists of a Val-Cit moiety, a Lys-β-Ala-Val-Cit moiety, a Phe-Lys moiety, or a Val-Ala moiety, wherein the side chain of lysine is optionally PEGylated, preferably by attachment of the PEG moiety to the terminal side chain amino group of lysine.

In various embodiments, RCG₁ is alkenyl, such as ethenyl, alkynyl, such as ethynyl, -N₃ or N-maleinimide.

In a still further aspect, the invention relates to a cryptophycin conjugate of formula (III) 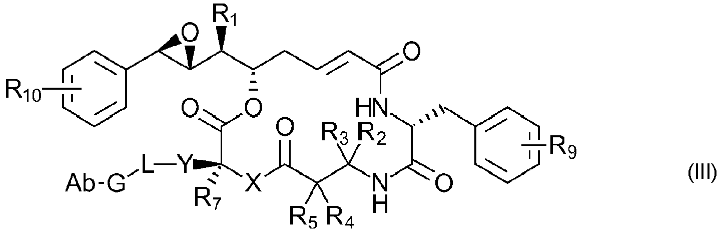 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, and R₇ represent, independently of each other, a hydrogen atom or a (C₁-C₆)alkyl group, preferably a hydrogen or (C₁-C₄)alkyl group; or alternatively R₄ and R₅ form together with the carbon atom to which they are attached a (C₃-C₆)cycloalkyl or a (C₃-C₆)heterocycloalkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-G-Ab represents a group of formula: -(C₁-C₆)alkylene-NR₁₃-L-G-Ab, -(C₁-C₆)alkylene-N⁺(R₁₃)₂-L-G-Ab, -(C₁-C₆)alkylene-O-L-G-Ab, -(C₁-C₆)alkylene-S(=O)-L-G-Ab, -(C₁-C₆)alkylene-S⁺(=O)(R₁₃)-L-G-Ab, -(C₁-C₆)alkylene-S-L-G-Ab, -(C₁-C₆)alkylene-S⁺(R₁₃)-L-G-Ab, or -(C₁-C₆)alkylene-S-S-L-G-Ab;
R₁₃ represents a (C₁-C₆)alkyl group;
L represents a linker group;
G represents the product of reaction between RCG1, a reactive chemical group present at the end of the linker and RCG2, an orthogonal reactive chemical group present on Ab; and
Ab represents a peptide moiety, preferably an oligopeptide or polypeptide moiety, preferably an antibody or antibody-like molecule, or a small molecule, preferably folic acid or DUPA (Glu-urea-Glu), as homing device.

All embodiments for R₁ to R₇ and R₉ to R₁₀ and X disclosed above in relation to the compounds of formulae (I), (1.1) and (II) also apply to the compounds of formula (III). Similarly, all embodiments of L disclosed above for the compounds of formula (II) also apply to the compounds of formula (III). Accordingly, in various embodiments, L is a linker of the formula Str-Pep-Sp, wherein Str is a stretcher unit, Pep is a peptide or non-peptide linker unit, and Sp is a spacer unit.

In the cryptophycin conjugates of the invention, Str may be a -(C₁-C₁₀)alkylene-C(=O)- group, a -(CH₂)ₐ-(O-CH₂CH₂)ₙ-(CH2)_{b}-C(=O)- group, or a -(CH₂)ₐ-(CH₂CH₂-O)ₙ-(CH₂)_{b}-C(=O)- group, wherein a and b are independently 0 or an integer of 1 to 4, n is an integer of 1 to 20.

G is a residue of reactive coupling group RCG₁ after the coupling reaction with RCG2 of Ab, and is preferably selected from:

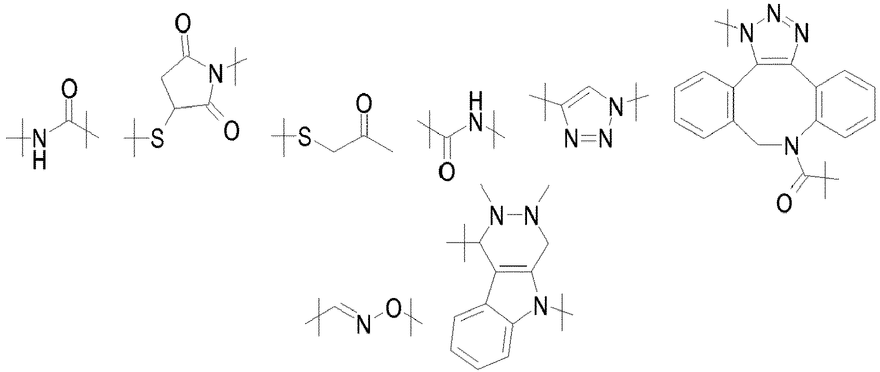

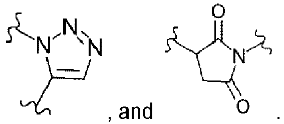

In all structures disclosed herein, if not explicitly indicated otherwise, each of R₁ to R₁₀ may adopt any one spatial configuration, e.g. S or R or alternatively E or Z.

The compounds of formulae (I), (1.1), (II), or (III) may contain one or more asymmetric carbon atoms. They may therefore exist in the form of enantiomers or diastereomers. These enantiomers or diastereomers, and also mixtures thereof, including racemic mixtures, form part of the invention.

The compounds of formulae (I), (1.1), (II), or (III) may exist in the form of bases or of acid addition salts, especially of pharmaceutically acceptable acids.

The present invention also encompasses the use of the cryptophycin compounds, derivatives and conjugates disclosed herein as a pharmaceutical, in particular the use of the conjugates of the present disclosure. The compounds, derivatives and conjugates for use as a pharmaceutical thus form one further aspect of the invention.

The cryptophycin compounds, derivatives and conjugates of the invention, in particular the conjugates, may be used as a pharmaceutical for treating cancer. The invention thus also covers methods for the treatment of cancer, typically in a subject in need thereof, by administrating an effective amount, typically a therapeutically effective amount, of the compounds, derivatives and conjugates disclosed herein.

In still another aspect, the invention features a pharmaceutical composition comprising any one or more of the cryptophycin compounds, derivatives or conjugates disclosed herein, and a pharmaceutically acceptable excipient, diluent, stabilizer and/or carrier.

### DETAILED DESCRIPTION

If not explicitly indicated otherwise, the terms used herein have the accepted meaning in the field.

The term "alkenyl group", as used herein, relates to a hydrocarbon group obtained by removing one hydrogen atom from an alkene. The alkenyl group may be linear or branched. Examples that may be mentioned include ethenyl (-CH=CH2, also termed vinyl) and propenyl (-CH2-CH=CH2, also termed allyl). Alkenyl can be preferably C₂₋₆ alkenyl or C₂₋₄ alkenyl or C₂₋₃ alkenyl. As stated above such groups may be in E or Z configuration and also mixtures of both configurations are included.

The term "alkoxy group", as used herein relates to the group -O-alkyl, in which the alkyl group is as defined below.

The term "alkyl group", as used herein, relates to a linear or branched saturated aliphatic hydrocarbon-based group obtained by removing a hydrogen atom from an alkane. Examples that may be mentioned include methyl, ethyl, propyl, isopropyl, butyl, isobutyl, fert-butyl, pentyl, neopentyl, 2,2-dimethylpropyl and hexyl groups. Alkyl can be preferably C₁₋₆ alkyl or C₁₋₄ alkyl or C₁₋₃ alkyl.

The term "alkylene group", as used herein, relates to a saturated divalent group of empirical formula - CₙH₂ₙ-, obtained by removing two hydrogen atoms from an alkane. The alkylene group may be linear or branched. Examples that may be mentioned include methylene (-CH₂-), ethylene (-CH₂CH₂-), propylene (-CH₂CH₂CH₂-), butylene (-CH₂CH₂CH₂CH₂-) and hexylene (-CH₂CH₂CH₂CH₂CH₂CH₂-) groups or the branched groups -CH(CH₃)-, -C(CH₃)₂-, -CH(CH(CH₃)₂)-, -C(CH₃)₂-CH₂-, and -C(CH₃)₂-CH₂-CH₂-, preferably, the alkylene group is of the formula -(CH₂)ₙ-, n representing an integer, for example 1 to 6; in the ranges of values, the limits are included (e.g. a range of the type "n ranging from 1 to 6" or "between 1 and 6" includes limits 1 and 6).

The term "antibody", as used herein, refers to an antibody with affinity for a biological target, more particularly a monoclonal antibody. The function of the antibody is to direct the biologically active compound as a cytotoxic compound towards the biological target. The antibody may be monoclonal, polyclonal or multispecific. It may also be an antibody fragment. In various embodiments, it may also be a murine, chimeric, humanized or human antibody. An "antibody" may be a natural or conventional antibody in which two heavy chains are linked to each other by disulfide bonds and each heavy chain is linked to a light chain by a disulfide bond (also referred to as a "full-length antibody"). The terms "conventional (or full-length) antibody" refers both to an antibody comprising the signal peptide (or pro-peptide, if any), and to the mature form obtained upon secretion and proteolytic processing of the chain(s). There are two types of light chain, lambda (I) and kappa (k). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1- H, CDR2-H, CDR3-H, respectively. A conventional antibody antigen-binding site, therefore, includes six CDRs, comprising the CDR set from each of a heavy and a light chain V region. As used herein, the term "antibody" denotes both conventional (full-length) antibodies and fragments thereof, as well as single domain antibodies and fragments thereof, in particular variable heavy chain of single domain antibodies. Fragments of (conventional) antibodies typically comprise a portion of an intact antibody, in particular the antigen binding region or variable region of the intact antibody, and retain the biological function of the conventional antibody. Examples of such fragments include Fv, Fab, F(ab')₂, Fab', dsFv, (dsFv)₂, scFv, sc(Fv)₂ and diabodies.

The function of the antibody, as used herein, is to direct the biologically active compound as a cytotoxic compound towards the biological target.

The term "aryl group", as used herein relates to a cyclic aromatic group containing between 5 to 10 carbon atoms. Examples of aryl groups include phenyl, tolyl, xylyl, naphtyl.

The term "biological target", as used herein, relates to an antigen (or group of antigens), preferably located at the surface of cancer cells or stromal cells associated with this tumor. These antigens may be, for example, a growth factor receptor, an oncogene product or a mutated "tumor suppressant" gene product, an angiogenesis-related molecule or an adhesion molecule

The term "conjugate", as used herein, relates to an antibody-drug conjugate or ADC, i.e. an antibody to which is covalently attached via a linker at least one molecule of a cytotoxic compound, namely the cryptophycin compounds disclosed herein.

The term "cycloalkyl group", as used herein, relates to a cyclic alkyl group comprising between 3 and 6 carbon atoms engaged in the cyclic structure. Examples that may be mentioned include cyclopropyl, cyclobutyl, cyclopentyl and cyclohexyl groups.

The term "DAR" (drug-to-antibody ratio) refers to an average number of cytotoxic molecules attached via a linker to an antibody.

The term "halogen", as used herein, relates to any of the four elements fluorine, chlorine, bromine and iodine.

The term "heteroaryl group", as used herein, relates to an aryl group containing between 2 to 10 carbon atoms and between 1 to 5 heteroatoms such as nitrogen, oxygen or sulfur engaged in the ring and connected to the carbon atoms forming the ring. Examples of heteroaryl groups include pyridyl, pyrimidyl, thienyl, imidazolyl, triazolyl, indolyl, imidazo-pyridyl, and pyrazolyl.

The term "heterocycloalkyl group", as used herein, relates to a cycloalkyl group containing between 2 to 8 carbon atoms and between 1 to 3 heteroatoms, such as nitrogen, oxygen or sulfur engaged in the ring and connected to the carbon atoms forming the ring. Examples include aziridinyl, pyrrolidinyl, piperidinyl, piperazinyl, morpholinyl, thiomorpholinyl, tetrahydrofuranyl, tetrahydrothiofuranyl, tetrahydropyranyl, azetidinyl, oxetanyl and pyranyl.

The term "linker", as used herein, relates to a group of atoms or a single bond that can covalently attach a cytotoxic compound to an antibody in order to form a conjugate.

The term "payload", as used herein, relates to a cytotoxic compound to which is covalently attached a linker.

The term "reactive chemical group", as used herein, relates to a group of atoms that can promote or undergo a chemical reaction.

The term "about", as used herein in relation to numerical values, refers to said numerical value ±10%, preferably ±5%.

The term "PEG", as used herein, relates to polyethylene glycol including residues thereof linked to another molecule, typically via an oxygen atom. Such PEG moieties typically contain 2 to 100 ethylene glycol units, for example 2 to 50 or 2 to 40 or 3 to 30.

The present invention relates to novel cryptophycin compounds. These compounds differ from known compounds in that they are differently functionalized to allow attachment of another moiety, typically a targeting moiety, usually via a linker moiety. Specifically, the compounds are functionalized in unit D or unit C of the cryptophycin structure, preferably unit D. It has been found that this position allows simple modification without significantly impairing activity of said compounds, i.e. their cytotoxicity, and, in various instances, even provides for increased cytotoxicity over coupling via different attachment points. The compounds of the invention can be synthesized in an efficient manner by existing methodologies.

Specifically, the inventors have identified cryptophycin compounds of formula (I) 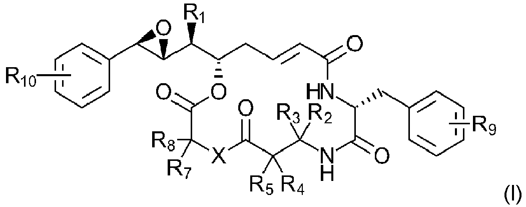 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, R₇ and R₈ represent, independently of each other, a hydrogen atom or a (C₁-C₆)alkyl group or a (C₁-C₆)alkylene-N(R₁₁)₂ group or a (C₁-C₆)alkylene-N⁺(R₁₁)₃ group or a (C₁-C₆)alkylene-OR₁₁ group or a (C₁-C₆)alkylene-SR₁₁ group or a (C₁-C₆)alkylene-S⁺(R₁₁)₂ group or a (C₁-C₆)alkylene-S(=O)R₁₁ group or a (C₁-C₆)alkylene-S⁺(=O)(R₁₁)₂ group or a (C₁-C₆)alkylene-S-SR₁₁ group or a (C₁-C₆)alkylene-COOR₁₁ group; or alternatively R₄ and R₅ or R₇ and R₈ form together with the carbon atom to which they are attached a (C₃-C₆)cycloalkyl or a (C₃-C₆)heterocycloalkyl group; with the proviso that at least one of R₄, R₅, R₆, R₇ and R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)alkylene-SR₁₁, (C₁-C₆)alkylene-S⁺(R₁₁)₂, (C₁-C₆)alkylene-S(=O)R₁₁, (C₁-C₆)alkylene-S⁺(=O)(R₁₁)₂, (C₁-C₆)alkylene-S-SR₁₁, and (C₁-C₆)alkylene-COOR₁₁, and the others represent a hydrogen atom or a (C₁-C₆)alkyl group, preferably a hydrogen or (C₁-C₄)alkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₁ independently represents a hydrogen atom or a (C₁-C₆)alk(en)yl group; and
each R₁₂ independently represents a hydrogen atom, a (C₁-C₆)alkyl group, a (C₃-C₆)cycloalkyl group or a (C₃-C₆)heterocycloalkyl group.

As described above, also encompassed are free bases of these compounds or acid addition salts thereof, such as acid addition salts with pharmaceutically acceptable acids. It is further understood that these compounds encompass all possible stereoisomers thereof, in particular enantiomers and diastereomers as well as mixtures thereof, including racemic mixtures.

In various preferred embodiments, the compound of formula (I) has a specific stereochemistry at the carbon atom bearing the R₇ and R₈ residues, and is a compound of formula (1.1)

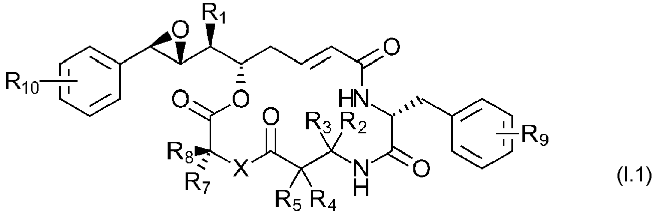

In said compound, the definitions of R₁-R₁₀ and X are as set forth above. It is again understood that said compound of formula (1.1) includes all possible stereoisomers thereof, in particular enantiomers and diastereomers as well as mixtures thereof, including racemic mixtures, that arise from other asymmetric carbon atoms in the structure.

In the compounds of formula (I) and (1.1), R₁ may be lower alkyl, i.e. C₁₋₄ alkyl, such as methyl, ethyl, n-propyl, isopropyl, n-butyl, and t-butyl. In exemplary embodiments it is methyl or ethyl, such as methyl.

In various embodiments of the compounds, each of R₂ and R₃ may represent a hydrogen atom. In certain embodiments, it may however be preferred that not both of R₂ and R₃ are hydrogen. In some embodiments, one of R₂ and R₃ thus represents a hydrogen atom and the other one represents an alkyl group, for example lower alkyl, i.e. C₁₋₄ alkyl, such as methyl, ethyl, n-propyl, isopropyl, n-butyl, and t-butyl. In exemplary embodiments, it is methyl or ethyl, such as methyl. In various embodiments, both of R₂ and R₃ are C₁₋₄ alkyl, such as methyl, ethyl, n-propyl, isopropyl, n-butyl, and t-butyl, preferably ethyl or methyl, most preferably methyl. While both may be selected independently, in various embodiments they are identical alkyl groups, such as methyl. In still other embodiments, R₂ and R₃ combine to form together with the carbon atom to which they are attached a cycloalkyl or heterogycloalkyl group. Particularly preferred cycloalkyl is a cyclopropyl group.

R₄, R₅, R₆, R₇ and R₈ represent, independently of each other, a hydrogen atom or a (C₁-C₆)alkyl group or a (C₁-C₆)alkylene-N(R₁₁)₂ group or a (C₁-C₆)alkylene-N⁺(R₁₁)₃ group or a (C₁-C₆)alkylene-OR₁₁ group or a (C₁-C₆)alkylene-SR₁₁ group or a (C₁-C₆)alkylene-S⁺(R₁₁)₂ group or a (C₁-C₆)alkylene-S(=O)R₁₁ group or a (C₁-C₆)alkylene-S⁺(=O)(R₁₁)₂ group or a (C₁-C₆)alkylene-S-SR₁₁ group or a (C₁-C₆)alkylene-COOR₁₁ group; or alternatively R₄ and R₅ or R₇ and R₈ form together with the carbon atom to which they are attached a (C₃-C₆)cycloalkyl or a (C₃-C₆)heterocycloalkyl group. These groups, with the exception of hydrogen and (C₁-C₆)alkyl, represent the reactive groups that allow attachment to another molecule, such as a targeting molecule, optionally via a linker group. Accordingly, said definition includes the proviso that at least one or only one of R₄, R₅, R₆, R₇ and R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)alkylene-SR₁₁, (C₁-C₆)alkylene-S⁺(R₁₁)₂, (C₁-C₆)alkylene-S(=O)R₁₁, (C₁-C₆)alkylene-S⁺(=O)(R₁₁)₂, (C₁-C₆)alkylene-S-SR₁₁, and (C₁-C₆)alkylene-COOR₁₁, while the others represent a hydrogen atom or a (C₁-C₆)alkyl group, preferably a hydrogen or (C₁-C₄)alkyl group. The limitation that only one of these residues can be selected from the list of functional groups above provides for molecules that have only one functionality for attachment to another molecule and thus to avoid undesired side reactions. While it is preferred that only one of these residues is selected from the list of functional groups defined above, in certain embodiments it may also be possible that two or more of these residues, for example any 2, 3, 4 or 5 thereof, are such a functional group.

In various embodiments, one of R₄, R₅, R₆, R₇ and R₈ represents a group selected from (C₁-C₄)alkylene-N(R₁₁)₂, (C₁-C₄)alkylene-N⁺(R₁₁)₃, (C₁-C₄)alkylene-OR₁₁, (C₁-C₄)alkylene-SR₁₁, (C₁-C₄)alkylene-S⁺(R₁₁)₂, (C₁-C₄)alkylene-S(=O)R₁₁, (C₁-C₄)alkylene-S⁺(=O)(R₁₁)₂, (C₁-C₄)alkylene-S-SR₁₁, and (C₁-C₄)alkylene-COOR₁₁, for example -(CH₂)ₚ-N(R₁₁)₂, -(CH₂)ₚ-N⁺(R₁₁)₃, -(CH₂)ₚ-OR₁₁, -(CH₂)ₚ-SR₁₁, -(CH₂)ₚ-S⁺(R₁₁)₂, - (CH₂)ₚ-S(=O)R₁₁, -(CH₂)ₚ-S⁺(=O)(R₁₁)₂, -(CH₂)_{P}-S-SR₁₁, and -(CH₂)ₚ-COOR₁₁, wherein p is 1, 2, 3 or 4, preferably 1, 2 or 3, more preferably 1 or 2.

In various embodiments, wherein the compounds of the invention have the stereochemistry of formula (1.1), the functional group is not in the position of R₇. In such embodiments, the functional group is preferably in the R₈ position.

In various embodiments, each of R₄ and R₅ represents a methyl or ethyl group, preferably methyl group, or one represents hydrogen and the other represents methyl or ethyl or both represent hydrogen or both combine to form together with the carbon atom to which they are attached to form a cycloalkyl group, such as a cyclopropyl group.

In various embodiments, X is O or NR₆, wherein R₆ represents a hydrogen atom or (C₁-C₆)alkyl, such as methyl or ethyl, preferably hydrogen or methyl, more preferably hydrogen.

R₇ may represent a hydrogen atom or (C₁-C₆)alkyl, such as methyl or ethyl, preferably hydrogen or methyl, more preferably hydrogen.

In various embodiments, R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)atkytene-SR₁₁, (C₁-C₆)alkylene-S⁺(R₁₁)₂, (C₁-C₆)alkyleneS(=O)R₁₁, (C₁-C₆)alkylene-S⁺(=O)(R₁₁)₂, (C₁-C₆)alkylene-S-SR₁₁, and (C₁-C₆)alkylene-COOR₁₁. In some embodiments, R₈ may be (C₁-C₆)alkylene-N(R₁₁)₂, (C₁-C₆)alkylene-N⁺(R₁₁)₃, (C₁-C₆)alkylene-SR₁₁, (C₁-C₆)alkylene-S⁺(R₁₁)₂, (C₁-C₆)alkylene-S(=O)R₁₁, (C₁-C₆)alkylene-S⁺(=O)(R₁₁)₂, or (C₁-C₆)alkylene-S-SR₁₁. In specific embodiments, R₈ may be (C₁-C₆)alkylene-N(R₁₁)₂, (C₁-C₆)alkylene-N⁺(R₁₁)₃, (C₁-C₆)alkylene-SR₁₁, or (C₁-C₆)alkylene-S⁺(R₁₁)₂. In all such embodiments of R₈, R₇ is preferably hydrogen.

In various embodiments, R₉ represents one or at least two substituents. Generally, in such embodiments, R₉ is selected from a methoxy group or a N((C₁-C₆)alkyl)₂ or -N⁺((C₁-C₆)alkyl)₃ group, preferably being in the 4-position, and/or a halogen, preferably chlorine, atom, preferably being in the 3-position. In various embodiments, R₉ represent 2 different substituents, one being selected from a methoxy group or a N((C₁-C₆)alkyl)₂ or -N⁺((C₁-C₆)alkyl)₃ group, preferably being in the 4-position, and the other being a halogen, preferably chlorine, atom, preferably being in the 3-position.

In some embodiments, R₁₀ represents a single substituent selected from the given list, preferably a hydrogen atom. This results in the phenyl ring of unit A of the cryptophycin structure being unsubstituted.

All of the above described more specific embodiments of R₁-R₁₀ and X may be present individually or in combination.

In various embodiments, R₁₁ is hydrogen or methyl. In various embodiments, wherein R₁₁ is attached to a nitrogen atom, at least one R₁₁ may not be hydrogen, for example methyl. In various other embodiments, in particular where R¹¹ is attached to an oxygen atom, R¹¹ may be an alkenyl group, such as ethenyl (vinyl) or 2-propenyl (allyl).

In various embodiments, not all or no R₁₂ is hydrogen.

Accordingly, in various embodiments, R₁ is methyl, each of R₂ and R₃ represents a hydrogen atom, R₆ represents a hydrogen atom, R₇ represents a hydrogen atom, R₉ represents two substituents selected from a methoxy group and a halogen, preferably chlorine, atom, more preferably 3-chloro-4-methoxy (relative to the phenyl ring to which these are attached), and R₁₀ represents a hydrogen atom. In such embodiments, R³, R⁴, R⁸ and X may be as defined above, preferably R₄ and R₅ may be methyl and X is NH. In such embodiments, R₈ may be as defined above, but may, in various embodiments, not be -CH₂-N(CH₃)₂ or -CH₂-COOH.

In various embodiments listed above, R₈ represents -(CH₂)ₚ-N(R₁₃)₂ or -(CH₂)ₚSR₁₃ wherein p is 1, 2, 3 or 4, such as 1, 2 or 3, or 1 or 2, and R₁₃ is preferably hydrogen or methyl.

The present invention also relates to cryptophycin derivatives that are obtainable using the compounds of formula (I) or (1.1). These may also be referred to as cryptophycin payloads and may be compounds of formula (I) or (1.1) or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, R₇ and R₈ represent, independently of each other, a hydrogen atom or a (C₁-C₆)alkyl group or -Y-L-RCG₁; or alternatively R₄ and R₅ or R₇ and R₈ form together with the carbon atom to which they are attached a (C₃-C₆)cycloalkyl or a (C₃-C₆)heterocycloalkyl group; with the proviso that one of R₄, R₅, R₆, R₇ and R₈ represents-Y-L-RCG₁, and the others represent a hydrogen atom or a (C₁-C₆)alkyl group, preferably a hydrogen or (C₁-C₄)alkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-RCG₁ represents a group of formula: -(C₁-C₆)alkylene-NR₁₃-L-RCG₁, -(C₁-C₆)alkylene-N⁺(R₁₃)₂-L-RCG₁, -(C₁-C₆)alkylene-O-L-RCG₁, -(C₁-C₆)alkylene-S(=O)-L-RCG₁, -(C₁-C₆)alkylene-S⁺(=O)(R₁₃)-L-RCG₁, -(C₁-C₆)alkylene-S-L-RCG₁, -(C₁-C₆)alkylene-S⁺(R₁₃)-L-RCG₁, or -(C₁-C₆)alkylene-S-S-L-RCG₁; R₁₃ represents a (C₁-C₆)alkyl group;
L represents a linker group; and
RCG₁ represents a reactive group.

While the Y-L-RCG₁ group may be any one of one of R₄, R₅, R₆, R₇ and R₈, in the following the invention is described in more detail based on embodiments, wherein R₈ is -Y-L-RCG1. While this is one specific exemplary embodiment, all alternative embodiments in which any other of the residues is said group are still considered to fall within the scope of the present invention.

In various embodiments, such cryptophycin derivatives or payloads may be compounds of formula (II) 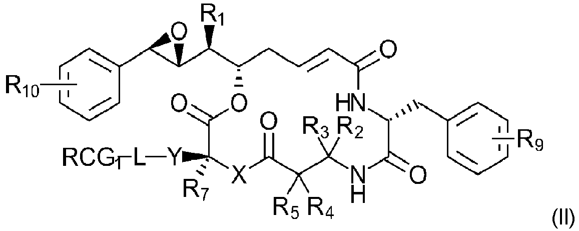 or stereoisomers or a pharmaceutically acceptable salts thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, and R₇ represent, independently of each other, a hydrogen atom or a (C₁-C₆)alkyl group, preferably a hydrogen or (C₁-C₄)alkyl group; or alternatively R₄ and R₅ form together with the carbon atom to which they are attached a (C₃-C₆)cycloalkyl or a (C₃-C₆)heterocycloalkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-RCG₁ represents a group of formula: -(C₁-C₆)alkylene-NR₁₃-L-RCG₁, -(C₁-C₆)alkylene-N⁺(R₁₃)₂-L-RCG₁, -(C₁-C₆)alkylene-O-L-RCG₁, -(C₁-C₆)alkylene-S(=O)-L-RCG₁, -(C₁-C₆)alkylene-S⁺(=O)(R₁₃)-L-RCG₁, -(C₁-C₆)alkylene-S-L-RCG₁, -(C₁-C₆)alkylene-S⁺(R₁₃)-L-RCG₁, or -(C₁-C₆)alkylene-S-S-L-RCG₁; R₁₃ represents a (C₁-C₆)alkyl group;
L represents a linker group; and
RCG₁ represents a reactive group.

The present invention is further directed to conjugates that are obtainable using the compounds of formula (II). These may be compounds of formula (I) or (1.1) or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, R₇ and R₈ represent, independently of each other, a hydrogen atom or a (C₁-C₆)alkyl group or -Y-L-G-Ab; or alternatively R₄ and R₅ or R₇ and R₈ form together with the carbon atom to which they are attached a (C₃-C₆)cycloalkyl or a (C₃-C₆)heterocycloalkyl group; with the proviso that one of R₄, R₅, R₆, R₇ and R₈ represents -Y-L-Ab, and the others represent a hydrogen atom or a (C₁-C₆)alkyl group, preferably a hydrogen or (C₁-C₄)alkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-G-Ab represents a group of formula: -(C₁-C₆)alkylene-NR₁₃-L-G-Ab, -(C₁-C₆)alkylene-N⁺(R₁₃)₂-L-G-Ab, -(C₁-C₆)alkylene-O-L-G-Ab, -(C₁-C₆)alkylene-S(=O)-L-G-Ab, -(C₁-C₆)alkylene-S⁺(=O)(R₁₃)-L-G-Ab, -(C₁-C₆)alkylene-S-L-G-Ab, -(C₁-C₆)alkylene-S⁺(R₁₃)-L-G-Ab, or -(C₁-C₆)alkylene-S-S-L-G-Ab;
R₁₃ represents a (C₁-C₆)alkyl group;
L represents a linker group;
G represents the product of reaction between RCG1, a reactive chemical group present at the end of the linker and RCG2, an orthogonal reactive chemical group present on Ab; and
Ab represents a peptide moiety, preferably an oligopeptide or polypeptide moiety, preferably an antibody or antibody-like molecule, or a small molecule, preferably folic acid or DUPA (Glu-urea-Glu), as homing device.

While the Y-L-G-Ab group may be any one of one of R₄, R₅, R₆, R₇ and R₈, in the following the invention is described in more detail based on embodiments, wherein R₈ is -Y-L-Ab. While this is one specific exemplary embodiment, all alternative embodiments in which any other of the residues is said group are still considered to fall within the scope of the present invention.

In various embodiments, these conjugates are compounds of formula (III) 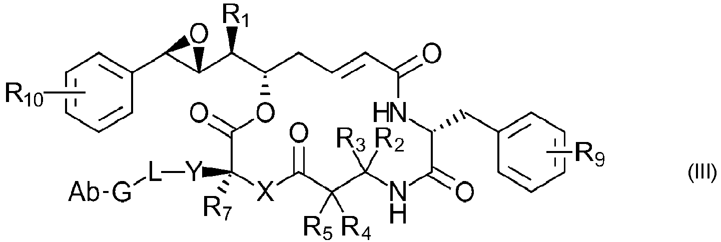 or stereoisomers or a pharmaceutically acceptable salts thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, and R₇ represent, independently of each other, a hydrogen atom or a (C₁-C₆)alkyl group, preferably a hydrogen or (C₁-C₄)alkyl group; or alternatively R₄ and R₅ form together with the carbon atom to which they are attached a (C₃-C₆)cycloalkyl or a (C₃-C₆)heterocycloalkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-Ab represents a group of formula: -(C₁-C₆)alkylene-NR₁₃-L-G-Ab, -(C₁-C₆)alkylene-N⁺(R₁₃)₂-L-G-Ab, -(C₁-C₆)alkylene-O-L-G-Ab, -(C₁-C₆)alkylene-S(=O)-L-G-Ab, -(C₁-C₆)alkylene-S⁺(=O)(R₁₃)-L-G-Ab, - (C₁-C₆)alkylene-S-L-G-Ab, -(C₁-C₆)alkylene-S⁺(R₁₃)-L-G-Ab, or -(C₁-C₆)alkylene-S-S-L-G-Ab;
R₁₃ represents a (C₁-C₆)alkyl group;
L represents a linker group;
G represents the product of reaction between RCG1, a reactive chemical group present at the end of the linker and RCG2, an orthogonal reactive chemical group present on Ab; and
Ab represents a peptide moiety, preferably an oligopeptide or polypeptide moiety, preferably an antibody or antibody-like molecule, or a small molecule, preferably folic acid or DUPA (Glu-urea-Glu), as homing device.

All embodiments for R₁ to R₇ and R₉ to R₁₀ and X disclosed above in relation to the compounds of formulae (I) and (1.1) also apply to the compounds of formula (II) and (III).

The attachment between the cryptophycin payload/derivative described herein, in particular those of formula (II), and the peptide moiety or small molecule Ab, in order to obtain the conjugates of the invention, in particular those of formula (III), are produced by means of the reactive chemical group RCG₁ present on the payload that is reactive towards a reactive group RCG₂ present on Ab, i.e. the peptide moiety or small molecule, for example an antibody. The reaction between RCG1 and RCG2 ensures the attachment of the cryptophycin compound, i.e. the payload or derivative, as defined herein, including those of formula (II) to the peptide moiety or small molecule by formation of a covalent bond. In the conjugates of the invention, such as those of formula (III), parts of RCG1 and RCG2 can remain, for example as G, forming the attachment between the linker and the antibody.

In various embodiments, RCG₁ is alkenyl, such as ethenyl, alkynyl, such as ethynyl, -N₃ or N-maleinimide.

Generally, examples of RCG1 include, without limitation,
(i) -C(=O)-ZₐRₐ wherein Zₐ represents a single bond, O or NH, preferably O, and Rₐ represents a hydrogen atom or a (C₁-C₆)alkyl, (C₃-C₇)cycloalkyl, alkenyl, aryl, heteroaryl or (C₃-C₇)heterocycloalkyl group. The aryl group may be substituted by 1 to 5 groups selected from halogen, in particular F, alkyl, alkoxy, nitro and cyano groups;
(ii) one of the following reactive groups, the maleimido 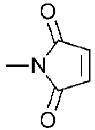 group; the haloacetamido group -N(R₁₄)-C(=O)-CH₂-Br or -N(R₁₄)-C(=O)-CH₂-I with R₁₄ representing a hydrogen atom or a (C₁-C₆)alkyl group, more specifically methyl; -CI; -N₃; -OH, -SH, -NH₂; -C≡CH or an activated C≡C such as a cyclooctyne moiety like 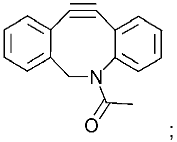 an O-alkyl hydroxylamine or a Pictet-Spengler reaction substrate, such as 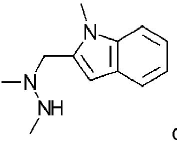 lescribed in Agarwal et al. (Bioconjugate Chem 2013, 24, 846-851).

In various embodiments, ZₐRₐ may represent -OH, -OCH₃, -OCH₂CH=CH₂, 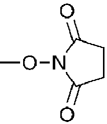 (O-NHS) or 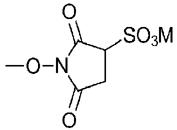 wherein M is hydrogen or a cation, such as sodium or potassium, or 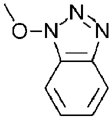 or 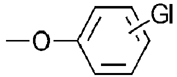 wherein GI represents at least one electroinductive group, such as nitro of halogen, preferably fluorine, with exemplary groups being 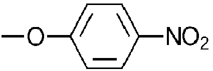 and 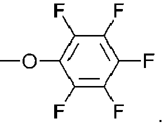 Another type of -C(=O)-ZₐRₐ includes

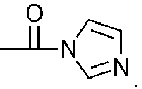

Examples of RCG2 include, without limitation, those described by Garnett et al. (Advanced Drug Delivery Reviews 2001, 53, 171-216). Exemplary groups include, without limitation,
(i) epsilon-amino groups of lysines borne by the side chains of lysine residues that are present in the peptide moiety or antibody;
(ii) alpha-amino groups of N-terminal amino acids of peptide moieties, such as antibody heavy and/or light chains;
(iii) saccharide groups that may, for example, be present in glycosylated peptides/proteins, such as the antibody hinge region;
(iv) the thiols of cysteines present in peptide moieties, such as antibodies, that may be engineered or generated by reducing disulfide bonds;
(v) amide groups, such as those present in the side chains of glutamine or asparagine in peptides or proteins, including antibodies; and
(vi) aldehyde groups, optionally introduced using formylglycine generating enzyme.

More recently, other conjugation approaches have been considered, for instance the introduction of cysteines by mutation (Junutula J.R., et al., Nature Biotechnology 2008, 26, 925-932), the introduction of unnatural amino acids allowing other types of chemistry (Axup J.Y., et al., PNAS 2012, 109, 40, 16101-16106) or the conjugation on antibody glycans (Zhou Q., et al., Bioconjugate Chem. 2014, 25, 510-520). Another approach for site-specific modifications of antibodies is based on enzymatic labeling using for example bacterial transglutaminase (Jeger S., et al., Angew. Chem. Int. Ed. 2010, 49, 9995-9997; Strop P., et al., Chem. Biol. 2013, 20, 161- 167) or formylglycine generating enzyme (Hudak J.E., et al., Angew. Chem. Int. Ed. 2012, 51, 4161-4165). For a review of site-specific conjugation strategies, see Agarwal P. and Bertozzi C.R., Bioconjugate Chem 2015, 26, 176-192. These conjugation technologies may also be applied to cryptophycin payloads described in the present invention.

It is also possible to chemically modify the peptide moiety, such as an antibody, so as to introduce novel reactive chemical groups RCG2. Thus, it is well known to those skilled in the art how to modify an antibody with the aid of a modifying agent introducing for example activated disulfide, thiol, maleimido or haloacetamido groups (see especially WO2005/077090 page 14 and WO2011/001052). The modification makes it possible to improve the conjugation reaction and to use a wider variety of groups RCG1. More particularly, in the case where RCG1 is of the type (ii) above, it is possible to chemically modify the antibody using an adequate modifying agent or to introduce one or more unnatural amino acids so as to introduce the adequate functions RCG2.

For example:
- when RCG1 represents a N-hydroxysuccinimidyl ester, RCG2 represents a -NH₂ group;
- when RCG1 represents a maleimido or haloacetamido function or a -Cl group, RCG2 may be a -SH group;
- when RCG1 represents a -N₃ group, RCG2 may be a -C≡CH group or an activated C≡C such as a cyclooctyne moiety;
- when RCG1 represents a -OH or -NH₂ group, RCG2 may be a carboxylic acid or amide function;
- when RCG1 represents a -SH group, RCG2 may be a maleimido or haloacetamido function;
- when RCG1 represents a -C≡CH function or an activated C≡C, RCG2 may be a -N₃ group;
- when RCG1 represents a -O-alkyl hydroxylamine function or a Pictet-Spengler reaction substrate, RCG2 may be an aldehyde or ketone function.

Examples of G that result from reaction of RCG1 and RCG2 include, without limitation,

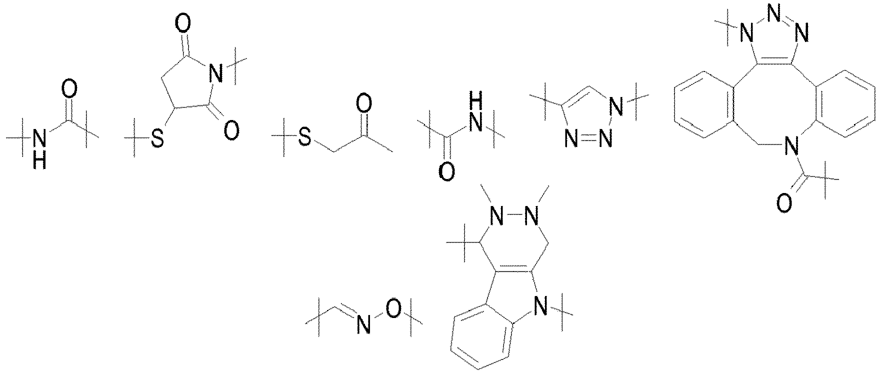

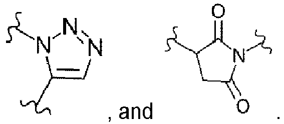

In various embodiments of the cryptophycin derivatives/payloads of the invention, in particular those of formula (II), or the conjugates of the invention, in particular those of formula (III), L is a linker of the formula Str-Pep-Sp, wherein Str is a stretcher unit, Pep is a bond, a peptidyl moiety or non-peptide linker unit, and Sp is a spacer unit. The linker is preferably oriented such that the Sp spacer unit is attached to the cryptophycin moiety. The Pep unit is preferably oriented such that the N-terminus is attached to the Str unit and the C-terminus to the Sp unit.

Str may be a-(C₁-C₁₀)alkylene- group, a -(C₁-C₁₀)alkylene-C(=O)- group, a -(C₁-C₁₀)alkylene-NH- group, a -(CH₂)ₐ-(O-CH₂CH₂)ₙ-(CH₂)_{b}-NH- group, a -(CH₂)ₐ-(CH₂CH₂-O)ₙ-(CH₂)_{b}-NH- group, a -(CH₂)ₐ-(O-CH₂CH₂)ₙ-(CH₂)_{b}-C(=O)- group, or a -(CH₂)ₐ-(CH₂CH₂-O)ₙ-(CH₂)_{b}-C(=O)- group, wherein a and b are independently 0 or an integer of 1 to 4, and n is an integer of 1 to 20.

In various embodiments, Sp may be a spacer unit of formula

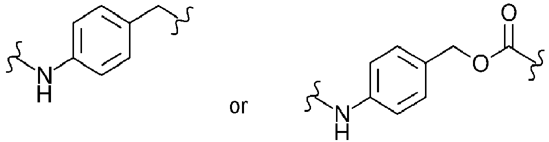

Pep may be a bond, a peptidyl moiety, or a non-peptide chemical moiety selected from the group consisting of: 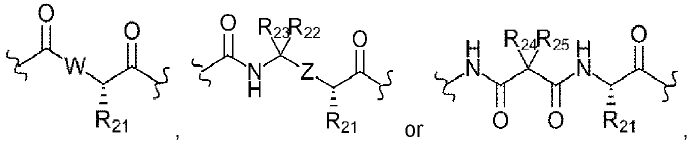 wherein
W is -NH-heterocycloalkylene- or heterocycloalkylene;
Z is bivalent heteroaryl, aryl, -C(=O)(C₁-C₆)alkylene, (C₂-C₆)alkenyl, (C₁-C₆)alkylene or (C₁-C₆)alkylene-NH-;
each R₂₁ is independently (C₁-C₁₀)alkyl, (C₂-C₁₀)alkenyl, (C₁-C₁₀)alkylNHC(=NH)NH₂, (C₁-C₁₀)alkylNHC(=O)NH₂ or (OCH₂CH₂)ₙ-OH or (CH₂CH₂O)ₙ-H with n = 3 to 50;
R₂₂ and R₂₃ are each independently H, (C₁-C₁₀)alkyl, (C₂-C₁₀)alkenyl, arylalkyl or heteroarylalkyl, or (OCH₂CH₂)ₙ-OH or (CH₂CH₂O)ₙ-H with n = 3 to 20, or R₂₂ and R₂₃ together with the carbon atom to which they are attached form (C₃-C₇)cycloalkyl; and
R₂₄ and R₂₅ are each independently (C₁-C₁₀)alkyl, (C₂-C₁₀)alkenyl, arylalkyl, or heteroarylalkyl, -CH₂-O-(C₁-C₁₀)alkyl, or R₂₂ and R₂₃ together with the carbon atom to which they are attached form (C₃-C₇)cycloalkyl.

In various embodiments, Pep is a peptidyl moiety and comprises or consists of 1 to 10 amino acids, typically 2 to 4 amino acids linked by peptide bonds. The amino acids may be in D or L configuration and may comprise natural and unnatural amino acids, in particular proteinogenic and non-proteinogenic amino acids. Said amino acids may be selected from, without limitation, alanine (Ala), beta-alanine, gamma-aminobutyric acid, 2-amino-2.cyclohexylacetic acid, 2-amino-2-phenylacetic acid, arginine (Arg), asparagine (Asn), aspartic acid (Asp), citrulline (Cit), cysteine (Cys), alpha,alpha-dimethyl-gamma-aminobutyric acid, beta,beta-dimethyl-gamma-aminobutyric acid, glutamine (Gln), glutamic acid (Glu), glycine (Gly), histidine (His), isoleucine (Ile), leucine (Leu), lysine (Lys), epsilon-acetyl-lysine (AcLys), methionine (Met), ornithine (Orn), phenylalanine (Phe), proline (Pro), serine (Ser), threonine (Thr), tryptophan (Trp), tyrosine (Tyr), and valine (Val). In various embodiments, the amino acids are selected from alanine, citrulline, glutamine, glycine, epsilon.acetyl-lysine, valine, lysine and beta-alanine. In various embodiments, the Pep moieity may be a dipeptide, tripeptide or tetrapeptide, such as Gly-Gly, Phe-Lys, Val-Lys, Val-AcLys, Val-Cit, Phe-Phe-Lys, D-Phe-Phe-Lys, Gly-Phe-Lys, Ala-Lys, Val-Ala, Phe-Cit, Leu-Cit, Ile-Cit, Trp-Cit, Phe-Ala, Ala-Phe, Gly-Gly-Gly, Gly-Ala-Phe, Gly-Val-Cit, Gly-Phe-Leu-Cit, Gly-Phe-Leu-Gyl, Ala-Leu-Ala-Leu, and Lys-Ala-Val-Cit. In all these peptides, Ala may be replaced by beta-alanine. In various embodiments, the Pep moiety is a Val-Cit moiety, a Lys-β-Ala-Val-Cit moiety, a Phe-Lys moiety, or a Val-Ala moiety.

In various embodiments, the amino acids in the Pep moiety may be further modified, in particular by side chain modifications. One exemplary modification is PEGylation, i.e. attachment of a polyethylene glycol moiety, typically comprising 2 to 25 units. In some embodiments, amino groups in the side chain are modified, such as those of lysine. PEGylation, for example by attachment of the PEG moiety to the terminal side chain amino group of lysine, can be achieved using routine methods (See, e.g., Veronese FM. Peptide and protein PEGylation: a review of problems and solutions. Biomaterials. 2001;22(5):405-417; Tan H, et al. Curr Pharm Des. 2018;24(41):4932-4946; Bumbaca, B. et al. Drug Metab Pharmacokinet. 2019;34(1):42-54). For this reaction, the PEG is typically activated with NHS forming N-hydroxylsuccinimide (NHS) functionalized polyethylene glycol (PEG-NHS). The terminal end of the PEG moiety may be capped, for example with a methoxy group.

In various embodiments of the cryptophycin derivatives of the present invention, in particular those of formula (II), RCG1 is a maleimido 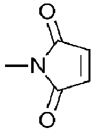 group and L is a group of formula Str-Pep-Sp. In such embodiments, the Str unit is attached to the maleimino group and preferably a -(C₁-C₁₀)alkylene-C(=O)-group, a -(CH₂)ₐ-(O-CH₂CH₂)ₙ-(CH₂)_{b}-C(=O)- group, or a -(CH₂)ₐ-(CH₂CH₂-O)ₙ-(CH₂)_{b}-C(=O)- group, wherein a and b are independently 0 or an integer of 1 to 4, preferably a is 1 to 4, for example 2 to 4, and b is 0 or 1, and n is an integer of 1 to 20, preferably a a -(C₁-C₁₀)alkylene-C(=O)- group, preferably a C₄₋₇ alkylene, more preferably linear C₅ alkylene group. In such embodiments, Pep is a peptidyl moiety, preferably selected from Gly-Gly, Phe-Lys, Val-Lys, Val-AcLys, Val-Cit, Phe-Phe-Lys, D-Phe-Phe-Lys, Gly-Phe-Lys, Ala-Lys, Val-Ala, Phe-Cit, Leu-Cit, Ile-Cit, Trp-Cit, Phe-Ala, Ala-Phe, Gly-Gly-Gly, Gly-Ala-Phe, Gly-Val-Cit, Gly-Phe-Leu-Cit, Gly-Phe-Leu-Gyl, Ala-Leu-Ala-Leu, and Lys-Ala-Val-Cit or the respective beta-alanine variants thereof, more preferably selected from a Val-Cit moiety, a Lys-β-Ala-Val-Cit moiety, a Phe-Lys moiety, or a Val-Ala moiety, most preferably a Lys-β-Ala-Val-Cit moiety, with Lys optionally being PEGylated. All of these peptides are given in N- to C-terminal orientation, with Str attached to N-terminus and Sp attached to the C-terminus. In these embodiments, Sp may be a spacer unit of formula 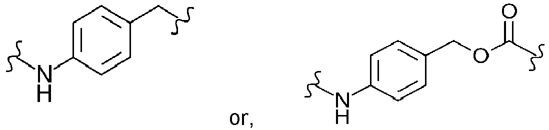 wherein the NH group is attached to the C-terminus of the Pep moiety. In these embodiments, Y is -(C₁-C₆)alkylene-NR₁₃-, -(C₁-C₆)alkylene-N⁺(R₁₃)₂-, -(C₁-C₆)alkylene-O-, -(C₁-C₆)alkylene-S(=O)-, -(C₁-C₆)alkylene-S⁺(=O)(R₁₃)-, -(C₁-C₆)alkylene-S-, -(C₁-C₆)alkylene-S⁺(R₁₃)-, or -(C₁-C₆)alkylene-S-S-, preferably -(C₁-C₆)alkylene-NR₁₃- or -(C₁-C₆)alkylene-N⁺(R₁₃)₂-. In these embodiments, the functional group of Y, i.e. the heteroatom, is attached to Sp.

The respective moieties RCG1-L-Y- may thus, in various embodiments, be groups of the formula (IV.1) or (IV.2):

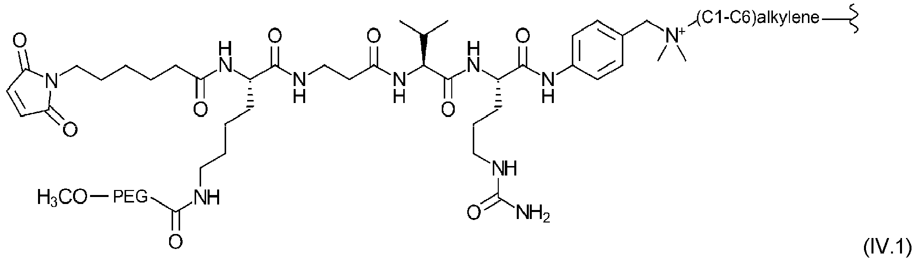

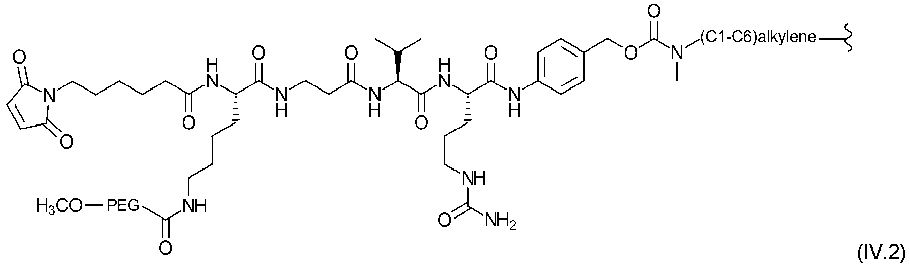

The peptidyl linkers/peptide moieties used in these formulae (Lys-β-Ala-Val-Cit moiety) may be replaced by any of those disclosed above, namely a dipeptide, tripeptide or tetrapeptide, such as Gly-Gly, Phe-Lys, Val-Lys, Val-AcLys, Val-Cit, Phe-Phe-Lys, D-Phe-Phe-Lys, Gly-Phe-Lys, Ala-Lys, Val-Ala, Phe-Cit, Leu-Cit, Ile-Cit, Trp-Cit, Phe-Ala, Ala-Phe, Gly-Gly-Gly, Gly-Ala-Phe, Gly-Val-Cit, Gly-Phe-Leu-Cit, Gly-Phe-Leu-Gyl, Ala-Leu-Ala-Leu, and Lys-Ala-Val-Cit. In all these, Ala may be replaced by beta-alanine.

In the compounds of formula (IV.1), the ammonium nitrogen (N⁺(CH₃)₂) may also be replace by a sulfonium group of the formula S⁺(CH₃).

All embodiments of L disclosed above for the compounds of formula (II) also apply to the compounds of formula (III). Suitable linkers and their chemistry are also described in more detail in WO 2016/090050 A1, which is herewith incorporated by reference in its entirety.

Exemplary moieties Ab-G-L-Y- can, in various embodiments, be selected from the groups of formula (V.1) and (V.2):

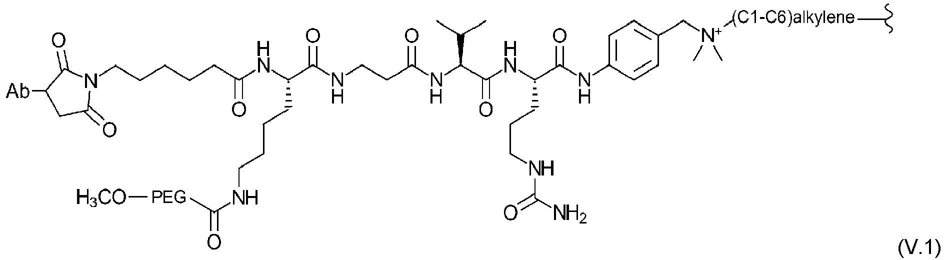

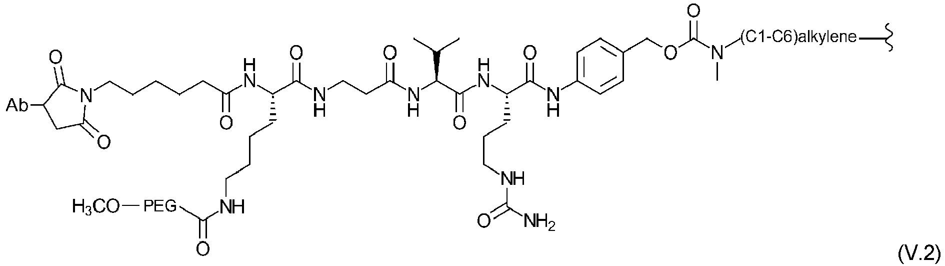

In the compounds of formula (V.1), the ammonium nitrogen (N⁺(CH₃)₂) may also be replaced by a sulfonium group of the formula S⁺(CH₃).

In the conjugates of the invention, in particular those of formula (III), (V.1) and (V.2), "Ab" represents a peptide moiety, preferably an oligopeptide or polypeptide moiety, preferably an antibody or antibody-like molecule, or a small molecule, preferably folic acid or DUPA (Glu-urea-Glu), as homing device. In these embodiments, the Ab moiety functions as a targeting or homing device that facilitates delivery of the molecule, in particular the cryptophycin payload, to its site of action, typically a tissue or cell type that is specifically recognized and bound by the Ab moiety. The function of the Ab moiety is thus to direct the biologically active compound as a cytotoxic compound towards the biological target.

The term "peptide", as used in this context, relates to a polymer of at least 2 amino acids, typically proteinogenic amino acids selected from the 20 naturally occurring proteinogenic amino acids Gly, Ala, Val, Leu, Ile, Phe, Met, Cys, His, Lys, Arg, Glu, Asp, Gln, Asn, Ser, Thr, Pro, Trp and Tyr, that are linked by a peptide bond and coupled to the linker moiety via the residue G (resulting from reaction of RCG1 with RCG2). "Oligopeptide", as used herein, relates to peptides of 3 to 50 amino acids, while "polypeptide" relates to peptides of more than 50 amino acids in length. In various embodiments, the polypeptide may be an antibody. The antibody may be monoclonal, polyclonal or multispecific. It may also be an antibody fragment. In various embodiments, it may also be a murine, chimeric, humanized or human antibody. The antibody may be a IgM, IgD, IgG (e.g. IgG1, IgG2, IgG3, IgG4), IgA (IgA1, IgA2) or IgE antibody or a hybrid form. Suitable antibodies encompass both conventional (full-length) antibodies and fragments thereof, as well as single domain antibodies and fragments thereof, in particular variable heavy chain of single domain antibodies. Fragments of (conventional) antibodies typically comprise a portion of an intact antibody, in particular the antigen binding region or variable region of the intact antibody, and retain the biological function of the conventional antibody. Examples of such fragments include Fv, Fab, F(ab')₂, Fab', dsFv, (dsFv)₂, scFv, sc(Fv)₂ and diabodies.

Antibody-like molecules may function similar to antibodies but are structurally no antibodies. Such molecules include, for example and without limitation, anticalins, aptamers and the like. They may chemically be peptides or include peptide moieties, but may also be non-peptide compounds, such as nucleic acids and derivatives thereof.

The Ab moiety may be a peptide or small molecule that may, in various embodiments, include amino acid moieties. Examples for such a compound are DUPA (Glu-urea-Glu; 2-[3-(1,3-dicarboxypropyl)ureido]pentanedioic acid) or EUK (Glu-urea-Lys). It is known that such Glu-ureido-based peptides target prostate specific membrane antigen (PSMA), an antigen expressed in certain prostate cancers. Other examples of small molecules that function as homing or targeting moieties include, amongst others, folic acid, HDAC (histone deacetylase) inhibitors, such as Givinostat, Panobinostat, and Vorinostat, KSP (kinesin spindle protein) inhibitors, such as 2-propylamino-2,4-diaryl-2,5-dihydropyrroles, ARRY-520, etc. It is known that folate receptors are overexpressed in a large number of tumors, rendering folic acid a suitable targeting moiety for targeting tumor cells.

Generally, the Ab moiety may direct the molecule to an antigen of choice. Such antigens include, for example, tumor-associated antigens (TAA), cell surface receptor proteins and other cell surface molecules, transmembrane proteins, signaling proteins, cell survival regulatory factors, cell proliferation regulatory factors, molecules associated with (for e.g., known or suspected to contribute functionally to) tissue development or differentiation, lymphokines, cytokines, molecules involved in cell cycle regulation, molecules involved in vasculogenesis and molecules associated with (for e.g., known or suspected to contribute functionally to) angiogenesis. The tumor-associated antigen may be a cluster differentiation factor (i.e., a CD protein). An antigen to which a compound/conjugate of the invention is capable of binding may be a member of a subset of one of the above-mentioned categories, wherein the other subset(s) of said category comprise other molecules/antigens that have a distinct characteristic (with respect to the antigen of interest).

Various polypeptide (antigen) targets, in particular TAAs, for the targeting moieties (Ab) of the present invention, in particular antibodies and antibody-like molecules, include, but are not limited to the following polypeptides CLL1; BMPR1B; E16; STEAP1; 0772P; MPF; NaPi2b; Sema 5b; PSCA hlg; ETBR; MSG783; STEAP2; TrpM4; CRIPTO; CD21; CD79b; FcRH2; HER2; NCA; MDP; IL20Rα; Brevican; EphB2R; ASLG659; PSCA; GEDA; BAFF-R; CD22; CD79a; CXCR5; HLA-DOB; P2X5; CD72; LY64; FcRH1; IRTA2; TENB2; PMEL17; TMEFF1; GDNF-Ra1; Ly6E; TMEM46; Ly6G6D; LGR5; RET; LY6K; GPR19; GPR54; ASPHD1; Tyrosinase; TMEM118; GPR172A; MUC16 and CD33. These targets and suitable antibodies, such as anti-CD33; anti-NaPi2b and anti-CD21 antibodies, are described in more detail in WO 2016/090050 A1, which is herein incorporated by reference in its entirety. Other suitable antibodies include those already approved and marketed as anti-cancer drugs, such as bevacizumab, rituximab, trastuzumab, gemtuzumab, alemtuzumab, cetuximab, ibritumomab, tositumomab, panitumumab, catumaxomab, ofatumumab, ipilimumab, and brentuximab vedotin.

The Ab moieties of the invention comprise a reactive group RCG2 or may be designed, engineered or synthesized to comprise such a reactive group orthogonal to the reactive group RCG1 present on the linker. In particular peptides, oligopeptides and polypeptides, such as antibodies, may be modified or designed to comprise such reactive groups, typically as side chains or amino acids that are easily accessible at the surface of the molecule.

Accordingly, in various embodiments, the compounds of the invention include antibody-drug conjugates comprising cysteine engineered antibodies where one or more amino acids of a wild-type or parent antibody are replaced with a cysteine amino acid. Any form of antibody may be so engineered, i.e. mutated. Mutants with replaced ("engineered") cysteine (Cys) residues are evaluated for the reactivity of the newly introduced, engineered cysteine thiol groups. The thiol reactivity value is a relative, numerical term in the range of 0 to 1.0 and can be measured for any cysteine engineered antibody. Thiol reactivity values of cysteine engineered antibodies may be in the ranges of 0.6 to 1.0; 0.7 to 1.0; or 0.8 to 1.0. To prepare a cysteine engineered antibody by mutagenesis, DNA encoding an amino acid sequence variant of the starting polypeptide is prepared by a variety of methods known in the art. These methods include, but are not limited to, preparation by site-directed (or oligonucleotide- mediated) mutagenesis, PCR mutagenesis, and cassette mutagenesis of an earlier prepared DNA encoding the polypeptide. Variants of recombinant antibodies may be constructed also by restriction fragment manipulation or by overlap extension PCR with synthetic oligonucleotides. Mutagenic primers encode the cysteine codon replacement(s). Standard mutagenesis techniques can be employed to generate DNA encoding such mutant cysteine engineered antibodies. General guidance can be found in Sambrook et al Molecular Cloning, A Laboratory Manual, Cold Spring Harbor Laboratory Press, Cold Spring Harbor, N.Y., 1989.

Cysteine amino acids may be engineered at reactive sites in an antibody and which do not form intrachain or intermolecular disulfide linkages (US 7521541; US 7723485; WO2009/052249). The engineered cysteine thiols may react with linker reagents or the linker-drug intermediates of the present invention which have thiol-reactive, electrophilic groups such as maleimide or alpha-halo amides to form ADC with cysteine engineered antibodies (ThioMabs) and the drug (D) moiety. The location of the drug moiety can thus be designed, controlled, and known. The drug loading can be controlled since the engineered cysteine thiol groups typically react with thiol-reactive linker reagents or linker-drug intermediates in high yield. Engineering an antibody to introduce a cysteine amino acid by substitution at a single site on the heavy or light chain gives two new cysteines on the symmetrical antibody. A drug loading near 2 can be achieved and near homogeneity of the conjugation product ADC. Cysteine engineered antibodies of the invention preferably retain the antigen binding capability of their wild type, parent antibody counterparts. Thus, cysteine engineered antibodies are capable of binding, preferably specifically, to antigens. Cysteine engineered antibodies may be prepared for conjugation with linker-drug intermediates by reduction and reoxidation of intrachain disulfide groups.

The present invention also encompasses the use of the cryptophycin compounds, derivatives and conjugates disclosed herein as a pharmaceutical, in particular the use of the conjugates of the present disclosure. The compounds, derivatives and conjugates for use as a pharmaceutical thus form one further aspect of the invention.

The cryptophycin compounds, derivatives and conjugates of the invention, in particular the conjugates, may be used as a pharmaceutical for treating cancer. The invention thus also covers methods for the treatment of cancer, typically in a subject in need thereof, by administrating an effective amount, typically a therapeutically effective amount, of the compounds, derivatives and conjugates disclosed herein.

As used herein, unless defined otherwise in a claim, the term "treatment" refers to alleviating the specified condition, eliminating or reducing one or more symptoms of the condition, slowing or eliminating the progression of the condition.

As used herein, unless defined otherwise in a claim, the term "effective amount" means that amount of a drug or pharmaceutical agent that will elicit the biological or medical response of a tissue, system, animal, or human that is being sought, for instance, by a researcher or clinician.

As used herein, unless defined otherwise in a claim, the term "therapeutically effective amount" means any amount which, as compared to a corresponding subject who has not received such amount, results in treatment of a disease, disorder, or side effect, or a decrease in the rate of advancement of a disease or disorder. The term also includes within its scope amounts effective to enhance normal physiological function. For use in therapy, therapeutically effective amounts of a compound/conjugates of the invention, as well as salts thereof, may be administered as the raw chemical. Additionally, the active ingredient may be presented as a pharmaceutical composition.

In still another aspect, the invention features a pharmaceutical composition comprising any one or more of the cryptophycin compounds, derivatives or conjugates disclosed herein, including pharmaceutically acceptable salts thereof, and a pharmaceutically acceptable excipient, diluent, stabilizer and/or carrier. Suitable diluents, carriers, excipients or stabilizers are known to those skilled in the art and for example described in Remington's Pharmaceutical Sciences (1980) 16th edition, Osol, A. Ed..

The phrase "pharmaceutically acceptable salt," as used herein, refers to pharmaceutically acceptable organic or inorganic salts of an antibody-drug conjugate (ADC) or a linker-cryptophycin moiety or the cryptophycin compounds disclosed herein. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis - (2-hydroxy-3- naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound.

Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion. Other salts, which are not pharmaceutically acceptable, may be useful in the preparation of compounds of this invention and these should be considered to form a further aspect of the invention. These salts, such as oxalic or trifluoroacetate, while not in themselves pharmaceutically acceptable, may be useful in the preparation of salts useful as intermediates in obtaining the compounds of the invention and their pharmaceutically acceptable salts.

Compounds, such as conjugates, of the present invention may exist in solid or liquid form. In the solid state, it may exist in crystalline or noncrystalline form, or as a mixture thereof.

The skilled artisan will appreciate that pharmaceutically acceptable solvates may be formed for crystalline or non- crystalline compounds. In crystalline solvates, solvent molecules are incorporated into the crystalline lattice during crystallization. Solvates may involve non-aqueous solvents such as, but not limited to, ethanol, isopropanol, DMSO, acetic acid, ethanolamine, or ethyl acetate, or they may involve water as the solvent that is incorporated into the crystalline lattice. Solvates wherein water is the solvent incorporated into the crystalline lattice are typically referred to as "hydrates." Hydrates include stoichiometric hydrates as well as compositions containing variable amounts of water. The invention includes all such solvates. The skilled artisan will further appreciate that certain compounds of the invention that exist in crystalline form, including the various solvates thereof, may exhibit polymorphism (i.e. the capacity to occur in different crystalline structures). These different crystalline forms are typically known as "polymorphs." The invention includes all such polymorphs. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. The skilled artisan will appreciate that different polymorphs may be produced, for example, by changing or adjusting the reaction conditions or reagents, used in making the compound. For example, changes in temperature, pressure, or solvent may result in polymorphs. In addition, one polymorph may spontaneously convert to another polymorph under certain conditions. Compounds of the present invention or a salt thereof may exist in stereoisomeric forms (e.g., it contains one or more asymmetric carbon atoms). The individual stereoisomers (enantiomers and diastereomers) and mixtures of these are included within the scope of the present invention.

Pharmaceutical formulations of therapeutic antibody-drug conjugates (ADC) of the invention are typically prepared for parenteral administration, i.e. bolus, intravenous, intratumor injection with a pharmaceutically acceptable parenteral vehicle and in a unit dosage injectable form. An antibody- drug conjugate (ADC) having the desired degree of purity is optionally mixed with pharmaceutically acceptable diluents, carriers, excipients or stabilizers (Remington's Pharmaceutical Sciences (1980) 16th edition, Osol, A. Ed.), in the form of a lyophilized formulation or an aqueous solution.

All documents cited herein are incorporated by reference in their entirety.

### EXAMPLES

The examples which follow describe the preparation of certain compounds in accordance with the invention. These examples serve only as means of illustration and not limitation.

### Example 1: Synthesis of cryptophycin compounds of the invention

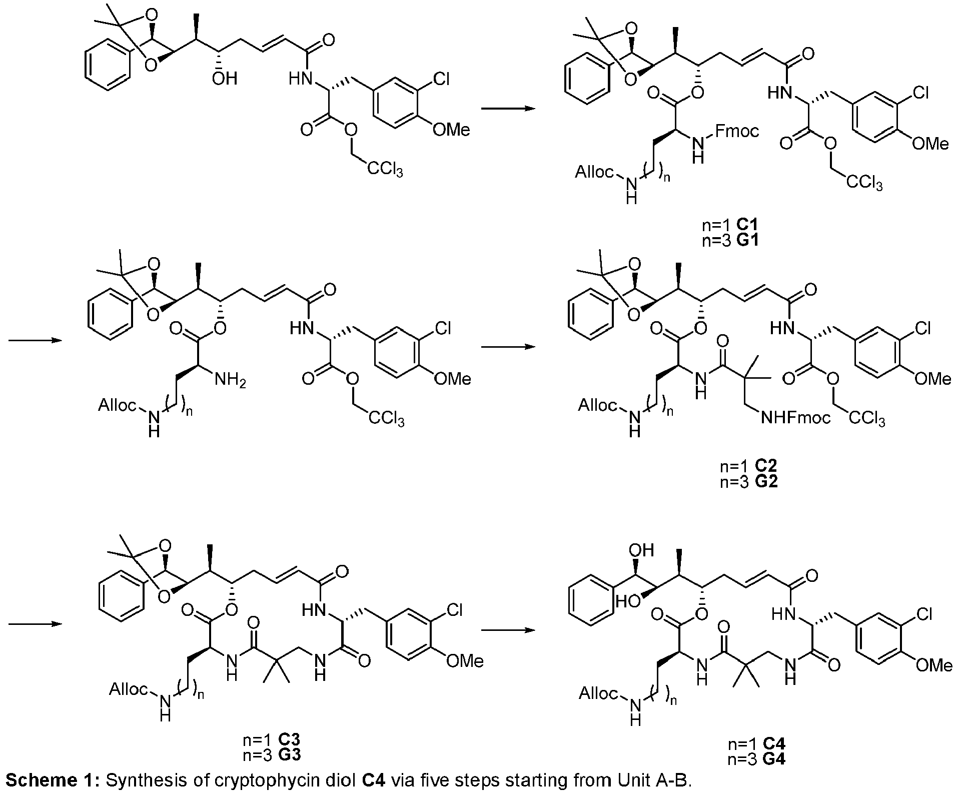

### Fmoc-uD[Dab(Alloc)]-uA[acetonide]-uB-OTCE C1

Building block A-B was synthesized according to Sewald *et al..* (N. Sewald et al., J. Org. Chem. 2010, 75, 6953-6960). Alloc = allyloxycarbonyl.

A solution of Fmoc-Dap(Alloc)-OH (517 mg, 1.22 mmol, 1.0 eq.), building block A-B (805 mg, 1.22 mmol, 1.0 eq.) and DMAP (27 mg, 0.22 mmol, 0.2 eq.) in abs. THF (19 mL) was stirred at 0 °C under argon. Triethylamine (340 µL, 2.45 mmol, 2.0 eq.) followed by 2,4,6-trichlorobenzoyl chloride (0.3 mL, 1.9 mmol, 1.6 eq.) were added. The solution was stirred for 4.5 h at 0 °C. A solution of citric acid (10 %, 50 mL) in water was added. The organic layer was separated, and the aqueous layer was extracted with EtOAc (3 x 50 mL). The organic layers were combined and dried over MgSO₄, then concentrated in vacuo. Column chromatography (d = 4 cm, I = 20 cm, PE/EtOAc 2:1) yielded **C1** a white foam (1.07 g, 1.00 mmol, 82 %).

| | | |
|---|---|---|
| **HRMS:** (ESI, +) | m/z (found) | 1090.2577 |
| | m/z (calc.) | 1090.2589 (M+Na⁺); (NaC₅₃H₅₇Cl₄N₃O₁₂⁺) |

**¹H NMR** (500 MHz, Chloroform-d) *δ* / *ppm =* 7.76 (d, ³*J* = 7.6 Hz, 2H, C^{ar}**H**), 7.60 (d, ³*J* = 7.5 Hz, 2H, C^{ar}**H**), 7.45-7.28 (m, 9H, C^{ar}**H**), 7.15 (s, 1H, uB-C^{car,2}**H**), 6.98 (d, ³*J* = 8.6 Hz, 1H, uB-C^{car,6}**H**), 6.80 (d, ³*J* = 8.5 Hz, 1H, uB-C^{ar,5}**H**), 6.52 (m, 1H, uA-C^{β}**H**), 6.28 (d, ³*J* = 7.8 Hz, 1H, uB-NH), 5.93 (m, 1H, uD-C**H**=CH₂), 5.63 (d, ³*J* = 7.5 Hz, 1H, uD-NH-Fmoc), 5.40 (d, ³*J* = 15.6 Hz, 1H, uA-C^{α}**H**), 5.37 (s (broad), 1H, uD-N**H**-Alloc), 5.31 (d, ³*J* = 17.1 Hz, 1H, uD-CH=C**H**₂*^{trans}*), 5.21 (d, ³*J* = 10.4 Hz, 1H, uD-CH=C**H**₂*^{cis}*), 4.98-4.90 (m, 2H, uB-C^{α}**H**, uA-C^{δ}**H**), 4.71 (d, ²*J* = 11.9 Hz, 1H, uB-CH^{A}H^{B}CCl₃), 4.68 (d, ³*J* = 8.7 Hz, 1H, uA-C^{η}**H**), 4.62 (d, ²*J* = 12.0 Hz, 1H, uB-CH^{A}**H**^{B}CCl₃), 4.57 (s (broad), 2H, uD-C**H**₂CH=CH₂), 4.38 (d, ³*J* = 7.2 Hz, 2H, uD-C**H**₂CH, Fmoc), 4.28 (m, 1H, uD-C^{α}**H**), 4.20 (t, ³*J* = 6.9 Hz, 1H, uD-CH₂C**H**, Fmoc), 3.87 (dd, ³*J* = 8.5 Hz, ³*J* = 8.1 Hz, 1H, uA-C^{ζ}**H**), 3.82 (s, 3H, uB-OC**H**₃), 3.42 (m, 1H, uD-C^{γ}**H**^{A}H^{B}), 3.14 (dd, ²*J* = 14.2 Hz, ³*J* = 5.9 Hz, 1H, uB-C^{β}**H**^{A}H^{B}), 3.08-2.95 (m, 2H, uD-C^{γ}H^{A}**H**^{B}, uB-C^{β}H^{A}**H**^{B}), 2.33 (m, 1H, uA-C^{γ}**H**^{A}H^{B}), 2.27 (m, 1H, uA-C^{γ}H^{A}**H**^{B}), 2.03-1.87 (m, 2H, uA-C^{ε}**H**, uD-C^{β}**H**^{A}H^{B}), 1.77 (m, 1H, uD-C^{β}H^{A}**H**^{B}), 1.52 (s, 3H, uA-C(C**H**₃)^{A}(CH₃)^{B}), 1.45 (s, 3H, uA-C(CH₃)^{A}(C**H**₃)^{B}), 1.10 (d, ³*J* = 7.1 Hz, 3H, uA-C^{ε}HC**H**₃).

**¹³C NMR** (126 MHz, Chloroform-d) *δ l ppm =* 171.5 (uD-**C**=O), 170.1 (uB-COOTce), 165.22 (**C**=O, Alloc), 165.16 (uB-CONH), 156.6 (**C**=O, Fmoc), 154.3 (uB-**C**⁴), 144.0 (**C**^{ar}), 143.6 (**C**^{ar}), 141.4 (**C**^{ar}), 139.4 (uA-**C**^{β}), 137.5 (**C**^{ar}), 133.0 (**C**=CH₂, Alloc), 131.3 (uB-**C**^{ar,2}), 129.0(**C**^{ar}), 128.8 (**C**^{ar}), 128.8 (**C**^{ar}), 128.6 (uB-**C**^{ar,6}), 127.9 (**C**^{ar}), 127.2 (**C**^{ar}), 125.7 (uA-**C**^{α}), 125.3 (**C**^{ar}), 125.2 (**C**^{ar}), 122.4 (**C**^{ar}), 117.9 (C=**C**H₂, Alloc), 112.3 (uB-**C**^{ar,5}), 109.4 (uA-**C**(CH₃)₂), 94.4 (uB-**C**Cl₃), 82.3 (uA-**C**^{ζ}), 80.8 (uA-**C**^{η}), 75.9 (uA-**C**^{δ}), 74.8 (uB-**C**H₂CCl₃), 67.3 (**C**H₂-CH, Fmoc), 65.8 (**C**H₂-CH=CH₂, Alloc), 56.3 (uB-O**C**H₃), 53.3 (uB-**C**^{α}), 52.1 (uD-**C**^{α}), 47.2 (CH₂=**C**H, Fmoc), 37.2 (uD-**C**^{γ}), 36.5 (uB-**C**^{β}), 35.7 (uA-**C**^{ε}), 33.0 (uA-**C**^{γ}), 32.5 (uD-**C**^{β}), 27.3 (uA-C(**C**H₃)^{A}(CH₃)^{B}), 27.1 (uA-C(CH₃)^{A}(**C**H₃)^{B}), 10.2 (uA-C^{ε}H**C**H₃).

### Fmoc-uD[Lys(Alloc)]-uA[acetonide]-uB-OTCE G1

Building block A-B was synthesized according to Sewald *et al..* (N. Sewald et al., J. Org. Chem. 2010, 75, 6953-6960). Alloc = allyloxycarbonyl.

A solution of Fmoc-Lys(Alloc)-OH (0.70 mg, 1.5 mmol, 1.5 eq.), building block A-B (0.67 g, 1.0 mmol, 1.0 eq.) and DMAP (27 mg, 0.22 mmol, 0.2 eq.) in abs. THF (19 mL) was stirred at 0 °C under argon. Triethylamine (340 µL, 2.45 mmol, 2.0 eq.) followed by 2,4,6-trichlorobenzoyl chloride (0.3 mL, 1.9 mmol, 1.6 eq.) were added. The solution was stirred for 4.5 h at 0 °C. A solution of citric acid (10 %, 50 mL) in water was added. The organic layer was separated, and the aqueous layer was extracted with EtOAc (3 x 50 mL). The organic layers were combined and dried over MgSO₄, then concentrated in vacuo. Column chromatography (d = 4 cm, I = 20 cm, PE/EtOAc 2:1) yielded **G1** as a white foam (1.10 g, 1.00 mmol, quant.).

**TLC:** R*_{f}* (PE/EtOAc 2:1) = 0.26.

**¹H NMR** (500 MHz, Chloroform-d) *δ*/*ppm* = 7.75 (d, ³*J* = 7.6 Hz, 2H, C^{ar}**H**), 7.63-7.54 (m, 2H, C^{ar}**H**), 7.43-7.38 (m, 2H, C^{ar}**H**), 7.37-7.27 (m, 7H, C^{ar}**H**), 7.10 (s, 1H, uB-C^{ar,2}**H**), 6.95 (d, ³*J* = 8.4 Hz, 1H, uB-C ^{ar,6}**H**), 6.89 (d, ³*J* = 7.7 Hz, 1H, N**H**), 6.75 (d, ³*J* = 8.4 Hz, 1H, uB-C^{ar,5}**H**), 6.55 (ddd, ³*J* = 14.5 Hz, ³*J* = 6.4 Hz, ³*J* = 6.4 Hz, 1H, uA-C^{β}**H**), 5.92 (m, 1H, uD-CH=CH₂), 5.61 (d, ³*J* = 15.6 Hz, 1H, uA-C^{α}**H**), 5.56 (d, ³*J* = 7.8 Hz, 1H, N**H**), 5.28 (d, ³*J* = 17.0 Hz, 1H, uD-CH=C**H**₂*^{trans}*), 5.19 (d, ³*J* = 10.5 Hz, 1H, uD-CH=C**H**₂*^{cis}*), 5.01-4.91 (m, 2H, uB-C^{α}**H**, uA-C^{δ}**H**, NH), 4.71 (d, ³*J*=8.7Hz, 1H, uA-C^{η}**H**), 4.62 (d, ²*J*= 12.0 Hz, 1H, uB-C**H**^{A}H^{B}CCl₃), 4.58-4.51 (m, 3H, uB-CH^{A}**H**^{B}CCl₃, uD-C**H**₂CH=CH₂), 4.38 (m, 1H, uD-C**H**^{A}H^{B}CH, Fmoc), 4.22 (m, 1H, uD-CH^{A}**H**^{B}CH, Fmoc), 4.20-4.12 (m, 2H, uD-CH₂C**H** Fmoc, uD-C^{α}**H**), 3.84 (m, 1H, uA-C^{ζ}**H**), 3.79 (s, 3H, uB-OC**H**₃), 3.24-3.13 (m, 2H, uD-C^{γ}**H**₂), 3.08 (dd, ²*J* = 14.3 Hz, ³*J* = 5.5 Hz, 1H, uB-C^{β}**H**^{A}H^{B}), 2.93 (dd, ²*J* = 14.4 Hz, ³*J* = 6.9 Hz, 1H, uB-C^{β}H^{A}**H**^{B}), 2.37 (m, 1H, uA-C^{γ}**H**^{A}H^{B}), 2.29 (m, 1H, uA-C^{γ}H^{A}**H**^{B}), 1.98 (m, 1H, uA-C^{ε}**H**), 1.74 (m, 1H, uD-C^{β}**H**^{A}H^{B}), 1.66 (m, 1H, uD-C^{β}H^{A}**H**^{B}), 1.55-1.48 (m, 5H, uA-C(C**H**₃)^{A}(CH₃)^{B}, uD-C^{δ}**H**₂), 1.46 (s, 3H, uA-C(CH₃)^{A}(C**H**₃)^{B}),1.42-1.35 (m, 2H, uD-C^{δ}**H**₂), 1.09 (d, ³*J* = 6.9 Hz, 3H, uA-C^{ε}HC**H**₃).

¹³**C NMR** (126 MHz, Chloroform-d) *δ*/*ppm =* 172.0 (uD-**C**=O), 169.8 (uB-COOTce), 166.4 (uD-Alloc-**C**=O), 157.0 (uB-CONH), 156.5 (Fmoc-**C**=O, Fmoc), 154.2 (uB-**C**^{ar,4}), 143.6 (**C**^{ar}), 141.4 (**C**^{ar}), 140.4 (uA-**C**^{β}), 137.5 (**C**^{ar}), 132.8 (uD-Alloc-**C**=CH₂), 131.4 (uB-**C**^{ar,2}), 128.9 (**C**^{ar}), 128.7 (**C**^{ar}), 128.6 (uB-**C**^{ar,6}), 127.9 (**C**^{ar}), 127.2 (**C**^{ar}), 127.1 (**C**^{ar}), 125.3 (**C**^{ar}), 125.2 (uA-**C**^{α}), 124.8 (**C**^{ar}), 122.3 (**C**^{ar}), 120.1(**C**^{ar}), 118.0 (uD-Alloc-C=**C**H₂), 112.2 (uB-**C**^{ar,5}), 109.2 (uA-**C**(CH₃)₂), 94.3 (uB-**C**Cl₃), 82.6 (uA-**C**^{ζ}), 80.8 (uA-**C**^{η}), 75.4 (uA-**C**^{δ}), 74.7 (uB-**C**H₂CCl₃), 67.5 (Fmoc-**C**H₂-CH), 65.2 (uD-Alloc-**C**H₂-CH=CH₂), 56.2 (uB-O**C**H₃), 54.3 (uD-**C**^{α}), 53.4 (uB-**C**^{α}), 47.1 (Fmoc-CH₂**C**H, Fmoc), 40.3 (uD-**C**^{ε}), 36.5 (uB-**C**^{β}), 36.3 (uA-**C**^{ε}), 32.1 (uA-**C**^{γ}), 31.0 (uD-**C**^{β}), 29.6 (uD-**C**^{δ}), 27.3 (uA-C(**C**H₃)^{A}(CH₃)^{B}), 27.2 (uA-C(CH₃)^{A}(**C**H₃)^{B}),22.5 (uD-**C**^{γ}), 9.6 (uA-C^{ε}H**C**H₃).

### Fmoc-uC-uD[Dab(Alloc)]-uA[acetonide]-uB-OTce C2

Piperidine (0.50 mL, 5.0 mmol, 5.1 eq.) was added to a solution of **C1** (1.05 g, 0.98 mmol) in DMF (30 mL) at 0 °C. After 30 minutes stirring at room temperature the solvent was evaporated. The resulting colorless oil was co-evaporated with toluene, then dissolved in DMF (10 mL). Fmoc-3-amino-2,2-dimethyl-propionic acid (682 mg, 2.01 mmol, 2.0 eq.), HOAt (319 mg, 2.28 mmol, 2.3 eq.) and D*i*PEA (0.90 mL, 5.3 mmol, 5.4 eq.) were dissolved in dichloromethane (40 mL) and DIC (0.35 mL, 2.30 mmol, 2.3 eq.) was added at 0 °C over 10 minutes and stirred for additional 10 minutes. The DMF solution was added. After stirring at RT for 17.5 h the solution was given to a solution of citric acid (10 %, 100 mL) in water. The organic layer was separated, and the aqueous layer was extracted with EtOAc (3 x 50 mL). The organic layers were combined washed with sodium bicarbonate solution (10 %, 50 mL) and brine (50 mL) and dried over MgSO₄, then concentrated in vacuo. Column chromatography (d = 3.5 cm, I = 22 cm, PE/EtOAc 2:1 →1:1) yielded **CD49** as a white foam (0.50 g, 0.42 mmol, 43 %).

| | | |
|---|---|---|
| **HRMS:** (ESI, +) | m/z (found) | 1189.3286 |
| | m/z (calc.) | 1189.3273 (M+Na⁺); (NaC₅₈H₆₆Cl₄N₄O₁₃⁺) |

**¹H NMR** (500 MHz, Chloroform-d) *δ*/*ppm* = 7.75 (d, ³*J* = 7.4 Hz, 2H, C^{ar}**H**), 7.61 (d, ³*J* = 7.5 Hz, 2H, C^{ar}**H**), 7.43-7.27 (m, 9H, C^{ar}**H**), 7.17 (d, ⁴*J* = 2.3 Hz, 1H, uB-C^{ar,2}**H**), 7.07-7.00 (m, 1H, uB-C^{ar,6}**H**), 6.82 (d, ³*J* = 8.5 Hz, 1H, uB-C^{ar, 5}**H**), 6.78 (s, 1H, uD-N**H**-uC), 6.63 (d, ³*J* = 7.8 Hz, 1H, uB-N**H**), 6.54 (ddd, ³*J* = 14.8 Hz, ³*J* = 6.8 Hz, ³*J* = 6.8 Hz, 1H, uA-C^{β}**H**), 5.88 (ddt, ³*J* = 16.3 Hz, ³*J* = 10.6 Hz, ³*J* = 5.6 Hz, 1H, uD-C**H**=CH₂), 5.80 (s, 1H, uC-N**H**-Fmoc), 5.47 (d, ³*J* = 14.9 Hz, 1H, uA-C^{α}**H**), 5.47 (s (broad), 1H, uD-N**H**-Alloc), 5.29 (d, ³*J* = 16.9 Hz, 1H, uD-CH=C**H**₂*^{trans}*), 5.18 (d, ³*J* = 10.5 Hz, 1H, uD-CH=C**H**₂*^{cis}*), 5.02-4.91 (m, 2H, uA-C^{δ}**H**, uB-C^{α}**H**), 4.79 (d, ²*J* = 11.8 Hz, 1H, C**H**^{A}H^{B}CCl₃), 4.68 (d, ³*J* = 8.7 Hz, 1H, uA-C^{η}**H**), 4.68 (d, ²*J* = 11.8 Hz, 1H, CH^{A}**H**^{B}CCl₃), 4.55 (d, ³*J* = 5.7 Hz, 2H, uD-CH₂CH=CH₂), 4.49 (m, 1H, uD-C^{α}**H**), 4.43-4.28 (m, 2H, uD-C**H**^{A}**H**^{B}CH, Fmoc), 4.20 (dd, ³*J* = 7.4 Hz, ³*J* = 7.4 Hz, 1H, CH₂C**H**, Fmoc), 3.87 (dm, ³*J* = 9.7 Hz, 1H, uA-C^{ζ}**H**), 3.85 (s, 3H, uB-OC**H**₃), 3.41 (m, 1H, uD-C^{γ}**H**^{A}H^{B}), 3.37-3.30 (m, 2H, uC-C^{β}**H**₂), 3.19 (dd, ²*J* = 14.3 Hz, ³*J* = 5.8 Hz, 1H, uB-C^{β}**H**^{A}H^{B}), 3.12-2.99 (m, 2H, uD-C^{γ}H^{A}**H**^{B}, uB-C^{β}H^{A}**H**^{B}), 2.41-2.18 (m, 2H, uA-C^{γ}**H**₂), 2.01-1.90 (m, 3H, uD-C^{β}**H**₂, uA-C^{ε}**H**), 1.54 (s, 3H, uA-C(C**H**₃)^{A}(CH₃)^{B}), 1.47 (s, 3H, uA-C(CH₃)^{A}(C**H**₃)^{B}), 1.25 (s, 3H, uC-C(C**H**₃)^{A}(CH₃)^{B}), 1.20 (s, 3H, uC-C(CH₃)^{A}(C**H**₃)^{B}), 1.12 (d, ³*J* = 6.9 Hz, 3H, uA-C^{ε}HC**H**₃).

¹³**C NMR** (126 MHz, Chloroform-d) *δ*/*ppm* = 177.8 (uC-**C**=O), 172.0 (uD-**C**=O), 170.2 (uB-**C**OOTce), 165.3 (, uD-**C**=O Alloc), 157.2 (**C**=O, Fmoc), 156.8 (uB-CONH), 154.2 (uB-**C**⁴), 144.2 (**C**^{ar}), 144.1 (**C**^{ar}), 141.4 (**C**^{ar}), 139.2 (uA-**C**^{β}), 137.4 (**C**^{ar}), 132.8 (**C**=CH₂, Alloc), 131.4 (uB-**C**²), 129.1 (**C**^{ar}), 129.0 (**C**^{ar}), 128.8 (uB-**C**⁶), 128.6 (**C**^{ar}), 127.8 (**C**^{ar}), 127.2 (**C**^{ar}), 127.1 (**C**^{ar}), 125.8 (uA-**C**^{α}), 125.3 (**C**^{ar}), 122.3 (**C**^{ar}), 120.1 (**C**^{ar}), 118.0 (**C**=CH₂, Alloc), 112.3 (uB-**C**⁵), 109.4 (uA-**C**(CH₃)₂), 94.5 (uB-**C**Cl₃), 82.2 (uA-**C**^{ζ}), 80.8 (uA-**C**^{η}), 76.1 (uA-**C**^{δ}), 74.7 (uB-**C**H₂CCl₃), 66.9 (**C**H₂-CH, Fmoc), 66.0 (**C**H₂-CH=CH₂, Alloc), 56.3 (uB-O**C**H₃), 53.5 (uB-**C**^{α}), 50.5 (uD-**C**^{α}), 49.7 (uC-**C**^{β}), 47.4 (CH₂=**C**H, Fmoc), 43.7 (uC-**C**^{α}), 37.1 (uD-**C**^{γ}), 36.5 (uB-**C**^{β}), 35.8 (uA-**C**^{ε}), 33.1 (uA-**C**^{γ}), 31.4 (uD-**C**^{β}), 27.4 (uA-C(**C**H₃)^{A}(CH₃)^{B}), 27.2 (uA-C(CH₃)^{A}(**C**H₃)^{B}), 23.6 (uC-C(**C**H₃)^{A}(CH₃)^{B}), 23.0 (uC-C(**C**H₃)^{A}(CH₃)^{B}), 10.4 (uA-C^{ε}H**C**H₃).

### Fmoc-uC-uD[Lys(Alloc)]-uA[acetonide]-uB-OTce G2

Piperidine (0.50 mL, 5.0 mmol, 5.0 eq.) was added to a solution of **G1** (1.1 g, 1.0 mmol) in DMF (30 mL) at 0 °C. After 30 minutes stirring at room temperature, the solvent was evaporated. The resulting colorless oil was co-evaporated with toluene, then dissolved in DMF (10 mL). Fmoc-3-amino-2,2-dimethyl-propionic acid (523 mg, 1.5 mmol, 1.5 eq.), HOAt (231 mg, 1.7 mmol, 1.65 eq.) and D*i*PEA (0.90 mL, 5.1 mmol, 5 eq.) were dissolved in dichloromethane (40 mL) and DIC (0.26 mL, 1.7 mmol, 1.65 eq.) was added at 0 °C over 10 minutes and stirred for additional 10 minutes. The DMF solution was added. After stirring at RT for 17.5 h the solution was given to a solution of citric acid (10 %, 100 mL) in water. The organic layer was separated, and the aqueous layer was extracted with EtOAc (3 x 50 mL). The organic layers were combined washed with sodium bicarbonate solution (10 %, 50 mL) and brine (50 mL) and dried over MgSO₄, then concentrated in vacuo. Column chromatography (PE/EtOAc 2:1→1:1) yielded **gb247** as a white foam (0.57 g, 0.48 mmol, 48 %).

| | | |
|---|---|---|
| **MS:** (ESI, +) | m/z (found) | 1217.4 |
| | m/z (calc.) | 1217.4 (M+Na⁺); (NaC₆₀H₇₀Cl₄N₄O₁₃⁺) |

**¹H NMR** (600 MHz, Chloroform-d) *δ*/*ppm =* 7.76 (d, ³*J* = 7.5 Hz, 2H, C^{ar}**H**), 7.60 (d, ³*J* = 7.5 Hz, 2H, C^{ar}**H**), 7.41-7.36 (m, 2H, C^{ar}**H**), 7.32-7.23 (m, 7H, C^{ar}**H**), 7.17 (d, ⁴*J* = 2.1 Hz, 1H, uB-C^{ar,2}**H**), 7.04 (dd, ³*J* = 8.4 Hz, ⁴*J* = 2.1 Hz, 1H, uB-C ^{ar,6}**H**),6.99 (d, ³*J* = 7.7 Hz, 1H, N**H**), 6.80 (d, ³*J* = 8.4 Hz, 1H, uB-C ^{ar,5}**H**), 6.57 (m, 1H, uA-C^{β}**H**), 6.34 (d, ³*J* = 6.4Hz, 1H, N**H**), 5.88 (ddt, ³*J*=16.2Hz, ³*J*=10.7Hz, ³*J* = 5.5 Hz, 1H, uD-C**H**=CH₂), 5.80 (s, 1H, N**H**), 5.68 (d, ³*J* = 15.6 Hz, 1H, uA-C^{α}**H**), 5.28 (d, ³*J* = 16.4 Hz, 1H, uD-CH=C**H**₂*^{trans}*), 5.19 (d, ³*J* = 10.4 Hz, 1H, uD-CH=C**H**₂*^{cis}*), 5.04-4.95 (m, 2H, uA-C^{δ}**H**, uB-C^{α}**H**), 4.91 (m, 1H, NH), 4.79 (d, ²*J* = 11.8 Hz, 1H, C**H**^{A}H^{B}CCl₃), 4.71-4.64 (m, 2H, uA-C^{η}**H**, CH^{A}**H**^{B}CCl₃), 4.53 (d, ³*J* = 5.2 Hz, 2H, uD-C**H**₂CH=CH₂), 4.39-4.28 (m, 3H, uD-Fmoc-C**H**₂CH, uD-C^{α}**H**), 4.18 (m, 1H, Fmoc-CH₂C**H**), 3.84 (s, 3H, uB-OC**H**₃), 3.80 (dd, ³*J* = 8.7 Hz, ³*J* = 2.9 Hz, 1H, uA-C^{ζ}**H**),, 3.41 (m, 1H, uD-C^{γ}**H**^{A}H^{B}), 3.40-3.27 (m, 2H, uC-C^{β}**H**₂), 3.22-3.12 (m, 3H, uB-C^{β}**H**^{A}H^{B}, uD-C^{ε}**H**₂), 3.02 (dd, ²*J* = 14.1 Hz, ³*J* = 7.4 Hz, uB-C^{β}H^{A}**H**^{B}), 2.44-2.30 (m, 2H, uA-C^{γ}**H**₂), 1.98 (m, 3H, uA-C^{ε}**H**), 1.78 (m, 1H, uD-C^{β}**H**^{A}H^{B}), 1.70 (m, 1H, uD-C^{β}H^{A}**H**^{B}), 1.52-1.47 (m, 5H, uA-C(C**H**₃)^{A}(CH₃)^{B}, uD-C^{δ}**H**₂), 1.43 (s, 3H, uA-C(CH₃)^{A}(C**H**₃)^{B}), 1.38 (m, 2H, uD-C^{γ}**H**₂), 1.23 (s, 3H, uC-C(C**H**₃)^{A}(CH₃)^{B}), 1.15 (s, 3H, uC-C(CH₃)^{A}(C**H**₃)^{B}), 1.10 (d, ³*J* = 6.9 Hz, 3H, uA-C^{ε}HC**H**₃).

¹³**C NMR** (151 MHz, Chloroform-d) *δ*/*ppm =* 177.6 (uC-**C**=O), 172.3 (uD-**C**=O), 170.3 (uB-**C**OOTce), 165.7 (uD-Alloc-**C**=O), 157.2 (Fmoc-**C**=O), 156.9 (uB-**C**ONH), 154.2 (uB-**C**^{ar,4}), 144.2 (**C**^{ar}), 141.5 (**C**^{ar}), 141.4 (C^{ar}), 139.2 (uA-**C**^{β}), 137.6 (**C**^{ar}), 133.0 (Alloc-**C**=CH₂), 131.4 (uB-**C**^{ar,2}), 128.9 (**C**^{ar}), 128.7 (**C**^{ar}), 128.6 (uB-**C**^{ar,6}), 127.8 (**C**^{ar}), 127.2 (**C**^{ar}), 127.0 (**C**^{ar}), 125.5 (**C**^{ar}), 125.4 (uA-**C**^{α}), 122.3 (**C**^{ar}), 120.1 (**C**^{ar}), 117.9 (Alloc-C=**C**H₂), 112.3 (uB-**C**^{ar,5}), 109.1 (uA-**C**(CH₃)₂), 94.5 (uB-**C**Cl₃), 82.6 (uA-**C**^{ζ}), 80.8 (uA-**C**^{η}), 75.8 (uA-**C**^{δ}), 74.7 (uB-**C**H₂CCl₃), 67.0 (Fmoc-**C**H₂-CH), 65.7 (Alloc-**C**H₂-CH=CH₂), 56.3 (uB-O**C**H₃), 53.5 (uB-**C**^{α}), 53.1 (uD-**C**^{α}), 47.4 (Fmoc-CH₂**C**H, Fmoc), 43.7 (uC-**C**^{β}), 41.5 (uC-**C**^{α}), 40.2 (uD-**C**^{ε}) 36.6 (uB-**C**^{β}), 36.3 (uA-**C**^{ε}), 32.1 (uA-**C**^{γ}), 29.8 (uD-**C**^{β}), 27.4 (uA-C(**C**H₃)^{A}(CH₃)^{B}), 27.2 (uA-C(**C**H₃)^{A}(**C**H₃)^{B}), 27.1 (uD-**C**^{δ}), 23.6 (uC-C(**C**H₃)^{A}(CH₃)^{B}), 22.8 (uC-C(**C**H₃)^{A}(CH₃)^{B}), 22.5 (uD-**C**^{γ}), 9.6 (uA-C^{ε}H**C**H₃).

### Cryptophycin-[uA- acetonide]-[uD-Dab(Alloc)] C3

Piperidine (0.20 mL, 2.0 mmol, 5 eq.) was added to a solution of seco-depsipeptide **C2** (463 mg, 0.40 mmol) in DMF (12 mL) at 0 °C. The cooling bath was removed, and the reaction stirred at room temperature for 16 hours. Ethyl acetate (200 mL) and water (200 mL) was added to the reaction solution.

The organic layer was isolated, and the aqueous layer was extracted with EtOAc (3 x 200 mL). The organic layers were dried over MgSO₄, then concentrated in vacuo. Column chromatography (d= 2 cm, I = 21 cm, 100 % EtOAc) yielded **C3** as a white foam (278 mg, 0.35 mmol, 87 %).

| | | |
|---|---|---|
| **HRMS:** (ESI, +) | m/z (found) | 819.3339 |
| | m/z (calc.) | 819.3342 (M+Na⁺); (NaC₄₁H₅₃ClN₄O₁₀⁺) |

**¹H NMR** (500 MHz, Chloroform-d) *δ*/*ppm* = 7.48-7.30 (m, 5H, uA-C^{ar}**H**), 7.17 (s, 1H, uB-C^{ar,2}**H**), 7.03 (d, ³*J* = 8.4 Hz, 1H, uB-C^{ar,6}**H**), 6.85 (d, ³*J* = 8.4 Hz, 1H, uB-C^{ar,5}**H**), 6.62 (m, 1H, uA-C^{β}**H**), 6.42-6.30 (m, 2H, uC-C^{α}-N**H**, uC-N**H**), 5.92 (ddt, ³*J* = 16.6 Hz, ³*J* = 11.0 Hz, ³*J* = 5.7 Hz, 1H, uD-C**H**=CH₂), 5.61 (d, ³*J* = 15.0 Hz, 1H, uA-C^{α}**H**), 5.50 (s (broad), 1H, uD-N**H**-Alloc), 5.44 (d, ³*J* = 6.5 Hz, 1H, uB-N**H**), 5.31 (d, ³*J* = 17.2 Hz, 1H, uD-CH=C**H**₂*^{trans}*), 5.22 (d, ³*J* = 10.4 Hz, 1H, uD-CH=C**H**₂*^{cis}*), 5.13 (m, 1H, uA-C^{δ}**H**), 4.69 (d, ³*J* = 8.9 Hz, 1H, uA-C^{η}**H**), 4.64-4.51 (m, 3H, uD-C**H**₂CH=CH₂, uB-C^{α}**H**), 4.38 (ddd, ³*J* = 8.3 Hz, ³*J* = 8.3 Hz, ³*J* = 7.3 Hz, 1H, uD-C^{α}**H**), 3.88 (s, 3H, uB-OC**H**₃), 3.79 (d, ³*J* = 8.8 Hz, 1H, uA-C^{ζ}**H**), 3.53-3.33 (m, 2H, uC-C^{β}**H**^{A}H^{B}, uD-C^{γ}**H**^{A}H^{B}), 3.28 (d, ²*J* = 12.6 Hz, 1H, uC-C^{β}H^{A}**H**^{B}), 3.10 (dd, ²*J* = 14.7 Hz, ³*J* = 5.0 Hz, 1H, uB-C^{β}**H**^{A}H^{B}), 2.93-2.86 (m, 2H, uD-C^{γ}H^{A}**H**^{B}, uB-C^{β}H^{A}**H**^{B}), 2.39 (dm, ²*J* = 14.1 Hz, 1H, uA-C^{γ}**H**^{A}H^{B}), 2.17 (ddd, ²*J* = 12.9, ³*J* = 12.5 Hz, ³*J* = 12.5 Hz, 1H, uA-C^{γ}H^{A}**H**^{B}), 1.86 (dd, ³*J* = 6.8 Hz, ³*J* = 6.8 Hz, 1H, uA-C^{ε}**H**), 1.78 (s (broad), 1H, uD-C^{β}**H**^{A}H^{B}), 1.56 (s (broad), 1H, uD-C^{β}H^{A}**H**^{B}), 1.52 (s, 3H, uA-C(CH₃)^{A}(C**H**₃)^{B}), 1.46 (s, 3H, uA-C(CH₃)^{A}(C**H**₃)^{B}), 1.21 (s, 3H, uC-C(C**H**₃)^{A}(CH₃)^{B}), 1.15 (s, 3H, uC-C(C**H**₃)^{A}(CH₃)^{B}), 1.12 (d, ³*J* = 7.1 Hz, 3H, uA-C^{ε}HC**H**₃).

¹³**C NMR** (126 MHz, Chloroform-d) *δ*/*ppm* = 178.1 (uC-**C**=O), 172.0 (uD-**C**OO), 170.9 (uB-**C**ONH-uC), 164.9 (uA-**C**ONH-uB), 156.2 (**C**=O, Alloc), 154.1 (uB-**C**^{ar,4}), 142.2 (uA-**C**^{β}), 137.4 (**C**^{ar}), 133.0 (**C**=CH₂, Alloc), 130.9 (uB-**C**^{ar,2}), 129.6 (**C**^{ar}), 128.9 (**C**^{ar}), 128.7 (**C**^{ar}), 128.2 (uB-**C**^{ar,6}), 126.8 (**C**^{ar}), 124.8 (uA-**C**^{α}), 122.5 (**C**^{ar}), 117.8 (C=**C**H₂, Alloc), 112.4 (uB-**C**^{ar,5}), 109.3 (uA-**C**(CH₃)₂), 82.5 (uA-**C**^{ζ}), 80.4 (uA-**C**^{η}), 75.5 (uA-**C**^{δ}), 65.7 (**C**H₂-CH=CH₂, Alloc), 56.2 (uB-O**C**H₃), 55.0 (uB-**C**^{α}), 50.0 (uD-**C**^{α}), 47.0 (uC-**C**^{β}), 47.0 (uC-**C**^{α}), 37.0 (uD-**C**^{γ}), 36.6, (uA-**C**^{ε}), 36.0 (uA-**C**^{γ}), 35.7 (uB-**C**^{β}), 33.0 (uD-**C**^{β}), 27.3 (uA-C(**C**H₃)^{A}(CH₃)^{B}), 27.1 (uA-C(**C**H₃)^{A}(CH₃)^{B}), 25.2 (uC-C(**C**H₃)^{A}(CH₃)^{B}), 21.8 (uC-C(**C**H₃)^{A}(**C**H₃)^{B}), 9.7 (uA-C^{ε}H**C**H₃).

### Cryptophycin-[uA-acetonide]-[uD-Lys(Alloc)] G3

Piperidine (0.23 mL, 2.4 mmol, 5 eq.) was added to a solution of seco-depsipeptide **G2** (0.57 g, 0.48 mmol) in DMF (12 mL) at 0 °C. The cooling bath was removed, and the reaction stirred at room temperature for 16 hours. Ethyl acetate (200 mL) and water (200 mL) was added to the reaction solution. The organic layer was isolated, and the aqueous layer was extracted with EtOAc (3 x 200 mL). The organic layers were dried over MgSO₄, then concentrated in vacuo. Column chromatography (d = 2 cm, I = 21 cm, PE/EtOAc 2:1 → 0:1) yielded **G3** as a white foam (0.33 g, 0.39 mmol, 81 %).

| | | |
|---|---|---|
| **MS:** (ESI, +) | m/z (found) | 847.4 |
| | m/z (calc.) | 847.5 (M+Na⁺); (NaC₄₃H₅₇ClN₄O₁₀⁺) |

**TLC:** R*_{f}* (EtOAc) = 0.60.

**¹H NMR** (600 MHz, Chloroform-d) *δ*/*ppm* = 7.40-7.31 (m, 5H, uA-C^{ar}**H**), 7.17 (d, ⁴*J* = 2.1 Hz, 1H, uB-C^{ar,2}**H**), 7.03 (dd, ³*J* = 8.4 Hz, ⁴*J* = 2.2 Hz, 1H, uB-C^{ar,6}**H**), 6.84 (d, ³*J* = 8.4 Hz, 1H, uB-C^{ar,5}**H**), 6.76 (s, 1H, N**H**), 6.57 (ddd, ³*J* = 15.1 Hz, ³*J* = 11.1 Hz, ³*J* = 4.1 Hz, 1H, uA-C^{β}**H**), 6.19 (m, 1H, N**H**), 5.90 (ddt, ³*J* = 16.4 Hz, ³*J* = 10.8 Hz, ³*J* = 5.6 Hz, 1H, uD-C**H**=CH₂), 5.62 (d, ³*J* = 14.6 Hz, 1H, uA-C^{α}**H**), 5.31 (d, ³*J* = 17.2 Hz, ²*J* = 1.6 Hz, 1H, uD-CH=C**H**₂*^{trans}*), 5.21 (d, ³*J* = 10.4 Hz, ²*J* = 1.4 Hz, 1H, uD-CH=C**H**₂*^{cis}*), 5.11 (m, 2H, uA-C^{δ}**H**, N**H**), 4.83 (s, 1H, N**H**), 4.70 (d, ³*J* = 8.7 Hz, 1H, uA-C^{η}**H**), 4.61 (m, 1H, uB-C^{α}**H**), 4.55 (d, ³*J* = 5.6 Hz, uD-C**H**₂CH=CH₂), 4.28 (m, 1H, uD-C^{α}H), 3.87 (s, 3H, uB-OC**H**₃), 3.76 (dd, ³*J* = 8.8 Hz, ³*J* = 2.5 Hz, 1H, uA-C^{ζ}**H**), 3.43-3.25 (m, 2H, uC-C^{β}**H**₂), 3.19-3.06 (m, 3H, uB-C^{β}**H**^{A}H^{B}, uD-C^{ε}**H**₂), 2.91 (m, 1H, uB-C^{β}H^{A}**H**^{B}), 2.39 (m, 1H, uA-C^{γ}**H**^{A}H^{B}), 2.15 (m, 1H, uA-C^{γ}H^{A}**H**^{B}), 1.86 (m, 1H, uA-C^{ε}**H**), 1.58-1.52 (m, 3H, uD-C^{β}**H**^{A}H^{B}, uD-C^{δ}**H**₂), 1.49 (s, 3H, uA-C(CH₃)^{A}(C**H**₃)^{B}), 1.46 (s, 3H, uA-C(CH₃)^{A}(C**H**₃)^{B}), 1.28-1.23 (m, 3H, uD-C^{β}H^{A}**H**^{B}, uD-C^{δ}**H**₂), 1.20 (s, 3H, uC-C(C**H**₃)^{A}(CH₃)^{B}), 1.15 (s, 3H, uC-C(C**H**₃)^{A}(CH₃)^{B}), 1.12 (d, ³*J* = 7.1 Hz, 3H, uA-C^{ε}HC**H**₃).
¹³**C NMR** (151 MHz, Chloroform-d) *δ*/*ppm =* 172.3 (uC-**C**=O), 164.8 (uD-**C**OO), 162.6 (uB-**C**ONH-uC), 162.4 (uA-**C**ONH-uB), 156.6 (**C**=O, Alloc), 154.3 (uB-**C**^{ar,4}), 142.4 (uA-**C**^{β}), 137.7 (**C**^{ar}), 133.0 (**C**=CH₂, Alloc), 130.9 (uB-**C**^{ar,2}), 129.5 (**C**^{ar}), 128.9 (**C**^{ar}), 128.8 (**C**^{ar}), 128.2 (uB-**C**^{ar,6}), 126.8 (**C**^{ar}), 124.8 (uA-**C**^{α}), 122.8 (**C**^{ar}), 117.9 (C=**C**H₂, Alloc), 112.6 (uB-**C**^{ar,5}), 109.2 (uA-**C**(CH₃)₂), 82.7 (uA-**C**^{ζ}), 80.4 (uA-**C**^{η}), 75.2 (uA-**C**^{δ}), 65.7 (uD-**C**H₂-CH=CH₂), 56.3 (uB-O**C**H₃), 54.9 (uB-**C**^{α}), 51.9 (uD-**C**^{α}), 47.1 (uC-**C**^{β}), 43.6 (uC-**C**^{α}), 40.5 (uD-C^{ε}H₂), 37.0 (uA-**C**^{ε}), 35.8 (uA-**C**^{γ}), 35.7 (uB-**C**^{β}), 32.1 (uD-**C**^{γ}), 32.0 (uD-**C**^{β}), 29.8 (uD-**C**^{δ}), 27.3 (uA-C(**C**H₃)^{A}(CH₃)^{B}), 27.2 (uA-C(CH₃)^{A}(**C**H₃)^{B}), 25.2 (uC-C(**C**H₃)^{A}(CH₃)^{B}), 22.3 (uC-C(CH₃)^{A}(**C**H₃)^{B}), 9.6 (uA-C^{ε}H**C**H₃).

### Cryptophycin-[uA-diol]-[uD-Dab(Alloc)] C4

TFA (3 mL) was added to a solution of acetonide protected cryptophycin **C3** (477 mg, 0.60 mmol) in dichloromethane (3 mL) at 0 °C. After 5 hours stirring at 0 °C the solvents were evaporated, and the residue was co evaporated with toluene (2 mL). The residue was dissolved in EtOAc (100 mL) and washed with sodium bicarbonate solution. The organic layer was separated, dried over MgSO₄, then concentrated in vacuo. Column chromatography (EtOAc-> DCM:MeOH 9:1) yielded **C4** as a white foam (340 mg, 0.44 mmol, 76 %).

| | | |
|---|---|---|
| **HPLC-MS** (ESI, +): | *m*/*z* (found): | 757.34, t_{R} = 8.6 min. |
| | *m*/*z* (calc.): | 757.32 (M+H⁺); (C₃₈H₅₀ClN₄O₁₀⁺) |

### Cryptophycin-[uA-diol]-[uD-Lys(Alloc)] G4

TFA (3 mL) was added to a solution of acetonide protected cryptophycin **G3** (61 mg, 0.77 µmol) in dichloromethane (3 mL) at 0 °C. After 4 hours stirring at rt the solvents were evaporated, and the residue was co evaporated with toluene (2 mL) three times. The residue was dissolved in EtOAc (50 mL) and washed with sodium bicarbonate solution. The organic layer was separated, dried over MgSO₄, then concentrated in vacuo. Column chromatography (EtOAc-> DCM:MeOH 9:1) yielded **G4** as a white foam (23 mg, 0.30 µmol,40 %).

| | | |
|---|---|---|
| **HPLC-MS** (ESI, +): | *m*/*z* (found): | 785.34, t_{R} = 8.6 min. |
| | *m*/*z* (calc.): | 785.35 (M+H⁺); (C₄₀H₅₄ClN₄O₁₀⁺) |

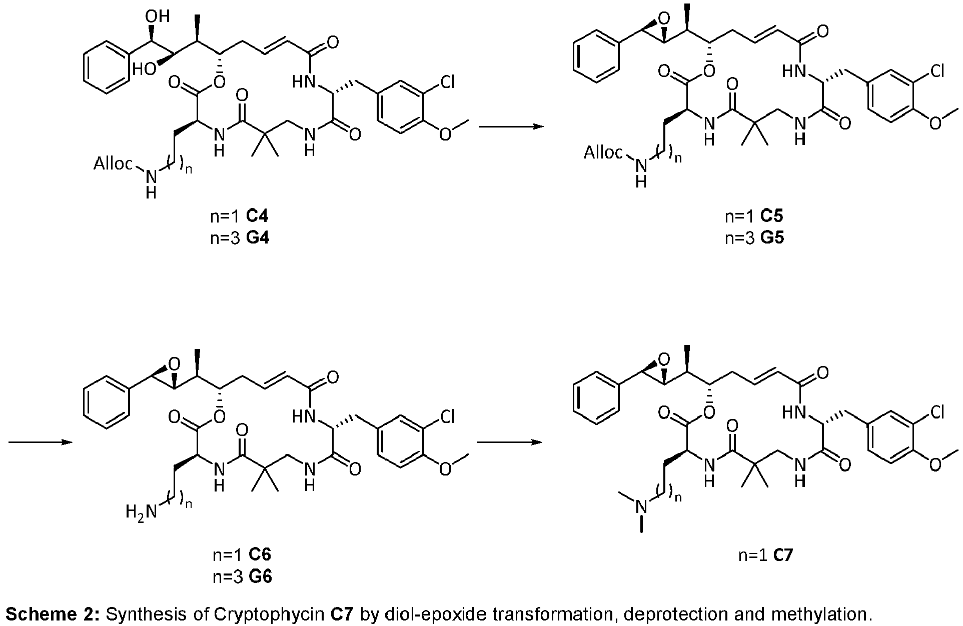

### Cryptophycin-[uD-Dab(Alloc)] C5

To a solution of diol **C4** (0.20 g, 0.26 mmol, 1 eq.) and PPTS in dichloromethane (7.5 mL) trimethylorthoformate (2.5 mL, 23 mmol, 88 eq.) was added. The solution was stirred at room temperature for 3 hours. The reaction solution was filtered over silica (d = 2.5 cm, I = 5 cm) and eluted with dichloromethan/ ethylacetate (1:1, 300 mL), then concentrated in vacuo and dried overnight in HV. The intermediate orthoester (0.18 g, 0.22 mmol, 86%) was dissolved in dichloromethane (3 mL) acetylbromide-solution (0.5 M in abs. DCM, 1.1 mL, 0.55 mmol, 2.5 eq.) was added and the reaction solution stirred at room temperature for 5 hours. The reaction solution was added to sodium bicarbonate solution (half sat., 50 mL). The organic layer was separated, and the aqueous layer was extracted with dichloromethane (3 × 20 mL). The organic layers were dried over MgSO₄, then concentrated in vacuo and dried overnight in HV.

An emulsion of abs. ethylene glycol (2.5 mL), abs. 1,2-dimethoxyethane (5.0 mL) and potassium carbonate was freshly prepared over 3 Å molecular sieves (320 mg) prepared and homogenized by vortexer and ultrasonic bath.

The potassium carbonate emulsion (6.5 mL, 1.31 mmol, 6.5 eq.) homogenized by constant shaking was mixed with bromo-formate (171 mg, 0.202 mmol). The mixture was stirred for 6 min at rt then diluted with abs. dichloromethane (20 mL). The solution was given to KHSO₄ solution (0.5 %, 20 mL), phases were separated immediately, and the aqueous phase was further extracted with dichloromethane (3 × 20 mL). The combined organic phases were dried over MgSO₄ and concentrated in vacuo. column chromatography (d = 2 cm, I = 18 cm, PE/EtOAc 1:9) yielded cryptophycin **CD57** as white solid foam (96 mg, 0.13 mmol, 50 % over 4 steps).

| | | |
|---|---|---|
| **HRMS:** (ESI, +) | m/z (found) | 761.2920 |
| | m/z (calc.) | 761.2924 (M+H⁺); (NaC₃₈H₄₇ClN₄O₉⁺) |

**¹H NMR** (600 MHz, Chloroform-d) *δ*/*ppm* = 7.45-7.29 (m, 3H, uA-C^{ar}**H**), 7.24 (d, ³*J* = 7.4 Hz, 2H, uA-C^{ar}**H**), 7.18 (s (broad), 1H, uB-C^{ar,2}**H**), 7.04 (dd, ³*J* = 8.5 Hz, ⁴*J* = 2.2 Hz, 1H, uB-C^{ar,6}**H**), 6.85 (d, ³*J* = 8.4 Hz, 1H, uB-C^{ar,5}**H**), 6.74 (ddd, ³*J* = 15.2 Hz, ³*J* = 11.4 Hz, ³*J* = 4.1 Hz, 1H, uA-C^{β}**H**), 6.54 (d, ³*J* = 6.9 Hz, 1H, uC-C^{γ}-N**H**), 6.50 (d, ³*J* = 8.1 Hz, 1H, uC-C^{α}-N**H**), 5.91 (ddt, ³*J* = 16.3 Hz, ³*J* = 10.7 Hz, ³*J* = 5.6 Hz, 1H, uD-C**H**=CH₂), 5.68 (d, ³*J* = 14.9 Hz, 1H, uA-C^{α}**H**), 5.58 (s (broad), 1H, uB-N**H**), 5.30 (dd, ³*J* = 17.2 Hz, ²*J* = 1.6 Hz, 1H, uD-CH=C**H**₂*^{trans}*), 5.22 (d, ³*J* = 10.1 Hz, 1H, uD-CH=C**H**₂*^{cis}*), 5.20-5.14 (m, 2H, uD-N**H**-Alloc, uA-C^{δ}**H**), 4.65-4.50 (m, 3H, uD-C**H**₂CH=CH₂, uB-C^{α}**H**), 4.38 (ddd, ³*J* = 9.1 Hz, ³*J* = 8.7 Hz, ³*J* = 3.8 Hz, 1H, uD-C^{α}**H**), 3.88 (s, 3H, uB-OC**H**₃), 3.67 (s (broad), 1H, uA-C^{η}**H**), 3.40 (dd, ²*J* = 13.2 Hz, ³*J* = 8.7 Hz, 1H, uC-C^{β}**H**^{A}H^{B}), 3.33-3.22 (m, 2H, uD-C^{γ}**H**^{A}H^{B}, uC-C^{β}H^{A}**H**^{B}), 3.11 (dd, ²*J* = 14.6 Hz, ³*J* = 4.9 Hz, 1H, uB-C^{β}**H**^{A}H^{B}), 2.92 (dd, ²*J* = 14.5 Hz, ³*J* = 6.8 Hz, 1H, uB-C^{β}H^{A}**H**^{B}), 2.89 (dd, ³*J* = 7.6 Hz, ³*J* = 2.0 Hz, 1H, uA-C^{ζ}**H**), 2.74 (dddd, ²*J* = 14.0 Hz, ³*J* = 9.2 Hz, ³*J* = 5.0 Hz, ³*J* = 5.0 Hz, 1H, uD-C^{γ}**H**^{A}H^{B}), 2.61 (dddd, ²*J* = 14.0 Hz, ³*J* = 4.3 Hz, ³*J* = 2.2 Hz, ⁴*J* = 2.2 Hz, 1H, uA-C^{γ}**H**^{A}H^{B}), 2.34 (ddd, ²*J* = 13.9 Hz, ³*J* = 11.5 Hz, ³*J* = 11.5 Hz, 1H, uA-C^{γ}H^{A}**H**^{B}), 1.80 (ddt, ³*J* = 7.0 Hz, ³*J* = 7.0 Hz, ³*J* = 7.0 Hz, 1H, uA-C^{ε}**H**), 1.71 (m, 1H, uD-C^{β}**H**^{A}H^{B}), 1.43 (m, 1H, uD-C^{β}H^{A}**H**^{B}), 1.21 (s, 3H, uC-C(C**H**₃)^{A}(CH₃)^{B}), 1.15 (d, ³*J* = 7.2 Hz, 3H, uA-C^{ε}HC**H**₃), 1.14 (s, 3H, uC-C(CH₃)^{A}(C**H**₃)^{B})

¹³**C NMR** (126 MHz, Chloroform-d) *δ*/*ppm =* 178.4 (uC-**C**=O), 172.4 (uD-**C**=O), 170.8 (uB-**C**ONH-uC), 164.9 (uB-**C**ONH-uA), 156.4 (**C**=O, Alloc), 154.2 (uB-**C**^{ar,4}), 141.9 (uA-**C**^{β}), 136.8 (**C**^{ar}), 133.0 (**C**=CH₂, Alloc), 130.9 (uB-**C**^{ar,2}), 129.5 (**C**^{ar}), 128.83 (**C**^{ar}), 128.79 (**C**^{ar}), 128.7 (**C**^{ar}), 128.3 (**C**^{ar}), 125.9 (uB-**C**^{ar,6}), 125.1 (uA-**C**^{α}), 122.6 (**C**^{ar}), 117.8 (C=**C**H₂, Alloc), 112.5 (uB-**C**^{ar,5}), 75.7 (uA-**C**^{δ}), 65.8 (**C**H₂-CH=CH₂, Alloc), 63.7 (uA-**C**^{ζ}), 59.3 (uA-**C**^{η}), 56.3 (uB-O**C**H₃), 55.0 (uB-**C**^{α}), 50.0 (uD-**C**^{α}), 47.1 (uC-**C**^{β}), 43.7 (uC-**C**^{α}), 40.6 (uA-**C**^{ε}), 37.2 (uA-**C**^{γ}), 36.9 (uD-**C**^{γ}), 35.7 (uB-**C**^{β}), 32.7(uD-**C**^{β}), 25.2 (uC-C(**C**H₃)₂), 22.1 (uC-C(**C**H₃)₂), 13.8 (uA-C^{ε}H**C**H₃).
**TLC:** R*f* (PE/EtOAc 1:9) = 0.20.

| | | |
|---|---|---|
| **HPLC-MS** (ESI +): | *m*/*z* (found) | 739.30, t_{R} = 9.5 min. |
| | *m*/*z* (calc.) | 739.31 (M+H⁺);(C₃₈H₄₈ClN₄O₉⁺) |

### Cryptophycin-[uD-Lys(Alloc)] G5

To a solution of diol **G4** (29 mg, 36 µmol, 1 eq.) and PPTS (27 mg, 107 µmol, 2.9 eq) in dichloromethane (7.5 mL) trimethyl orthoformate (0.5 mL, 4.5 mmol, 124 eq.) was added. The solution was stirred at room temperature for 3 hours. The reaction solution was filtered over silica (d = 2 cm, I = 4 cm) and eluted with dichloromethan/ ethylacetate (1:1, 200 mL), then concentrated in vacuo and dried overnight in HV. The intermediate orthoester (23 mg, 28 µmol, 75 %) was dissolved in dichloromethane (2 mL) acetyl bromide-solution (0.5 M in abs. DCM, 0.15 mL, 75 µmol, 2.7 eq.) was added and the reaction solution stirred at room temperature for 5 hours. The reaction solution was added to sodium bicarbonate solution (half sat., 50 mL). The organic layer was separated, and the aqueous layer was extracted with dichloromethane (3 × 20 mL). The organic layers were dried over MgSO₄, then concentrated in vacuo and dried overnight in HV.

An emulsion of abs. ethylene glycol (2.5 mL), abs. 1,2-dimethoxyethane (5.0 mL) and potassium carbonate was freshly prepared over 3 Å molecular sieves (320 mg) prepared and homogenized by vortexer and ultrasonic bath.

The potassium carbonate emulsion (1.0 mL, 0.21 mmol, 6.5 eq.) homogenized by constant shaking was mixed with bromoformate (20 mg, 26 µmol). The mixture was stirred for 6 min at rt then diluted with abs. dichloromethane (20 mL). The solution was given to KHSO₄ solution (0.5 %, 15 mL), phases were separated immediately, and the aqueous phase was further extracted with dichloromethane (3 × 20 mL). The combined organic phases were dried over MgSO₄ and concentrated in vacuo. column chromatography (d = 1 cm, I = 18 cm, PE/EtOAc 1:9) yielded cryptophycin **G5** as white solid foam (15 mg, 20 µmol, 56 % over 3 steps).

**¹H NMR** (500 MHz, Chloroform-d) *δ*/*ppm* = 7.40-7.30 (m, 3H, uA-C^{ar}**H**), 7.25-7.21 (m, 2H, uA-C^{ar}**H**), 7.17 (d, ⁴*J* = 1.9 Hz, 1H, uB-C^{ar,2}**H**), 7.03 (dd, ³*J* = 8.4 Hz, ⁴*J* = 1.9 Hz, 1H, uB-C^{ar,6}**H**), 6.89-6.82 (m, 2H, uB-C^{ar,5}**H**, NH), 6.74 (ddd, ³*J* = 15.1 Hz, ³*J* = 11.1 Hz, ³*J* = 4.0Hz, 1H, uA-C^{β}**H**), 6.15 (d, ³*J* = 7.5Hz, 1H, N**H**), 5.90 (m, 1H, uD-C**H**=CH₂), 5.69 (d, ³*J* = 15.0 Hz, 1H, uA-C^{α}**H**), 5.57 (m, 1H, N**H**), 5.29 (dd, ³*J* = 17.2 Hz, ²*J* = 1.2 Hz, 1H, uD-CH=C**H**₂*^{trans}*), 5.24-5.17 (m, 2H, uD-CH=C**H**₂*^{cis}*, uA-C^{δ}**H**), 4.76 (m, 1H, N**H**), 4.65 (m, 1H, uB-C^{α}**H**), 4.54 (d, ³*J* = 5.2 Hz uD-C**H**₂CH=CH₂), 4.31 (m, 1H, uD-C^{α}**H**), 3.88 (s, 3H, uB-OC**H**₃), 3.67 (s (broad), 1H, uA-C^{η}**H**), 3.42 (m, 1H, uC-C^{β}**H**^{A}H^{B}), 3.21 (m, 1H, uC-C^{β}H^{A}**H**^{B}), 3.14-3.04 (m, ³H, uB-C^{β}**H**^{A}H^{B}, uD-C^{ε}**H**₂), 2.96 (dd, ²*J* = 14.5 Hz, ³*J* = 8.1 Hz, 1H, uB-C^{β}H^{A}**H**^{B}), 2.91 (dd, ³*J* = 7.4 Hz, ³*J* = 1.8 Hz, 1H, uA-C^{ζ}**H**), 2.57 (m, 1H, uA-C^{γ}**H**^{A}H^{B}), 2.39 (m, 1H, uA-C^{γ}H^{A}**H**^{B}), 1.86-1.56 (m, 5H, uA-C^{ε}**H**, uD-C^{β}**H**₂, uD-C^{δ}**H**₂), 1.52-1.43 (m, 2H, uD-C^{γ}**H**₂), 1.25 (s, 3H, uC-C(C**H**₃)^{A}(CH₃)^{B}), 1.20 (s, 3H, uC-C(C**H**₃)^{A}(CH₃)^{B}), 1.14 (d, ³*J* = 6.6 Hz, 3H, uA-C^{ε}HC**H**₃)

¹³**C NMR** (151 MHz, Chloroform-d) *δ*/*ppm =* 177.2 (uC-**C**=O), 171.3 (uD-**C**=O), 171.1 (uB-CONH-uC), 166.2 (uB-CONH-uA), 156.7 (Alloc-**C**=O), 154.2 (uB-**C**^{ar,4}), 143.2 (uA-**C**^{β}), 139.2 (**C**^{ar}), 133.1 (Alloc-**C**=CH₂), 131.0 (uB-**C**^{ar,2}), 129.6 (**C**^{ar}), 128.7 (**C**^{ar}), 128.4 (**C**^{ar}), 127.8 (**C**^{ar}), 125.4 (uB-**C**^{ar,6}), 125.3 (uA-**C**^{α}), 122.7 (**C**^{ar}), 117.8 (Alloc-C=**C**H₂), 112.5 (uB-**C**^{ar,5}), 88.6 (uA-**C**^{δ}), 84.6 (uA-**C**^{ζ}), 84.0 (uA-**C**^{η}), 65.6 (uD-**C**H₂-CH=CH₂), 56.3 (uB-O**C**H₃), 55.0 (uB-**C**^{α}), 53.6 (uD-**C**^{α}), 47.0 (uC-**C**^{β}), 43.1 (uC-**C**^{α}), 42.1 (uA-**C**^{ε}), 40.0 (uD-**C**^{ε}) 35.1 (uA-**C**^{γ}), 35.0 (uB-**C**^{β}), 31.1 (uD-**C**^{β}), 29.2 (uD-**C**^{δ}) 24.2 (uC-C(**C**H₃)₂), 22.7 (uC-C(**C**H₃)₂), 22.6 (uD-**C**^{γ}), 17.4 (uA-C^{ε}H**C**H₃).
**TLC:** R*f* (PE/EtOAc 1:9) = 0.20.

| | | |
|---|---|---|
| **HPLC-MS** (ESI +): | *m*/*z* (found) | 789.4, t_{R} = 9.5 min. |
| | *m*/*z* (calc.) | 789.3 (M+H⁺); (C₄₀H₅₂ClN₄O₉⁺) |

### Cryptophycin-[uD-Dab] C6

Cryptophycin **C5** (26.2 mg, 35.2 µmol) and Tetrakis(triphenylphosphin)palladium (5.5 mg, 4.8 µmol, 13 mol-%) was dissolved in degassed dichlormethan (2 mL) and morpholin (4 drops) was added. The reaction solution was stirred at room temperature for 30 minutes then concentrated in vacuo. Column chromatography (d = 1 cm, l =21 cm, DCM/MeOH 9:1) yielded Cryptophycin **C6** (17.9 mg, 27.3 µmol, 77 %) as white solid.

| | | |
|---|---|---|
| **HRMS:** (ESI, +) | *m*/*z* (found) | 655.2896 |
| | *m*/*z* (calc.) | 655.2893 (M+H⁺); 0; (C₃₄H₄₄ClN₄O₇⁺) |

**TLC:** R*_{f}* (DCM/MeOH 9:1) = 0.15.

| | | |
|---|---|---|
| **HPLC-MS** (ESI +): | *m*/*z* (found) | 655.31, t_{R} = 6.3 min. |
| | *m*/*z* (calc.) | 655.29 (M+H⁺); (C₃₄H₄₄ClN₄O₇⁺). |

**¹H NMR** (600 MHz, Chloroform-*d*) *δ*/*ppm* = 7.36-7.27 (m, 3H, uA-C^{ar}**H**), 7.26-7.22 (m, 2H, uA-C^{ar}**H**), 7.17 (d, ³*J* = 7.2 Hz, 1H, uB-C^{ar,2}**H**), 7.07 (d, ³*J* = 8.3 Hz, 1H, uB-C^{ar,6}**H**), 6.82 (d, ³*J* = 8.4 Hz, 1H, uB-C^{ar,5}), 6.70 (m, 1H, uA-C^{β}**H**), 5.95 (s (broad), 1H, uA-C^{α}**H**), 5.14 (m, 1H, uA-C^{δ}**H**), 4.68 (m, 1H, uB-C^{α}**H**), 4.46 (s (broad), 1H, uD-C^{α}**H**), 3.86 (s, 3H, uB-OC**H**₃), 3.64 (s (broad), 1H, uA-C^{η}**H**), 3.49 (m, 1H, uC-C^{β}**H**^{A}H^{B}), 3.12-3.01 (m, 3H, uB-C^{β}**H**₂, uC-C^{β}H^{A}**H**^{B}), 2.85 (dm, ³*J* = 7.4 Hz, 1H, uA-C^{ζ}**H**), 2.74 (dm, ³*J* = 9.0 Hz, 2H uD-C^{γ}**H**₂), 2.56 (m, 1H, uA-C^{γ}**H**^{A}H^{B}), 2.40 (m, 1H, uA-C^{γ}H^{A}**H**^{B}), 1.76 (s (broad), 1H, uD-C^{β}**H**^{A}H^{B}), 1.66 (s (broad), 1H, uA-C^{ε}**H**), 1.36 (s (broad), 1H, uD-C^{β}H^{A}**H**^{B}), 1.20 (s, 3H, uC-C(C**H**₃)₂), 1.12 (s, 3H, uC-C(C**H**₃)₂), 1.10 (d, ³*J* = 6.8 Hz, 3H, uA-C^{ε}HC**H**₃).

¹³**C NMR** (126 MHz, Chloroform-d) *δ*/*ppm =* 178.2 (uC-**C**=O), 172.3 (uD-**C**=O), 171.1 (uB-**C**ONH-uC), 165.4 (uA-**C**ONH-uB), 154.1 (uB-**C**^{ar,4}), 140.4 (uA-**C**^{β}), 136.9 (**C**^{ar}), 131.2 (uB-**C**^{ar,2}), 129.9 (**C**^{ar}), 129.2 (**C**^{ar}), 128.7 (**C**^{ar}), 128.7 (**C**^{ar}), 128.6 (**C**^{ar}), 128.4 (**C**^{ar}), 125.9 (uA-**C**^{α}), 125.4 (**C**^{ar}), 122.6 (**C**^{ar}), 112.4 (uB-**C**^{ar,5}), 75.9 (uA-**C**^{δ}), 63.9 (uA-**C**^{ζ}), 59.5 (uA-**C**^{η}), 56.3 (uB-O**C**H₃), 54.2 (uB-**C**^{α}), 52.0 (uD-**C**^{α}), 47.3 (uC-**C**^{β}), 42.6 (uC-**C**^{α}), 41.0 (uA-**C**^{ε}), 37.9 (uD-**C**^{γ}), 37.1 (uA-**C**^{γ}), 35.4 (uB-**C**^{β}), 31.9 (uD-**C**^{β}), 24.9 (uC-C(**C**H₃)₂), 23.0 (uC-C(**C**H₃)₂), 13.8 (uA-C^{ε}H**C**H₃).

### Cryptophycin-[uD-Lys] G6

Cryptophycin **G5** (11.9 mg, 15.5 µmol) and Tetrakis(triphenylphosphin)palladium (2.0 mg, 1.7 µmol, 11 mol-%) was dissolved in degassed dichloromethane (2 mL) and morpholine (2 drops) was added. The reaction solution was stirred at room temperature for 30 minutes then concentrated in vacuo. Preparative RP-HPLC yielded cryptophycin **G6** (4.5mg, 6.6 µmol, 41 %) as white solid.

| | | |
|---|---|---|
| **HPLC-MS** (ESI +): | *m*/*z* (found) | 683.36, t_{R} = 6.3 min. |
| | *m*/*z* (calc.) | 683.32 (M+H⁺); (C₃₆H₄₈ClN₄O₇⁺) |

**¹H NMR** (600 MHz, DMSO-*d*₆) *δ l ppm* = 8.57 (d, ³*J* = 8.3 Hz, 1H, N**H**), 7.85 (d, ³*J* = 6.8 Hz, 1H, N**H**) 7.66 (s, 3H, N**H**), 7.39-7.27 (m, 6H, uA-C^{ar}**H**, uB-C²**H**), 7.23-7.15 (m, 1H, uB-C⁶**H**, N**H**), 7.05 (d, ³*J* = 8.5 Hz, 1H, uB-C⁵), 6.667 (ddd, ³*J* = 15.7 Hz, ³*J* = 10.9 Hz, ³*J* = 5.1 Hz, 1H, uA-C^{β}**H**), 6.00 (d, ³*J* = 15.4 Hz, 1H, uA-C^{α}**H**), 4.84 (m, 1H, uA-C^{δ}**H**), 4.56 (d, ³*J* = 6.5 Hz, 1H, uA-C^{η}**H**), 4.33 (m, 1H, uD-C^{α}**H**), 4.28 (m, 1H, uB-C^{α}**H**), 4.10 (dd, ³*J* = 9.3 Hz, ³*J* = 5.3 Hz, 1H, uA-C^{ζ}**H**), 3.81 (s, 3H, uB-OC**H**₃), 3.05 (d, ²*J* = 13.4 Hz, 1H, uC-C^{β}**H**^{A}H^{B}), 3.00 (dd, ²*J* = 13.4 Hz, ³*J* = 3.8 Hz, 1H, uC-C^{β}H^{A}**H**^{B}), 2.80-2.72 (m, 4H, uB-C^{β}**H**₂, uD-C^{ε}**H**₂), 2.69 (m, 1H, uA-C^{γ}**H**^{A}H^{B}), 2.28 (m, 1H, uA-C^{γ}H^{A}**H**^{B}), 2.06 (m, 1H, uA-C^{ε}**H**), 1.80 (m, 1H, uD-C^{β}**H**^{A}H^{B}), 1.63 (m, 1H, uD-C^{β}H^{A}**H**^{B}), 1.54-1.44 (m, 2H, uD-C^{δ}**H**₂), 1.34-1.25 (m, 2H, uD-C^{γ}**H**₂), 1.18 (s, 3H, uC-C(C**H**₃)₂), 1.06 (s, 3H, uC-C(C**H**₃)₂), 1.02 (d, ³*J* = 6.7 Hz, 3H, uA-C^{ε}HC**H**₃).

**¹³C NMR** (126 MHz, DMSO-*d₆*) *δ l ppm* = 177.4 (uC-**C**=O), 171.4 (uD-**C**=O), 171.3 (uB-CONH-uC), 164.6 (uA-**C**(=O)NH), 153.1 (uB-**C**^{ar,4}), 140.0 (uA-**C**^{β}), 138.6 (**C**^{ar}), 131.4 (uB-**C**^{ar,2}), 130.3 (**C**^{ar}), 128.6 (**C**^{ar}), 128.5 (**C**^{ar}), 127.9 (**C**^{ar}), 127.8 (**C**^{ar}), 125.7 (**C**^{ar}), 120.5 (uA-**C**^{α}), 112.7 (uB-**C**^{ar,5}), 85.5 (uA-**C**^{δ}), 82.6 (uA-**C**^{ζ}), 82.4 (uA-**C**^{η}), 56.2 (uB-**C**^{α}), 56.0 (uB-O**C**H₃), 51.4 (uD-**C**^{α}), 46.3 (uC-**C**^{β}), 42.0 (uA-**C**^{ε}), 41.8 (uC-**C**^{α}), 38.6 (uB-**C**^{β}), 35.5 (uD-**C**^{ε}), 33.1 (uA-**C**^{γ}), 29.4 (uD-**C**^{β}), 26.5 (uD-**C**^{δ}), 24.5 (uC-C(**C**H₃)₂), 22.7 (uC-C(**C**H₃)₂), 21.9 (uD-**C**^{γ}), 14.8 (uA-C^{ε}H**C**H₃).

### Cryptophycin-[uD-Dab-Me₂] C7

Cryptophycin **C6** (4.2 mg, 6.1 µmol, 1 eq.) was dissolved in 2-propanol (1 mL) and formalin (37 %, 10 µL, 0.12 mmol, 20 eq.) was added. After 10 minutes sodium cyanoborohydride (3.7 mg, 61 µmol, 10 eq.) was added and stirred for further 30 minutes at room temperature then concentrated in vacuo. Column chromatography (d = 5 mm, I=22 cm, DCM/MeOH 19:1) yielded cryptophycin **C7** (1.3 mg, 1.9 µmol, 31 %) as white solid.

**TLC:** R*f* (DCM/MeOH 19:1) = 0.09.

| | | |
|---|---|---|
| **HPLC-MS** (ESI +): | *m*/*z* (found) | 683.31, t_{R} = 6.3 min |
| | *m*/*z* (calc.) | 683.32 (M+H⁺); (C₃₆H₄₈ClN₄O₇⁺). |

**¹H NMR** (600 MHz, Chloroform-d) *δ l ppm* = 9.52 (s (broad), 1H, uD-N**H**), 7.75 (s (broad), 1H, uC-C^{α}-NH), 7.37-7.30 (m, 3H, uA-C^{ar}**H**), 7.23-7.20 (m, 2H, uA-C^{ar}**H**), 7.18 (d, ⁴*J* = 2.1 Hz, 1H, uB-C^{ar,2}**H**), 7.03 (dd, ³*J* = 8.4 Hz, ⁴*J* = 2.1 Hz, 1H, uB-C^{ar,6}**H**), 6.82 (d, ³*J* = 8.4 Hz, 1H, uB-C^{ar,5}**H**), 6.77 (ddd, ³*J* = 15.1 Hz, ³*J* = 10.1 Hz, ³*J* = 4.9 Hz, 1H, uA-C^{β}**H**), 5.77 (d, ³*J* = 15.2 Hz, 1H, 1H, uA-C^{α}**H**), 5.57 (s (broad), 1H, uB-N**H**), 5.13 (ddd, ³*J* = 11.2 Hz, ³*J* = 5.6 Hz, ³*J* = 1.7 Hz, 1H, uA-C^{δ}**H**), 4.79 (m, 1H, uB-C^{α}**H**), 4.14 (m, 1H, uD-C^{α}**H**), 3.87 (s, 3H, uB-OC**H**₃), 3.66 (d, ³*J* = 1.8 Hz, 1H, uA-C^{η}**H**), 3.25 (m, 1H, uC-C^{β}H^{A}**H**^{B}), 3.16 (dd, ²*J* = 13.3 Hz, ³*J* = 4.4 Hz, 1H, uC-C^{β}**H**^{A}H^{B}), 3.13 (dd, ²*J* = 14.6 Hz, ³*J* = 6.9 Hz, 1H, uB-C^{β}**H**^{A}H^{B}), 3.03 (dd, ²*J* = 14.4 Hz, ³*J* = 4.9 Hz, 1H, uB-C^{β}H^{A}**H**^{B}), 2.87 (dd, ³*J* = 7.9, ³*J* = 1.9 Hz, 1H, uA-C^{ζ}**H**), 2.56 (m, 1H, uA-C^{γ}**H**^{A}H^{B}), 2.48 - 2.40 (2, 2H, uD-C^{γ}**H**^{A}H^{B}, uA-C^{γ}H^{A}**H**^{B}), 2.20 (s, 6H, uD-N(C**H**₃)₂), 2.04 (m, 1H, uD-C^{γ}H^{A}**H**^{B}), 1.74 (m, 1H, uA-C^{ε}**H**) 1.67 (m, 1H, uD-C^{β}**H**^{A}H^{B}), 1.41 (m, 1H, uD-C^{β}H^{A}**H**^{B}), 1.13 (d, ³*J* = 6.9 Hz, 1H, uA-C^{ε}CH₃), 1.12 (s, 3H, uC-C(C**H**₃)^{A}(CH₃)^{B}), 1.03 (s, 3H, uC-C(CH₃)^{A}(C**H**₃)^{B}).

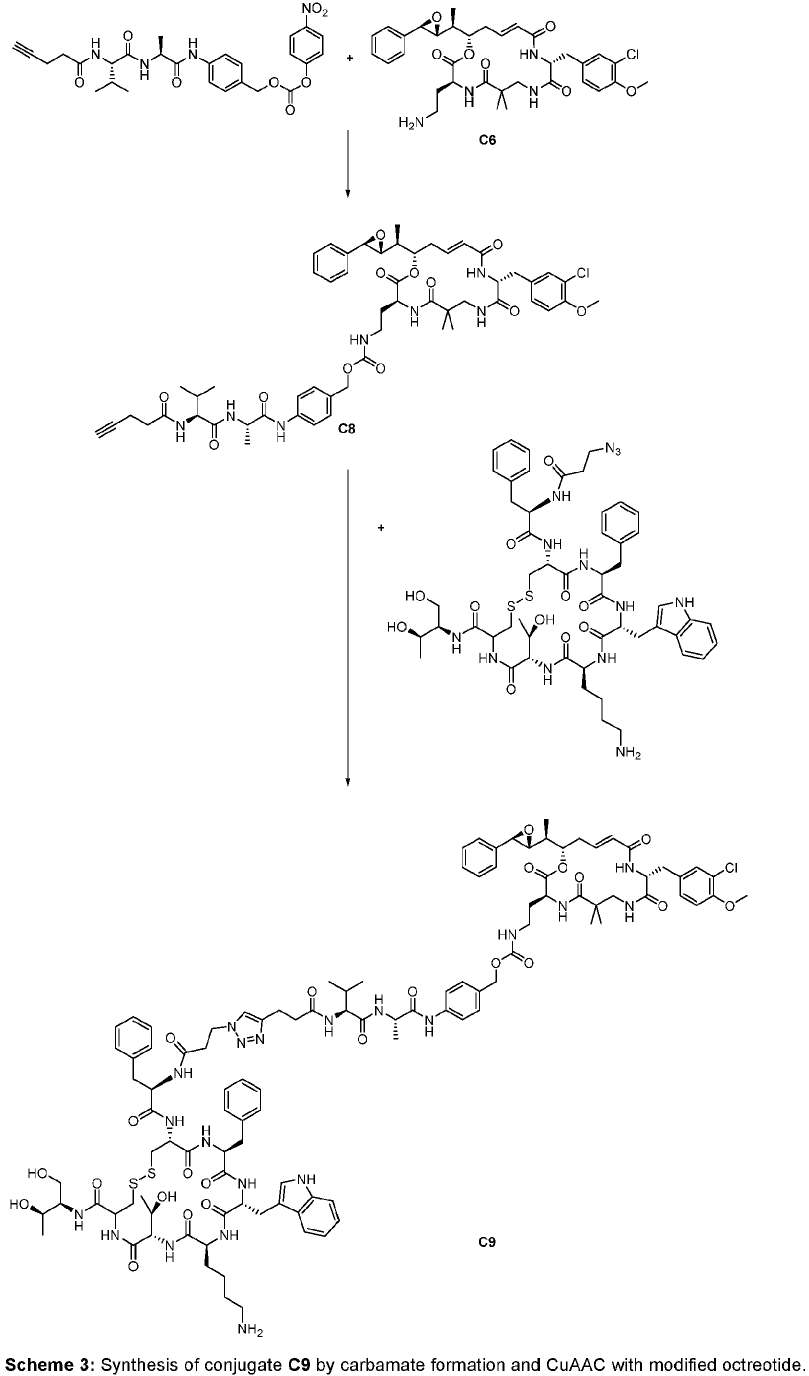

### 4-Pentynoic acid-Val-Ala-PAB-Cryptophycin C8

4-Pentynoic acid-Val-Ala-PAB-PNP (3.16 mg, 5.76 µL, 1 eq.) and crypto **C6** (4.07 mg, 6.21 µmol, 1.08 eq.) was dissolved in dry DMF (1 mL) and D*i*PEA (3.0 µL 17.2 µmol, 3 eq) was added. After 3 hours the crude was injected into preparative HPLC and yielded the conjugate **C8** as white solid (3.30 mg, 3.13 µmol, 54 %).

| | | |
|---|---|---|
| **HPLC-MS** (ESI +): | *m*/*z* (found) | 1054.45, t_{R} = 9.1 min. |
| | *m*/*z* (calc.) | 1054.47 (M+H⁺); (C₅₅H₆₉ClN₇O₁₂+⁺) |

### Octreotide-4-Pentynoic acid-Val-Ala-PAB-Cryptophycin C9

Conjugate **C8** (3.30 mg, 3.13 µmol, 1 eq) and octreotide azide (3.50 mg, 3.21 µmol, 1 eq) was dissolved in water (0.5 mL) and *tert*-butanol (1 mL) and degassed properly. Then copper dust (2 mg) was added to the mixture. After stirring for 23 hours at rt it was diluted with water/acetonitrile (1:1, 5 mL) and filtered over celite. The filtrate was lyophilized and resolved in water/acetonitrile (1:1, 1 mL). Preparative HPLC and yielded the conjugate **C9** as white solid (0.60 mg, 0.27 µmol, 9 %)

| | | |
|---|---|---|
| **HPLC-MS** (ESI +): | *m*/*z* (found) | 1085.30, t_{R} = 7.6 min. |
| | *m*/*z* (calc.) | 1085.47 (M+2H⁺); (C₁₀₇H₁₃₉ClN₂₀O₂₃S₂²⁺). |

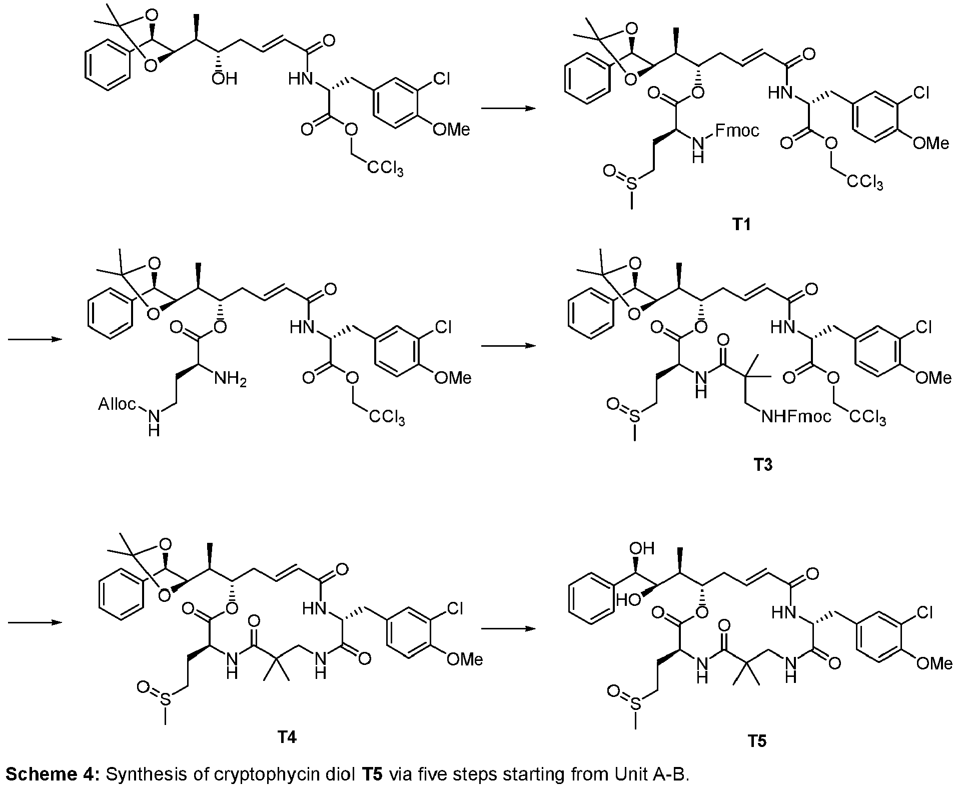

### Fmoc-uD[Met(O)]-uA[acetonide]-uB-OTce T1

A solution of Fmoc-Met(O)-OH (0.33 g, 0.84 mmol, 1.1 eq.) and building block A-B (0.50 g, 0.76 mmol, 1.0 eq.) in abs. THF (19 mL) was stirred at 0 °C under argon. Triethylamine (211 µL, 1.52 mmol, 2.0 eq.) and DMAP (18 mg, 0.15 mmol, 0.2 eq.) followed by 2,4,6-trichlorobenzoyl chloride (0.19 mL, 1.21 mmol, 1.5 eq.) were added. The solution was stirred for 3 h at 0 °C. A solution of citric acid (10 %, 50 mL) in water was added. The organic layer was separated, and the aqueous layer was extracted with EtOAc (3 x 50 mL). The organic layers were combined and dried over MgSO₄, then concentrated in vacuo. Column chromatography (d = 4 cm, I = 20 cm, PE/EtOAc 2:1) yielded **T1** as a white foam (0.63 g, 0.61 mmol, 80 %).

| | | |
|---|---|---|
| **HRMS:** (ESI+) | m/z (found) | 1053.2079 |
| | m/z (calc.) | 1053.2095 (M+Na⁺); (NaC₅₀H₅₄Cl₄N₂O₁₁S⁺) |
| **HPLC-MS** (ESI+): | *m*/*z* (found) | 1031.23, t_{R} = 12.0 min. |
| | *m*/*z* (calc.) | 1031.23 (M+H⁺); (C₅₀H₅₅Cl₄N₂O₁₁S⁺). |

**¹H NMR** (500 MHz, Chloroform-d, partly double signal set caused by chiral sulfur) *δ* / *ppm* = 7.76 (d, ³*J* = 7.5 Hz, 2H, Fmoc-C^{ar}**H**), 7.64-7.54 (m, 2H, Fmoc-C^{ar}**H**), 7.46 - 7.27 (m, 9H, uA-C^{ar}**H**, Fmoc-C^{ar}**H**), 7.15 (m, 1H, uB-c^{ar,2}**H**), 6.98 (d, ³*J* = 8,6 Hz, 1H, uB-C^{ar,6}**H**), 6.85 (d, ³*J* = 8.6 Hz, 1H, uB-C^{ar,5}**H**), 6.77 (d, ³*J* = 7.9 Hz, 1H, uB-N**H**), 6.54 (ddd, ³*J* = 15.9 Hz, ³*J* = 7.8 Hz, ³*J* = 7.8 Hz, 1H, uA-C^{β}**H**), 6.09, 5.99 (2s, 1H, uD-N**H**), 5.60 (d, ³*J* = 15.5 Hz, 1H, uA-C^{α}**H**), 5.05 - 4.81 (m, 2H, uB-C^{α}**H**, uA-C^{δ}**H**), 4.78 (m, 1H, uA-C^{η}**H**), 4.71 - 4.66 (m, 1H, uB-C-**H**^{A}H^{B}-CCl₃), 4.59 (d, ²*J* = 11.9 Hz, 1H, uB-C-**H**^{A}H^{B}-CCl₃), 4.45 - 4.27 (m, 3H, uD-Fmoc-C**H**₂, uD-C^{α}**H**), 4.20 (t, ³*J* = 7,0.Hz, 1H, uD-Fmoc-CH₂C**H**), 3.89 - 3.76 (m, 4H, uA-C^{ζ}**H**, uB-OC**H**₃), 3.11 (dd, ²*J* = 14.3 Hz, ³*J* = 7.0 Hz, 1H, uB-C^{β}**H**^{A}H^{B}), 2.97 (m, 1H, uB-C^{β}H^{A}**H**^{B}), 2.86 - 2.69(m, 2H, uD-C^{γ}**H**₂), 2.59, 2.60 (2s, 3H, uD-SC**H**₃), 2.43 - 2.10 (m, 4H, uA-C^{γ}**H**₂, uD-C^{β}**H**²), 1.97 (d, ³*J* = 7.1 Hz, 1H, uA-C^{ε}**H**), 1.51 (s, 3H, uA-C(C**H**₃^{A})(C**H**₃^{B})), 1.44 (s, 3H, uA-C(CH₃^{A})(C**H**₃^{B})), 1.09 (d, ³*J* = 7.0 Hz, 3H, uA-C^{ε}C**H**₃).

**¹³C NMR** (126 MHz, Chloroform-d, partly double signal set caused by chiral sulfur) *δ l ppm* = 170.3 (uD-**C**=O) 170.3 (uB- **C**(=O)O-CCl₃), 165.5 (uB-**C**(=O)NH), 156.4 (Fmoc-**C**=O), 154.2 (uB-**C**^{**ar**,4}), 144.0 (**C**^{ar}), 143.7 (**C**^{ar}), 141.4 (**C**^{ar}), 139.1 (uA-**C**^{β}), 137.5 (**C**^{ar}), 132.4 (**C**^{ar}), 131.3 (uB-**C**^{**ar**,2}), 129.1 (**C**^{ar}), 129.0 (**C**^{ar}), 128.8 (**C**^{ar}), 128.6 (uB-**C**^{ar,1}), 128.1 (uB-**C**^{**ar**,6}), 127.9 and 127.2 (**C**^{ar}), 127.1 (**C**^{ar}), 126.0 (**C**^{ar}), 125.3 (uA-**C**^{α}), 122.3 (uB-**C**³), 120.2 (**C**^{ar}), 112.2 (uB-**C**⁵), 109.3 and 109.3 (uA-**C**(CH₃)₂, 94.5 (uB-**C**Cl₃), 82.51 and 82.46 (uA-**C**^{ζ}, 80.8 and 80.7 (uA-**C**^{η}), 76.0 (uA-**C**^{δ}), 74.7 (uB-**C**H₂CCl₃), 67.4, and 67.3 (Fmoc-**C**H₂), 56.3 (uB-O**C**H₃), 53.7 and 53.4 (uB-**C**^{α}), 49.8 and 49.7 (uD-**C**^{γ}), 47.2 (Fmoc-CH₂**C**H), 38.5 and 38.3 (uD-OS**C**H₃), 36.4 (uA-**C**^{γ}), 36.2 and 36.1 (uB-**C**^{β}), 32.6 (uA-**C**^{ε}), 27.4 (uA-C(**C**H₃)^{A}(CH₃)^{B}), 27.1 (uA-C(CH₃)^{A}(**C**H₃)**^{B}**), 25.3 (uD-**C**^{β}), 9.9 (uA-C^{ε}H**C**H₃).

### Fmoc-uC-uD[Met(O)]-uA[acetonide]-uB-OTce T3

Piperidine (0.50 mL, 5.0 mmol, 9 eq.) was added to a solution of **T1** (625 mg, 611 µmol) in DMF (15 mL) at 0 °C. After 2 hours stirring at 0 °C the solvent was evaporated. The resulting colorless oil was co-evaporated with toluene three times, then dissolved in DMF (9 mL). Fmoc-3-amino-2,2-dimethyl-propionic acid (248 mg, 730 mmol, 1.2 eq.), HOAt (319 mg, 1.38 mmol, 2.3 eq.) and D*i*PEA (0.54 mL, 3.2 mmol, 5.4 eq.) were dissolved in dichloromethane (40 mL) and DIC (0.2 mL, 1.3 mmol, 2.2 eq.) was added at 0 °C over 10 minutes and stirred for additional 10 minutes. The DMF solution was added over 15 minutes. After stirring at RT for 20 h the solution was given to a solution of citric acid (10 %, 50 mL) in water. The organic layerwas separated, and the aqueous layer was extracted with EtOAc (3 × 30 mL). The organic layers were combined washed with sodium bicarbonate solution (sat., 40 mL) and brine (40 mL) and dried over MgSO₄, then concentrated in vacuo. Column chromatography (d = 1.5 cm, I = 25 cm, PE/EtOAc 2:1→0:1) yielded **T3** as a white foam (445 mg, 393 µmol, 64 %).

| | | |
|---|---|---|
| **HRMS:** (ESI, +) | m/z (found) | 1152.2782 |
| | m/z (calc.) | 1152.2779 (M+H⁺); (C₅₅H₆₃Cl₄N₃O₁₂S⁺) |

**¹H NMR** (500 MHz, Chloroform-d, partly double signal set caused by chiral sulfur) *δ l ppm* = 7.75 (d, ³*J* = 7.6 Hz, 2H, Fmoc-C^{ar}**H**), 7.67 - 7.56 (m, 2.5H, Fmoc-C^{ar}**H**, uD-N**H**), 7.53 (d, ³*J* = 6.5 Hz, 0.5H, uD-NH), 7.42 - 7.26 (m, 9H, uA-C^{ar}**H**, Fmoc-C^{ar}**H**), 7.20 - 7.13 (m, 2H, uB-C^{ar,2}**H**, uB-N**H**), 7.05 (m, 1H, uB-C^{ar,6}**H**), 6.80 (d, ³*J* = 8.4 Hz, 1H, uB-C^{ar,5}**H**), 6.55 (ddd, ³*J* = 17.6 Hz, ³*J* = 11.9 Hz, ³*J* = 6.4 Hz, 1H, uA-C^{β}**H**), 5.83 - 5.66 (m, 2H, Fmoc-NH, uA-C^{α}**H**), 5.03 (ddt, ³*J* = 8.3 Hz, ³*J* = 8.3 Hz, ³*J* = 4.3 Hz, 1H, uA-C^{δ}**H**), 4.97 (m, 1H, uB-C^{α}**H**), 4.79 (dd, ²*J* = 11.9 Hz, ³*J* = 4.2 Hz, 1H, uB-C-**H**^{A}H^{B}-CCl₃), 4.70 (d, ³*J* = 8.7 Hz, 1H, uA-C^{η}**H**), 4.66 (d, ²*J* = 11.9 Hz, 1H, uB-C-H^{A}**H**^{B}-CCl₃), 4.49 - 4.28 (m, 3H, Fmoc-C**H**₂, uD-C^{α}**H**), 4.19 (m, 1H, Fmoc-CH₂C**H**), 3.84 (s, 3H, uB-OC**H**₃), 3.80 (dd, ³*J* = 9.2 Hz, ³*J* = 2.9 Hz, 1H, uA-C^{ζ}**H**), 3.36-3.24 (m, 2H, uC-C^{β}**H**₂), 3.17 (dd, ²*J* = 14.1 Hz, ³*J* = 3.6 Hz, 1H, uB-C^{β}**H**^{A}H^{B}), 3.00 (dd, ²*J* = 14.1 Hz, ³*J* = 7.8 Hz, 1H, uB-C^{β}H^{A}**H**^{B}), 2.87 (m, 0.5H, uD-C^{γ}**H**^{A}H^{B}), 2.75 (m, 1H, uD-C^{γ}**H**^{A}H^{B}), 2.63 (m, 0.5H, uD-C^{γ}H^{A}**H**^{B}), 2.54 (s, 1.5H, uD-SC**H**₃), 2.50 (s, 1.5H, uD-SC**H**₃), 2.45-2.18 (m, 4H, uA-C^{γ}**H**₂, uD-C^{β}**H**²), 2.00 (m, 1H, uA-C^{ε}**H**), 1.51 (s, 3H, uA-C(C**H**₃)^{A}(CH₃)^{B}), 1.45 (s, 3H, uA-C(C**H**₃)^{A}(CH₃)^{B}), 1.23-1.03 (m, 9H, uA-C^{ε}C**H**₃, uC-(C**H**₃)₂).

**¹³C NMR** (126 MHz, Chloroform-d, partly double signal set caused by chiral sulfur) *δ* / *ppm* = 177.9 and 177.9 (uC-**C**=O), 171.1 and 171.0 (uD-C=O), 170.3 (uB-**C**(=O)-OCH₂CCl₃)), 165.8 (uA-**C**(=O)NH), 157.1 (Fmoc-**C**=O), 154.1 (uB-**C**^{ar,4}), 144.23 and 144.19, (**C**^{ar}), 141.42 and 141.38 (**C**^{ar}), 138.9 and 138.7 (uA-**C**^{β}), 137.58 and 137.55 (**C**^{ar}), 131.4 (uB-**C**^{ar,2}), 129.38 and 129.36 (uB-**C**^{ar,1}), 128.9 and 128.9 (**C**^{ar}), 128.7 (**C**^{ar}), 128.7 and 128.6 (uB-**C**^{**ar**,6}), 127.8 and 127.7 (**C**^{ar}H), 127.2 and 127.2 (**C**^{ar}H), 127.1 (**C**^{ar}H), 127.0 and 126.9 (**C**^{ar}H), 125.8 and 125.8 (**C**^{ar}), 125.5 and 125.4 (uA-**C**^{α}), 125.4 and 125.4 (**C**^{ar}), 122.2 and 122.1 (uB-**C**^{ar,3}), 120.1 (**C**^{ar}), 112.3 and 112.2 (uB-**C**^{**ar**,5}), 109.2 and 109.1 (uA-**C**(CH₃)₂), 94.6 (uB-**C**Cl₃), 82.6 and 82.5 (uA-**C**^{ζ}), 80.7 and 80.7 (uA-**C**^{η}), 76.0 and 76.0 (uA-**C**^{δ}), 74.7 and 74.7 (uB-**C**H₂-CCl₃), 66.9 (Fmoc-**C**H₂), 56.3 and 56.2 (uB-O**C**H₃), 53.4 and 53.4 (uB-**C**^{α}), 52.2 and 51.9 (uD-**C**^{α}), 50.0 and 49.9 (uC-**C**^{β}), 49.5 and 48.9 (uD-**C**^{γ}), 47.4 and 47.4 (Fmoc-CH₂**C**H), 43.7 (uC-**C**^{α}), 38.5 and 37.9, (uD-OS**C**H₃), 36.6 and 36.5 (uB-**C**^{β}), 36.4 and 36.3 (uA-**C**^{ε}), 32.14 and 32.05 (uA-**C**^{γ}), 27.4 (uA-C(**C**H₃)^{A}(CH₃)^{B}), 27.2 (uA-C(CH₃)^{A}(**C**H₃)^{B}), 24.2 and 23.6 (uD-**C**^{β}), 23.4 and 23.3 (uC-C(**C**H₃)^{A}(CH₃)^{B}), 22.94 and 22.85 (uC-C(CH₃)^{A}(**C**H₃)^{B}), 9.8 and 9.7 (uA-C^{ε}**C**H₃).

### Cryptophycin-[uA-acetonide]-[uD-Met(O)] T4

Piperidine (0.20 mL, 2.0 mmol, 5 eq.) was added to a solution of seco-depsipeptide **T3** (444 mg, 0.40 mmol) in DMF (12 mL) at 0 °C. The cooling bath was removed, and the reaction stirred at room temperature for 16 hours. EtOAc (200 mL) and water (200 mL) was added to the reaction solution. The organic layer was isolated, and the aqueous layer was extracted with EtOAc (3 × 200 mL). The organic layers were dried over MgSO₄, then concentrated in vacuo. Column chromatography (d= 2 cm, I = 20 cm, EtOAc/MeOH 19:1→4:1) yielded **T4** as a white foam (229 mg, 301 µmol, 77 %).

| | | |
|---|---|---|
| **HRMS:** (ESI+) | m/z (found) | 782.2850 |
| | m/z (calc.) | 782.2849 (M+Na⁺); (NaC₃₈H₅₀ClN₃O₉S⁺) |
| **HPLC-MS** (ESI+): | *m*/*z* (found) | 760.3, t_{R} = 8.7 min. |
| | *m*/*z* (calc.) | 760.30 (M+H⁺); (C₃₈H₅₁ClN₃O₉S⁺). |

**¹H NMR** (500 MHz, Chloroform-d, partly double signal set caused by chiral sulfur) *δ l ppm* = 7.44 - 7.27 (m, 6H, uA-C^{ar}**H**, uD-NH), 7.17 (s, 1H, uB-C^{ar,2}**H**), 7.03 (d, ³*J* = 8.5 Hz, 1H, uB-C^{ar,6}**H**), 6.96 (d, ³*J* = 7.1 Hz, 0.5H, uC-N**H**), 6.89 (s, 0,5H, uC-N**H**), 6.83 (d, ³*J* = 8.3 Hz, 1H, uB-C^{ar,5}**H**), 6.60 (ddd, ³*J* = 14.8 Hz, ³*J* = 10.4 Hz, ³*J* = 4.2 Hz, 1H, uA-C^{β}**H**), 5.71 (d, ³*J* = 7.4 Hz, 1H, uB-N**H**,), 5.65 (dd, ²*J* = 14.7 Hz, ³*J* = 6.2 Hz, 1H, uA-C^{α}**H**,), 5.08 (dd, ³*J* = 10.8 Hz, ³*J* = 5.7 Hz, 1H, uA-C^{δ}**H**), 4.70 (d, ³*J* = 8.9 Hz, 1H, uB-C^{α}**H**), 4.65 (m, 1H, uA-C^{η}**H**), 4.32 (m, uD-C^{α}**H**), 3.86 (s, 3H, uB-OC**H**₃), 3.77 (d, ³*J* = 8.7 Hz, 1H, uA-C^{ζ}**H**), 3.36 (t, ²*J* = 11.2 Hz, 1H, uC-C^{β}**H**^{A}H^{B}), 3.19 (t, ²*J* = 14.6 Hz, 1H, uC-C^{β}H^{A}**H**^{B}), 3.07 (m, 1H, uB-C^{β}**H**^{A}H^{B}), 2.97 (m, 1H, uB-C^{β}H^{A}**H**^{B}), 2.79 (ddd, ²*J* = 12.3 Hz, ³*J* = 6.3 Hz, ³*J* = 6.3 Hz, 1H, uD-C^{γ}**H**^{A}H^{B}), 2.65 (ddd, ²*J* = 13.5, ³*J* = 6.8 Hz, ³*J* = 6.8 Hz, 1H, uD-C^{γ}H^{A}**H**^{B}), 2.55 (m, 3H, uD-SC**H**₃), 2.39 (m, 1H, uA-C^{γ}**H**^{A}H^{B}), 2.25-1,95 (m, 3H, uA-C^{γ}H^{A}**H**^{B}, uD-C^{β}**H**₂), 1.87 (m, 1H, uA-C^{ε}**H**), 1.50 (s, 3H, uA-C(C**H**₃)^{A}(CH₃)^{B}), 1.46 (s, 3H, uA-C(CH₃)^{A}(C**H**₃)^{B}), 1.23-0.96 (m, 9H, uA-C^{ε}-C**H**₃, uC-(C**H**₃)₂).

**¹³C NMR** (126 MHz, Chloroform-d, partly double signal set caused by chiral sulfur) *δ* / *ppm* = 178.7 and 178.5 (uC-CONH-uD), 171.2 and 171.1 (uB-**C**(=O)NH-uC), 170.6 and 170.5 (uD-**C**(=O)O), 165.0 and 164.9 (uA-**C**(=O)NH-uB), 154.2 (uB-**C**^{ar,4}), 142.3 and 142.1 (uA-**C**^{β}), 137.6 and 137.5 (**C**^{ar}), 131.0 and 131.0 (uB-**C**^{ar,2}), 129.6 and 129.5 (**C**^{ar}), 129.0 and 128.8 (uB-**C**^{**ar**,6}), 128.4 and 128.4 (**C**^{ar}), 126.86 and 126.80 (**C**^{ar}), 124.9 (uA-**C**^{α}), 122.7 (uB-**C**^{ar,3}), 112.5 (uB-**C**^{ar,5}), 109.3 and 109.2 (uA-**C**(CH₃)₂), 82.7 and 82.7 (uA-**C**^{ζ}, 80.4 (uA-**C**^{η}), 75.9 and 75.9 (uA-**C**^{δ}), 56.3 (uB-O**C**H₃), 54.7 and 54.6 (uB-**C**^{α}), 51.5 and 51.3 (uD-**C**^{α}), 49.4 (uD-**C**^{γ}), 47.3 and 47.2 (uC-**C**^{α}), 43.2 and 42.9 (uC-**C**^{β}), 38.7 (uA-**C**^{ε}), 38.4 (uD-**C**H₃SO), 37.0 and 36.9 (uA-**C**^{γ}), 35.7 (uB-**C**^{β}), 27.4 and 27.2 (uA-C(**C**H₃)^{A}(CH₃)^{B}), 25.0 and 24.8 (uA-C(CH₃)^{A}(**C**H₃)^{B}), 24.5 (uC-C(**C**H₃)^{A}(CH₃)^{B}), 22.7 and 22.4 (uC-C(CH₃)^{A}(**C**H₃)^{B}), 9.8 and 9.7 (uA-C^{ε}H**C**H₃).

### Cryptophycin-[uA-Diol]-[uD-Met(O)] T5

Acetonide **T4** (229 mg, 301 µmol) was cleaved by 3 cycles of: dissolving in dichlormethan (4 mL) under ice bath cooling adding water (5 drops) and trifluoracetic acid (4 mL), stirred for 5 minutes in the ice bath, then for 10 minutes at RT, removing volatile components under reduced pressure.
After 3 cycles the residue was dried in high vacuum. Column chromatography (d = 2 cm, I = 20 cm, DCM/MeOH 20:1) yielded **T5** as a white foam (47.0 mg, 65.3 µmol, 22 %).

| | | |
|---|---|---|
| **HRMS:** (ESI, +) | m/z (found) | 742.2539 |
| | m/z (calc.) | 742.2569 (M+Na⁺); (NaC₃₅H₄₆ClN₃O₉S⁺). |

**TLC:** R*f* (DCM/MeOH 20:1) = 0.02.

| | | |
|---|---|---|
| **HPLC-MS** (ESI+): | *m*/*z* (found) | 720.27, t_{R} = 7.3 min. |
| | *m*/*z* (calc.) | 720.27 (M+H⁺); (C₃₅H₄₇ClN₃O₉S⁺). |

**¹H NMR** (500 MHz, Methanol-*d*₄, partly double signal set caused by chiral sulfur) *δ l ppm* = 8.33 (d, ³*J* = 7.8 Hz, 1H, uB-NH), 7.92 (t, ³*J* = 7.9 Hz, 1H, uD-NH), 7.69 (m, 1H, uC-NH), 7.43-7.34 (m, 4H, uA-C^{ar}**H**), 7.31 (m, 1H, uA-C^{ar}**H**), 7.26 (d, ⁴*J* = 2.1 Hz, 1H, uB-C^{ar,2}**H**), 7.15 (dd, ³*J* = 8.4, ⁴*J* = 2.1 Hz, 1H, uB-C^{ar,6}**H**), 6.96 (d, ³*J* = 8.5 Hz, 1H, uB-C^{ar,5}**H**), 6.64 (ddd, ³*J* = 15.2, ³*J* = 11.3 Hz, ³*J* = 4.0 Hz, 1H, uA-C^{β}**H**), 5.82 (d, ³*J* = 15.2 Hz, 1H, uA-C^{α}**H**), 5.12 (ddd, ³*J* = 10.9 Hz, ³*J* = 8.5 Hz, ³*J* = 2.3 Hz, 1H, uA-C^{δ}**H**), 4.52 (m, 2H, uD-C^{α}**H**, uA-C^{η}**H**), 4.48 (m, 1H, uB-C^{α}**H**), 3.83 (s, 3H, uB-OC**H**₃), 3.79 (m, 1H, uA-C^{ζ}**H**), 3.39 (d, ²*J* = 13.2 Hz, 1H, uC-C^{β}**H**^{A}H^{B}) 3.19-3.11 (m, 2H, uC-C^{β}H^{A}**H**^{B}, uB-C^{β}**H**^{A}H^{B}), 2.97-2.76 (m, 2H, uD-C^{γ}**H**₂) 2.73 (m, 1H, uB-C^{β}H^{A}**H**^{B}), 2.69 (s, 3H, uD-SC**H**₃), 2.57 (m,1H, uA-C^{γ}**H**^{A}H^{B}), 2.26 (m, 1H, uD-C^{β}**H**^{A}H^{B}), 2.16 (m, 1H, uD-C^{β}H^{A}**H**^{B}), 2.06 (m, 1H, uA-C^{γ}H^{A}**H**^{B}), 1.44 (m, 1H, uA-C^{ε}**H**), 1.20 (s, 3H, uA-C(C**H**₃)^{A}(CH₃)^{B}), 1.18 (s, 3H, uA-C(CH₃)^{A}(C**H**₃)^{B}), 0.97 (d, ³*J* = 7.0 Hz, 3H, uA-C^{ε}C**H**₃).

**¹³C NMR** (126 MHz, Methanol-*d*₄, partly double signal set caused by chiral sulfur) *δ l ppm* = 180.2 and 180.1 (uC-**C**=O), 173.7 and 173.0 (uD-**C**(=O)O), 167.8 and 167.8 (uB-**C**(=O)NH-uC), 162.7 (uA-**C**(=O)NH-uB), 155.3 (uB-**C**^{ar,4}), 143.91 and 143.88 (uA-**C**^{β}), 143.2 and 143.1 (**C**^{ar}), 132.04 (uB-**C**^{ar,1}), 131.4 (uB-**C**^{**ar**,2}), 129.6 (**C**^{ar}), 129.5 (**C**^{ar}), 129.3 (**C**^{ar}), 129.0 (**C**^{ar}), 128.2 (**C**^{ar}), 128.2 (**C**^{ar}), 125.4 (uA-**C**^{α}), 123.2 (uB-**C**^{**ar**,3}), 113.5 (uB-**C**^{ar,5}), 77.4 and 77.3 (uA-**C**^{η}), 76.7 and 76.6 (uA-**C**^{δ}), 75.7 and 75.6 (uA-**C**^{ζ}), 57.4 (uB-**C**^{α}), 56.6 (uB-O**C**H₃), 52.8 and 52.5 (uD-**C**^{α}), 50.8 and 50.7 (uC-**C**^{α}), 49.9 (uD-**C**^{γ}), 48.4 (uD-**C**^{β}), 44.42 and 44.36 (uC-**C**^{α}), 39.7 (uA-**C**^{ε}), 38.3 and 38.2 (uD-S**C**H₃), 37.9 and 37.8 (uA-**C**^{γ}), 36.6 (uB-**C**^{β}), 25.8 and 25.5 (uD-**C**^{β}), 24.7 and 24.6 (uC-C(**C**H₃)^{A}(CH₃)^{B}), 22.8 and 22.7 (uC-C(CH₃)^{A}(**C**H₃)^{B}), 9.9 and 9.9 (uA-C^{ε}**C**H₃).

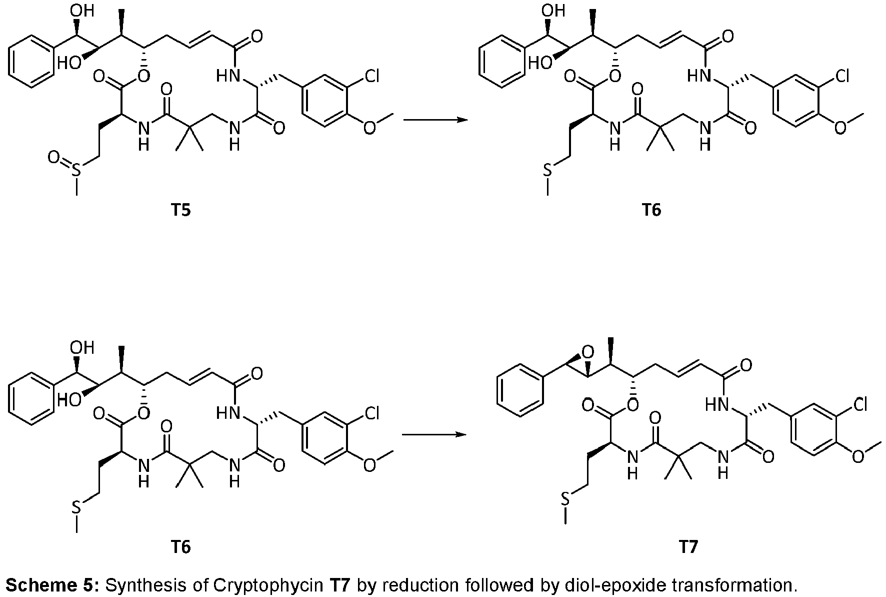

### Cryptophycin-[uA-Diol]-[uD-Met] (T6)

Diol **T5** (47.0 mg, 65.3 µmol) was dissolved in methanol (2 mL), *N*-bromsuccinimide (35 mg, 194 µmol, 3 eq.) and 1,3-dithian (42.5 µmol, 353 µmol, 5.5 eq). The mixture was stirred for 1 hour at 50 °C, volatile components were removed under reduced pressure. Column chromatography (d = 1 cm, I = 20 cm, DCM/MeOH 20:1 → 4:1) yielded **T6** as a white solid (9.0 mg, 12 µmol, 19 %).

| | | |
|---|---|---|
| **HRMS:** (ESI+) | m/z (found) | 704.2746 |
| | m/z (calc.) | 704.2767 (M+H⁺); (NaC₃₅H₄₇ClN₃O₈S⁺) |

**TLC:** R*f* (DCM/MeOH 20:1) = 0.17.

| | | |
|---|---|---|
| **HPLC-MS** (ESI+): | *m*/*z* (found) | 704.25, t_{R} = 8.5 min. |
| | *m*/*z* (calc.) | 704.27 (M+H⁺); (C₃₅H₄₇ClN₃O₈S⁺). |

**¹H NMR** (600 MHz, Chloroform-d) *δ l ppm* = 7.40-7.29 (m, 5H, uA-C^{ar}**H**), 7.17 (d, ⁴*J* = 2.1 Hz, 1H, uB-C^{ar,2}**H**), 7.04 (dd, ³*J* = 8.4 Hz, ⁴J = 2.2 Hz, 1H, uB-C^{ar,6}**H**), 6.90-6.80 (m, 2H, uB-C^{ar,5}**H**, uC-N**H**), 6.70 (ddd, ³*J* = 15.2 Hz, ³*J* = 11.2 Hz, ³*J* = 3.9 Hz, 1H, uA-C^{β}**H**), 6.50 (s, 1H, uD-N**H**), 5.79 (s, 1H, uA-N**H**), 5.67 (d, ³*J* = 14.9 Hz, 1H, uA-C^{α}**H**), 5.14 (m, 1H, uA-C^{δ}**H**), 4.64 (ddd, ³*J* = 7.8 Hz, ³J = 7.8 Hz, ³*J* = 5.0 Hz, 1H, uB-C^{α}**H**), 4.61-4.53 (m, 2H, uA-C^{η}**H**, uD-C^{α}**H**), 3.87 (s, 3H, uB-OC**H**₃), 3.80 (d, ³*J* = 8.3 Hz, 1H, uA-C^{ζ}**H**), 3.38 (d, ³*J* = 10.2 Hz, 1H, uC-C^{β}**H**^{A}H^{B}), 3.24 (d, ³*J* = 13.0 Hz, 1H, uC-CH^{A}**H**^{B}), 3.10 (dd, ²*J* = 14,5 Hz, ³*J* = 4,9 Hz, 1H, uB-C^{β}**H**^{A}H^{B}), 2.89 (dd, ²*J* = 14.6 Hz, ³*J* = 8.4 Hz, 1H, uB-C^{β}H^{A}**H**^{B}), 2.56-2.42 (m, 3H, uD-C^{γ}**H**₂, uA-C^{γ}**H**^{A}H^{B}), 2.34 (m, 1H, uD-C^{β}**H**^{A}H^{B}), 2.15 (ddd, ²*J* = 13.3 Hz, ³*J* = 11.5 Hz, ³*J* = 11.5 Hz, 1H, uA-C^{γ}H^{A}**H**^{B}), 2.01 (m, 1H, uD-C^{β}**H**^{A}H^{B}), 1.91 (m, 1H, uD-C^{β}H^{A}**H**^{B}), 1.48 (m, 1H, uA-C^{ε}**H**), 1.20 (s, 3H, uC-C(C**H**₃)^{A}CH₃)^{B}), 1.15 (s, 3H, uC-C(CH₃)^{A}(C**H**₃)^{B}), 1.01 (d, ³*J* = 6.9 Hz, 3H, uA-C^{ε}C**H**₃).

**¹³C-NMR** (151 MHz, Chloroform-d): *δ l ppm* = 178.0 (uC-CONH-uA), 172.4 (uD-COO), 171.0 (uB-**C**(=O)NH-uC), 165.1 (uA-**C**(=O)NH-uB), 154.3, (uB-**C**^{ar,4}), 142.9 (uA-**C**^{β}), 140.5 (uA-**C**^{ar,1}), 130.9 (uB-**C**^{**a**r,2}), 129.4 (uB-**C**^{ar,1}), 129.0 (**C**^{ar}), 128.7 (**C**^{ar}), 128.3 (uB-**C**^{**ar**,6}), 127.0 (**C**^{ar}), 124.7 (uA-**C**^{α}), 122.7 (uB-**C**^{ar,3}), 112.6 (uB-**C**^{ar,5}), 76.0 (uA-**C**^{δ}, uA-**C**^{η}), 74.9 (uA-**C**^{ζ}), 56.3 (uB-O**C**H₃), 54.9 (uB-**C**^{α}), 51.7 (uD-**C**^{α}), 47.2 (uC-**C**^{β}), 43.5 (uC-**C**^{α}), 38.0 (uA-**C**^{ε}), 36.6 (uA-**C**^{γ}), 35.8 (uB-**C**^{β}), 32.1 (uD-**C**^{β}), 29.8 (uD-**C**^{γ}) 25.1 (uC-C(**C**H₃)^{A}(CH₃)^{B}), 22.6 (uC-C(CH₃)^{A}(**C**H₃)^{B}), 14.3 (uD-S**C**H₃), 9.87 (uA-C^{ε}**C**H₃).

### Cryptophycin-[uD-Met] T7

Trimethylorthoformate (150 µL, 1.37 mmol, 107 eq.) was added to a solution of diol **T6** (9.0 mg, 12.8 µmol, 1 eq.) and PPTS (10.2 mg, 40.6 µmol, 3.2 eq) in dichloromethane (2.0 mL). The mixture was stirred at room temperature for 3 hours. The reaction solution was filtered over silica (d = 1.0 cm, I = 1.5 cm) and eluted with dichloromethane/ ethylacetate (1:1, 60 mL), then concentrated in vacuo and dried overnight in HV. The intermediate orthoester (5.8 mg, 7.7 µmol, 61 %) was dissolved in dichloromethane (2 mL) acetylbromide-solution (0.5 M in abs. DCM, 0.1 mL, 0.05 mmol, 6.5 eq.) was added and the reaction solution stirred at room temperature for 6 hours. The reaction solution was added to sodium bicarbonate solution (half sat., 20 mL). The organic layer was separated, and the aqueous layer was extracted with dichloromethane (3 × 10 mL). The organic layers were dried over MgSO₄, then concentrated in vacuo and dried overnight in HV.

An emulsion of abs. ethylene glycol (2.5 mL), abs. 1,2-dimethoxyethane (5.0 mL) and potassium carbonate (212 mg, 1.53 mmol) was freshly prepared over 3 Å molecular sieves (340 mg) and homogenized by vortexer and ultrasonic bath.

The potassium carbonate emulsion (0.5 mL, 102 µmol, 16. eq.) homogenized by constant shaking was mixed with bromo-formate (5 mg, 6.3 µmol). The mixture was stirred for 5 min at rt then diluted with abs. dichloromethane (10 mL). The solution was given to KHSO₄ solution (0.5 %, 10 mL), phases were separated immediately, and the aqueous phase was further extracted with dichloromethane (3 × 10 mL). The combined organic phases were dried over MgSO₄ and concentrated in vacuo. Column chromatography (d = 0.5 cm, I = 20 cm, EtOAc 100 %) yielded cryptophycin **T7** as white solid foam (1.8 mg, 2.6 µmol, 20 % over 3 steps).

| | | |
|---|---|---|
| **HPLC-MS** (ESI+): | *m*/*z* (found) | 686.12, t_{R} = 8.8 min. |
| | *m*/*z* (calc.) | 686.27 (M+H⁺); (C₃₅H₄₅ClN₃O₇S⁺). |

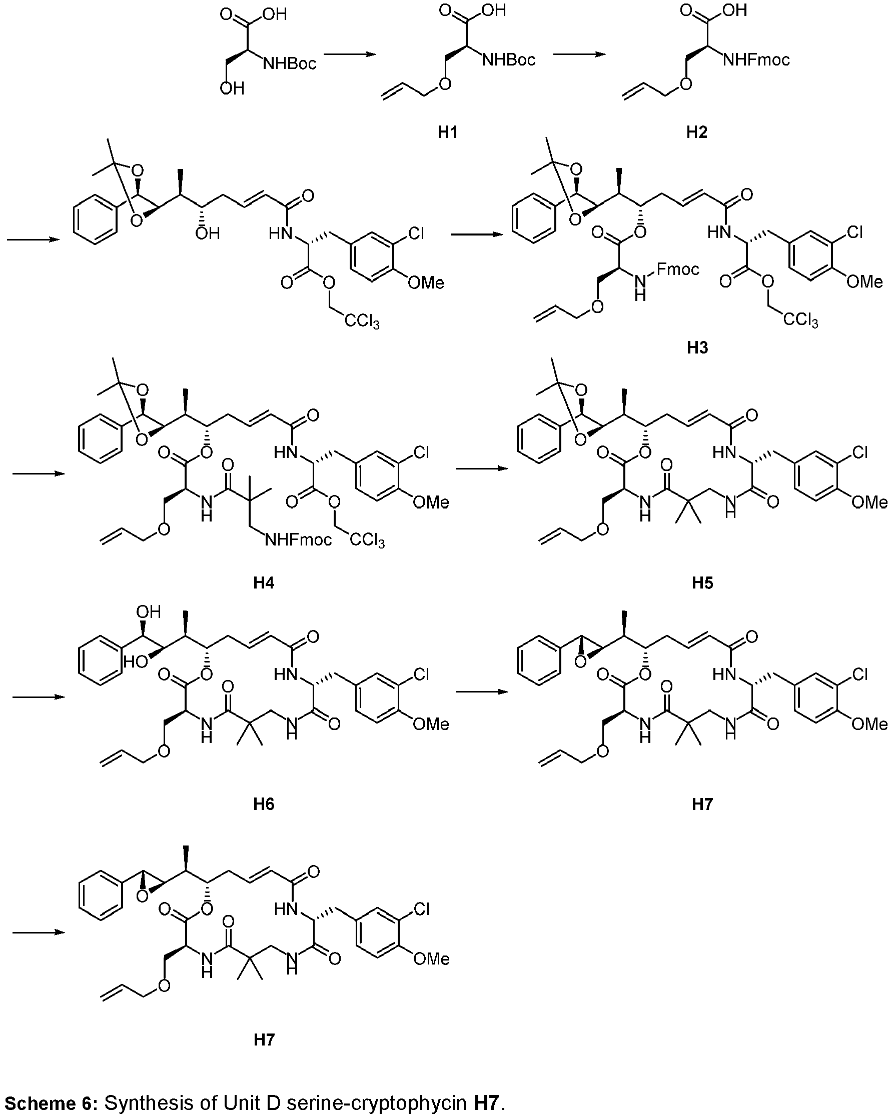

### Boc-L-Ser(All)-OH H1

Under inert conditions sodium hydride (2.47 g of a 60 % oil dispersion, 1.46 g, 61.0 mmol, 2.5 eq.) was suspended in dry dimethylformamide (20 mL) and cooled to 0 °C in an ice-water bath. Boc-L-Ser-OH (4.96 g, 24.2 mmol, 1.0 eq.) was dissolved in dry dimethylformamide (40 mL) and added dropwise via a dropping funnel at 0 °C within 35 min. Afterwards the reaction mixture was warmed to RT, stirred for 20 min and cooled again to 0 °C before adding allyl bromide (2.0 mL, 23.1 mmol, 0.95 eq.). Then the reaction solution was warmed up to RT again and stirred for 3 h. Water (12 mL) was added, and the orange solution was evaporated. The residue was dissolved in water (40 mL) and washed with ethyl acetate (2 x 20 mL). The aqueous phase was then acidified with 6 M HCI to pH = 2 and extracted with ethyl acetate (2 x 40 mL), dried over MgSO₄ and evaporated to yield a yellow oil. The residue was further purified via column chromatography (5 x 20 cm, DCM/MeOH, 9:1) to yield Boc-L-Ser(All)-OH **H1** (4.83 g, 19.2 mmol, 81%) as a colorless viscous oil.

**R*f*** (DCM/MeOH, 9:1) = 0.33.

**¹H-NMR** (500 MHz, Chloroform-d): *δ l ppm* = 5.85 (dddd, ³*J* = 16.2 Hz, ³*J* =10.8 Hz, ³*J* = 5.6 Hz, ³*J* = 5.5 Hz, 1H, C**H**=CH₂), 5.42 (d, ³*J* = 8.5 Hz, 1H, N**H**), 5.25 (d, ³*J* = 17.2 Hz, 1H, CH=C**H**₂*^{trans}*), 5.18 (d, ³*J* = 10.4 Hz, 1H, CH=C**H**₂*^{cis}*), 4.45 (dd, ³*J* = 8.0 Hz, ³*J* = 3.3 Hz, 1H, C^{α}**H**), 4.00 (d, ³*J* = 5.7 Hz, 2H, OC**H**₂CH=CH₂), 3.90 (dd, ²*J* = 9.6 Hz, ³*J* = 3.2 Hz, 1H, C^{β}H^{A}**H**^{B}OH), 3.67 (dd, ²*J* = 9.6 Hz, ³*J* = 3.6 Hz, 1H, C^{β}**H**^{A}H^{B}OH), 1.46 (s, 9H, C(C**H**₃)₃).

### Fmoc-L-Ser(Alll)-OH H2

Boc-L-Ser(All)-OH **H1** (2.50 g, 10.20 mmol, 1.0 eq) was dissolved in dichloromethane (20 mL) and cooled to 0 °C. Trifluoroacetic acid (20.4 mL, 275 mmol, 27.0 eq.) was added, the reaction mixture was stirred at 0 °C for 40 min and co-evaporated with toluene (3 x 10 mL). The residue was dissolved in acetone/water (34 mL, 1:1 v/v) and sodium carbonate (2.23 g, 50.0 mmol, 4.9 eq.) and Fmoc-OSu (3.60 g, 10.70 mmol, 1.1 eq.) were added. After stirring for 50 min at rt the suspension was acidified with 3 M HCl to pH = 1, extracted with ethyl acetate (3 x 30 mL) and the combined organic layers dried over MgSO₄ and evaporated. The residue was further purified via column chromatography (5 x 25 cm, DCM/MeOH, 9:1) to give Fmoc-L-Ser(All)-OH **H2** (696 mg, 1.89 mmol, 19%) as a colorless foam.

**R*_{f}*** (DCM/MeOH, 9:1) = 0.23.

| | | |
|---|---|---|
| **HPLC-MS** (ESI+): | *m*/*z* (found) | 368.0531, *t*_{R} = 9.2 min |
| | *m*/*z* (calc.) | 368.1496 (M+H)⁺ = (C₂₁H₂₂NO₅)⁺. |

**¹H NMR** (500 MHz, Chloroform-d): *δ l ppm* = 7.76 (d, ³*J* = 7.6 Hz, 2H, Fmoc-C^{ar}**H**), 7.60 (dt, ³*J* = 14.2 Hz, ³*J* = 7.2 Hz, 2H, Fmoc-C^{ar}**H**), 7.40 (t, ³*J* = 7.5 Hz, 2H, Fmoc-C^{ar}**H**), 7.31 (t, ³*J* = 7.4 Hz, 2H, Fmoc-C^{ar}**H**), 5.87 (dddd, ³*J* = 16.4 Hz, ³*J* = 10.9 Hz, ³*J* = 5.7 Hz, ³*J* = 5.6 Hz, 1H, C**H**=CH₂), 5.69 (d, ³*J* = 8.4 Hz, 1H, N**H**), 5.28 (d, ³*J* = 18.3 Hz, 1H, CH=C**H**₂*^{trans}*) , 5.21 (d, ³*J* = 10.5 Hz, 1H, CH=C**H**₂*^{cis}*), 4.54 (dd, ³*J* = 8.1 Hz, ³*J* = 3.3 Hz, 1H, C^{α}**H**), 4.45 - 4.35 (m, 2H, Fmoc-C**H**₂), 4.25 (t, ³*J* = 7.2 Hz, 1H, Fmoc-C**H**), 4.03 (d, ³*J* = 5.7 Hz, 2H, OC**H**₂CH=CH₂), 3.96 (dd, ²*J* = 9.4 Hz, ³*J* = 2.6 Hz, 1H, C^{β}**H**^{A}H^{B}), 3.72 (dd, ²*J* = 9.7 Hz, ³*J* = 3.6 Hz, 1H, C^{β}H^{A}**H^{B}**).

### Fmoc-uD[Ser(All)]-uA[Acetonide]-uB-OTCE H3

Building block A-B was synthesized according to SEWALD *et al.* (N. Sewald et al., J. Org. Chem. 2010, 75, 6953-6960).
Under inert conditions unit A-B (549 mg, 0.83 mmol, 1.0 eq.), **H2** (305 mg, 0.83 mmol, 1.0 eq.) and DMAP (12 mg, 0.10 mmol, 0.1 eq.) were dissolved in dry THF (12 mL) and stirred at 0 °C. Triethylamine (227 µL, 1.66 mmol, 2.0 eq.) followed by 2,4,6-trichlorobenzoyl chloride (259 µL, 1.66 mmol, 2.0 eq.) were added dropwise. The reaction mixture was stirred at 0 °C for 4.5 h. A solution of citric acid (10 %, 25 mL) in water was added and the organic layer was separated. The aqueous layer was extracted with ethyl acetate (3 x 50 mL). The combined organic layers were washed with brine (25 mL), dried over MgSO₄ and evaporated to yield a grew foam. This was further purified via column chromatography (3 x 20 cm, PE/EtOAc, 2:1) to give compound **H3** (474 mg, 0.47 mmol, 56%) colorless foam.

**TLC:** R*_{f}* (PE/EtOAc, 2:1) = 0.22

**¹H NMR** (500 MHz, Chloroform-d): *δ l ppm* = 7.74 (d, ³*J* = 7.5 Hz, 2H, uD-Fmoc-C^{ar}**H**), 7.60 (dd, ³*J* = 7.6 Hz, ⁴*J* = 2.3 Hz, 2H, uD-Fmoc-C^{ar}**H**), 7.37 (t, ³*J* = 7.5 Hz, 2H, uD-Fmoc-C^{ar}**H**), 7.31 (t, ³*J* = 7.4 Hz, 2H, uD-Fmoc-C^{ar}**H**), 7.35 - 7.28 (m, 5H, uA-C^{ar}**H**), 7.13 (d, ³*J* = 2.0 Hz, 1H, uB-C^{ar,2}**H**), 6.96 (dd, ³*J* = 8.5 Hz, ⁴*J* = 2.1 Hz, 1H, uB-C^{ar,6}**H**), 6.74 (d, ³*J* = 8.4 Hz, 1H, uB-C^{ar,5}**H**), 6.65 (d, ³*J* = 7.8 Hz, 1H, uB-N**H**), 6.56 (ddd, ³*J* = 15.7 Hz, ³*J* = 6.5 Hz, ³*J* = 6.5 Hz, 1H, uA-C^{β}**H**), 5.84 (dddd, ³*J* = 16.5 Hz, ³*J* = 10.9 Hz, ³*J* =5.7 Hz, ³*J* = 5.6 Hz, 1H, uD-C**H**=CH₂), 5.76 (dd, ³*J* = 8.7, ⁴*J* = 2.8 Hz, 1H, uB-NH), 5.55 (d, ³*J* = 15.6 Hz, 1H, uA-C^{α}**H**), 5.26 (d, ³*J* = 17.8 Hz, 1H, uD-CH=C**H**₂*^{trans}*), 5.21 (d, ³*J* = 10.3 Hz, 1H, uD-CH=C**H**₂*^{cis}*), 5.03 (ddd, ³*J* = 8.6 Hz, ³*J* = 4.6 Hz, ³*J* = 4.6 Hz, 1H, uA-C^{δ}**H**), 4.96 (dd, ³*J* = 6.9 Hz, ³*J* = 6.9 Hz, 1H, uB-C^{α}**H**), 4.72 (d, ³*J* = 8.7 Hz, 1H, uA-C^{η}**H**), 4.67 (d, ²*J* = 11.9 Hz, 1H, uB-C**H**^{A}H^{B}CCl₃), 4.59 (d, ²*J* = 11.9 Hz, 1H, uB-CH^{A}**H^{B}**CCl₃), 4.38 (dd, ²*J* = 10.4 Hz, ³*J* = 7.0 Hz, 1H, uD-Fmoc-C**H**₂), 4.33 (ddd, ³*J* = 7.5 Hz, ³*J* = 3.3 Hz, ³*J* = 3.3 Hz, 1H, uD-C^{α}**H**), 4.26 (dd, ²*J* = 10.5 Hz, ³*J* = 7.7 Hz, 1H, uD-Fmoc-C**H**₂), 4.19 (t, ³*J* = 7.3 Hz, 1H, uD-Fmoc-C**H**), 3.95 (d, ³*J* = 5.7 Hz, 2H, uD-OC**H**₂CH=CH₂), 3.85 (dd, ³*J* = 8.8 Hz, ³*J* = 2.9 Hz, 1H, uA-C^{ζ}**H**), 3.78 (m, 1H, uD-C*^{β}***H**^{A}H^{B}), 3.76 (s, 3H, uB-OC**H**₃), 3.66 (dd, ²*J* = 9.7 Hz, ³*J* = 3.3 Hz, 1H, uD-C^{β}H^{A}**HB**), 3.10 (dd, ²*J* = 14.2 Hz, ³*J* = 5.8 Hz, 1H, uB-C^{β}**H**^{A}H^{B}), 2.96 (dd, ²*J* = 14.1 Hz, ³*J* = 6.8 Hz, 1H, uB-C^{β}H^{A}**H^{B}**), 2.42-2.29 (m, 2H, uA-C^{γ}**H**z), 2.00 (ddq, ³*J* = 10.1 ³*J* = 10.1, ³*J* = 5.1 Hz, 1H, uA-C^{ε}**H**), 1.53 (s, 3H, uA-C(C**H**₃)^{A}(CH₃)^{B}), 1.48 (s, 3H, uA-C(CH₃)^{A}(C**H**₃)^{B}), 1.10 (d, ³*J* = 6.8 Hz, 3H, uA-C^{ε}HC**H**₃).

**¹³C NMR** (126 MHz, Chloroform-*d*): *δ* /*ppm* = 169.9 (uB-**C**(=O)), 169.7 (uD-**C**O₂CH), 165.2 (uA-**C**ONH₂), 156.3 (uD-Fmoc-N**C**O₂), 154.0 (uB-**C**^{ar,4}), 143.9 (uD-Fmoc-**C**), 143.5 (uD-Fmoc-**C**), 141.2 (uD-Fmoc-**C**), 139.1 (uA-**C**^{β}H), 137.6 (uA-**C**^{ar}), 133.8 (uD-**C**H=CH₂), 131.2 (uB-**C**^{ar,2}H), 128.8 (uB-**C**^{ar,5}), 128.7 (uA-**C**^{ar}H), 128.5 (uB-**C**^{ar,6}H), 128.4 (uA-**C**^{ar}H), 127.7 (uA-**C**^{ar}H), 127.0 (uD-Fmoc-**C**^{ar}), 126.9 (uD-Fmoc-**C**^{ar}), 125.3 (uA-**C**^{α}H) 125.2 (uD-Fmoc-**C**^{ar}), 122.1 (uB-**C**^{ar,3}), 120.0 (uD-Fmoc-**C**^{ar}), 117.7 (uD-CH=**C**H₂), 112.0 (uB-**C**^{ar,5}H), 109.0 (uA-**C**(CH₃)₂), 94.3 (uB-CH₂**C**Cl₃), 82.4 (uA-**C**^{ζ}H), 80.6 (uA-**C**^{η}H), 75.5 (uA-**C**^{δ}H), 74.5 (uB-**C**H₂CCl₃), 72.2 (uD-O**C**H₂CH=CH₂), 69.0 (uD-**C**^{β}Hz), 67.4 (uD-Fmoc-**C**H₂), 56.0 (uB-O**C**H₃), 54.6 (uD-**C**^{α}H), 53.1 (uB-**C**^{α}H), 46.9 (uD-Fmoc-**C**H), 36.4 (uB-**C**^{β}H₂), 36.1 (uA-**C**^{ε}HCH₃), 31.9 (uA-**C**^{γ}H), 27.2 (uA-C(**C**H₃)₂), 27.1 (uA-C(**C**H₃)₂), 9.4 (uA-C^{ε}H**C**H₃).

### Fmoc-uC-uD[Ser(All)]-uA[Acetonide]-uB-OTce H4

Piperidine (0.30 mL, 3.01 mmol, 5.2 eq.) was added dropwise to a solution of **H3** (585 mg, 0.58 mmol, 1.0 eq.) in dimethylformamide (20 mL) at 0 °C. The solution was warmed up to RT, stirred for 35 min and evaporated under high vacuum. The residue was co-evaporated with toluene (2 x 20 mL) and dried in vacuum to yield a yellow solid. The primary amine was used without further purification in the next step.
Under inert conditions Fmoc-3-amino-2,2-dimethyl-propionic acid (394 mg, 1.16 mmol, 2.0 eq.), *N,N*-diisopropylethylamine (0.50 mL, 2.90 mmol, 5.0 eq.) and 1-hydroxy-7-azabenzotriazole (174 mg, 1.28 mmol, 2.2 eq.) were dissolved in dry dichloromethane (30 mL) and stirred at 0 °C. *N,N*'-diisopropylcarbodiimide (0.20 mL, 1.28 mmol, 2.2 eq.) was added dropwise to the solution over 10 min and stirred for an additional 10 min. The reaction mixture was added dropwise to a solution of the deprotected unit DAB (0.58 mmol, 1.0 eq.) in dry dimethylformamide (6 mL) at 0 °C within 20 min. After stirring at RT for 17.5 h the solution was given to a solution of citric acid (10 %, 100 mL) in water. The organic layer was separated, and the aqueous layer was extracted with ethyl acetate (3 x 50 mL). The combined organic layers were washed with sodium hydrogen carbonate solution (50 %, 50 mL) and brine (50 mL), were dried over MgSO₄ and evaporated. The residue was further purified via column chromatography (3x20 cm, PE/EtOAc, 1:1) to yield compound **H4** (570 mg, 0.51 mmol, 88%) as a colorless foam.

**TLC: R***_{f}*(PE/EtOAc, 1:1) = 0.34.

**¹H NMR** (500 MHz, Chlorform-d): δ /ppm = 7.75 (d, ³*J* = 7.6 Hz, 2H, uC-Fmoc-C^{ar}**H**), 7.59 (d, ³*J* = 7.9 Hz, 2H, uC-Fmoc-C^{α}**H**), 7.41 - 7.35 (m, 2H, uC-Fmoc-C^{ar}**H**), 7.34-7.31 (m, 2H, uC-Fmoc-C^{ar}**H**), 7.30 - 7.22 (m, 5H, uA-C^{ar}**H**), 7.16 (d, ⁴*J* = 2.1Hz, 1H, uB-C^{ar,2}**H**), 7.04 (dd, ³*J* = 8.5 Hz, ⁴*J* = 2.2 Hz, 1H, uB-C^{ar,6}**H**), 6.91 (d, ³*J* = 8.0 Hz, 1H, uB-N**H**), 6.80 (d, ³*J* = 8.4 Hz, 1H, uB-C^{ar,5}**H**), 6.57 (ddd, ³*J* = 15.6 Hz, ³*J* = 6.6 Hz, ³*J* = 6.6 Hz, 1H, uA- C^{β}**H**), 6.54 (d, ³*J* = 7.4 Hz, 1H, uD-N**H**), 5.88 (dd, ³*J* = 6.6 Hz, ³*J*=6.6 Hz, 1H, uC-N**H**), 5.80 (dddd, ³*J* = 16.3 Hz, ³*J* = 10.8 Hz, ³*J* = 5.6 Hz, ³*J* = 5.6 Hz, 1H, uD-C**H**=CH₂), 5.61 (d, ³*J* = 15.7 Hz, 1H, uA-C^{α}**H**), 5.23 (dd, ³*J* = 17.2 Hz, ²*J* = 1.7 Hz, 1H, uD-CH=C**H**₂*^{trans}*), 5.18 (d, ³*J* = 10.4Hz, 1H, uD-CH=C**H**₂*^{cis}*), 5.06 (m, 1H, uA-C^{δ}**H**), 4.99 (dd, ³*J* = 7.1 Hz, Hz, ³*J* = 7.1 Hz, 1H, uB-C^{α}**H**), 4.79 (d, ²*J* = 11.9 Hz, 1H, uB-C**H**^{A}H^{B}CCl₃), 4.68 (d, ²*J* = 11.9 Hz, 1H, uB-CH^{A}**H^{B}**CCl₃), 4.67 (d, ³*J* = 8.7 Hz, 1H, uA-C^{η}**H**), 4.48 (ddd, ³*J* = 7.4 Hz, ³*J* = 3.4 Hz, ³*J* = 3.4 Hz, 1H, uD-C^{α}**H**), 4.37 - 4.30 (m, 2H, uC-Fmoc-C**H**₂), 4.19 (t, ³*J* = 7.5 Hz, 1H, uC-Fmoc-C**H**), 3.95 (d, ³*J* = 5.7 Hz, 2H, uD-OC**H**₂CH=CH₂), 3.83 (s, 3H, uB-OC**H**₃), 3.82 (m, 1H, uA-C^{ζ}**H**), 3.81 (dd, ²*J* = 8.1 Hz, ³*J* = 3.1 Hz, 1H, uD-C*^{β}***H^{A}**H^{B}), 3.65 (dd, ²*J* = 9.8 Hz, ³*J* = 3.4 Hz, 1H, uD-C^{β}H^{A}**H^{B}**), 3.37 (dd, ²*J* = 13.8 Hz, ³*J* = 7.5 Hz, 1H, uC-C^{β}**H^{A}**H^{B}), 3.33 (dd, ²*J* = 13.7Hz, ³*J* = 5.6Hz, 1H, uC-C^{β}H^{A}**H^{B}**), 3.19 (dd, ²*J* = 14.2 Hz, ³*J* = 5.6 Hz, 1H, uB-C^{β}**H**^{A}H^{B}), 3.03 (dd, ²*J* = 14.2 Hz, ³*J* = 7.3 Hz, 1H, uB-C^{β}H^{A}**H^{B}**), 2.30 - 2.45 (m, 2H, uA-C^{γ}**H**₂), 2.00 (m, 1H, uA-C^{ε}**H**), 1.50 (s, 3H, uA-C(C**H**₃)^{A}(CH₃)^{B}), 1.43 (s, 3H, uA-C(CH₃)^{A}(C**H**₃)**^{B}**), 1.25 (s, 3H, uC-C(C**H**₃)^{A}(CH₃)^{B}), 1.17 (s, 3H, uC-C(CH₃)^{A}(C**H**₃)**^{B}**), 1.11 (d, ³*J* = 6.9 Hz, 3H, uA-C^{ε}C**H**₃).

### Cryptophycin-[uA-Acetonide]-[uD-Ser(All)] H5

Piperidine (0.30 mL, 3.01 mmol, 5.2 eq.) was added dropwise to a solution of **H4** (535 mg, 0.48 mmol, 1.0 eq.) in dimethylformamide (15 mL) at 0 °C. The solution was warmed up to RT, stirred for 15.5 h. Then water (200 mL) and ethyl acetate (200 mL) were added to the yellow solution, the organic layer was separated, and the aqueous layer was extracted with ethyl acetate (3 x 200 mL). The combined organic layers were dried over MgSO₄ and evaporated. The residue was further purified via column chromatography (3 x 25 cm, EtOAc/MeOH, 96:4) to give compound **H5** (247 mg, 0.33 mmol, 70%) as a colorless foam.

**TLC:** R*_{f}*(EtOAc/MeOH, 96:4) = 0.41.

| | | |
|---|---|---|
| **HPLC-MS** (ESI+): | *m*/*z* (found) | 740.35, *t*_{R} = 10.4 min |
| | *m*/*z* (calc.) | 740.33 (M+H)⁺ = (C₃₉H₅₁ClN₃O₉)⁺ |

**¹H NMR** (500 MHz, Chloroform-*d*): δ /*ppm* = 7.39 - 7.31 (m, 5H, uA-C^{ar}**H**), 7.17 (d, ⁴*J* = 2.1 Hz, 1H, uB-C^{ar,2}**H**), 7.03 (dd, ³*J* = 8.4 Hz, ⁴*J* = 2.2 Hz, 1H, uB-C^{ar,6}**H**), 6.83 (d, ³*J* = 8.4 Hz, 1H, uB-C^{ar,5}**H**), 6.80 (dd, ³*J* = 8.4 Hz, ³*J* = 3.6 Hz, 1H, uC-N**H**), 6.62 (ddd, ³*J* = 15.2 Hz, ³*J* = 11.1 Hz, ³*J* = 4.3 Hz, 1H, uA-C^{β}**H**), 6.30 (d, ³*J* = 8.0 Hz, 1H, uD-N**H**), 5.75 (ddt, ³*J* = 17.3 Hz, ³*J* = 10.8 Hz, ³*J* = 5.6 Hz, 1H, uD-C**H**=CH₂), 5.63 (d, ³*J* = 15.4 Hz, 1H, uA-C^{α}**H**), 5.61 (d, ³*J* = 7.8, 1H, uB-N**H**), 5.19 (dd, ³*J* = 17.2 Hz, ²*J* = 1.7 Hz, 1H, uD-CH=C**H**₂*^{trans}*), 5.14 (dd, ³*J* = 10.5 Hz, ²*J* = 1.5 Hz, 1H, uD-CH=C**H**₂*^{cis}*), 5.11 (ddd, ²*J* = 11.3 Hz, ³*J* = 4.8 Hz, ³*J* = 2.1 Hz, 1H, uA-C^{δ}**H**), 4.69 (d, ³*J* = 8.8 Hz, 1H, uA-C^{η}H), 4.66 (ddd, ³*J* = 7.8 Hz, ³*J* = 7.8 Hz, 5.0 Hz, 1H, uB-C^{α}**H**), 4.46 (ddd, ³*J* = 8.6 Hz, ³*J* = 4.8 Hz, ³*J* = 4.7 Hz, 1H, uD-C^{α}**H**), 3.88 (d, ³*J* = 5.6 Hz, 2H, uD-OC**H**₂CH=CH₂), 3.87 (s, 3H, uB-OC**H**₃), 3.80 (dd, ³*J* = 8.8 Hz, ³*J* = 2.4 Hz, 1H, uA-C^{ζ}**H**), 3.49 (d, ³*J* = 4.8 Hz, 2H, uD-C^{β}**H**₂), 3.37 (dd, ²*J* = 13.1 Hz, ³*J* = 8.3 Hz, 1H, uC-C**H^{A}**H^{B}), 3.24 (dd, ²*J*= 13.2 Hz, ³*J* = 3.6 Hz, 1H, uC-CH^{A}**H^{B}**), 3.10 (dd, ²*J* = 14.6 Hz, ³*J* = 4.9 Hz, 1H, uB-C^{β}**H^{A}**H^{B}), 2.94 (dd, ²*J* = 14.5 Hz, ³*J* = 8.1 Hz, 1H, uB-C^{β}CH^{A}**H^{B}**), 2.42 (ddd, ²*J* = 12.0 Hz, ³*J* = 4.4 Hz, ³*J* = 2.2 Hz, 1H, uA-C^{γ}**H^{A}**H^{B}), 2.17 (ddd, ²*J* = 14.2 Hz, ³*J* = 11.2 Hz, ³*J* = 2.7 Hz, 1H, uA-C^{γ}H^{A}H**^{B})**, 1.87 (qdd, ³*J* = 6.9 Hz, ³*J* = 6.6 Hz, ³*J* = 2.4 Hz, 1H, uA-C^{ε}**H**), 1.50 (s, 3H, uA-C(C**H**₃)^{A}(CH₃)^{B}), 1.46 (s, 3H, uA-C(CH₃)^{A}(C**H**₃)^{B}), 1.20 (s, 3H, uC-C(CH₃)^{A}(C**H**₃)^{B}), 1.12(s, 3H, uC-C(CH₃)^{A}(C**H**₃)^{B}), 1.11 (d, ³*J* = 6.9 Hz, 3H, uA-C^{ε}C**H**₃).

**¹³C NMR** (126 MHz, Chloroform-d): δ/ppm = 177.8 (uC-**C**(=O)), 170.6 (uB-**C**(=O)), 170.0 (uD-**C**(=O)), 164.9 (uA-**C**(=O)), 154.3 (uB-**C**^{ar,4}OCH₃), 142.8 (uA-**C**^{β}H), 137.7 (uA-**C**^{ar}), 133.9 (uD-**C**H=CH₂), 131.0 (uB-**C**^{ar,2}H), 129.6 (uB-**C**^{ar,5}H), 128.9 (uA-**C**^{ar}H), 128.7 (uA-**C**^{ar}H), 128.3 (uB-**C**^{ar,6}H), 126.8 (uA-**C**^{ar}H), 124.5 (uA-**C**^{α}H), 122.7 (uB-**C**^{ar,3}), 117.8 (uD-CH=**C**H₂), 112.5 (uB-**C**^{ar,5}), 109.2 (uA-**C**(CH₃)₂), 82.5 (uA-**C**^{ζ}H), 80.4 (uA-**C**^{η}H), 75.6 (uA-**C**^{δ}H), 72.2 (uD-O**C**H₂CH=CH₂), 68.9 (uD-**C**^{β}H₂), 56.3 (uB-O**C**H₃), 54.8 (uB-**C**^{α}H), 52.5 (uD-**C**^{α}H), 47.3 (uC-**C**H₂), 43.3 (uC-**C**(CH₃)₂), 36.7 (uA-**C**^{ε}H), 35.8 (uB-**C**^{β}Hz), 35.9 (**C**^{γ}H), 27.3 (uA-C(**C**H₃)^{A}(CH₃)^{B}), 27.2 (uA-C(CH₃)^{A}(**C**H₃)^{B}), 24.8 (uC-C(**C**H₃)^{A}(CH₃)^{B}), 22.6 (uC-C(CH₃)^{A}(**C**H₃)^{B}), 9.5 (uA-C^{ε}H**C**H₃).

### Cryptophycin-[uA-Diol]-[uD-Ser(All)] H6

To a solution of acetonide protected cryptophycin **H5** (237 mg, 0.32 mmol, 1.0 eq.) in dichloromethane (4 mL) at 0 °C trifluoroacetic acid (4 mL) was added dropwise. The yellow solution was warmed up to RT, stirred for 30 min and evaporated. The residue was dissolved in dichloromethane (4 mL), cooled to 0 °C and trifluoroacetic acid (4 mL) was added dropwise. After stirring for 30 min at RT and evaporating again, the residue was co-evaporated with toluene (2 mL). Ethyl acetate (100 mL) and sat. NaHCO₃-solution (70 mL) were added and the organic layer was separated. The aqueous layer was extracted with ethyl acetate (3 x 70 mL). The combined organic layers were dried over MgSO₄ and evaporated. The residue was further purified via column chromatography (3x20 cm, EtOAc/MeOH, 95:5) to give compound **H6** (129 mg, 0.18 mmol, 58%) as a colorless frozen foam.

**TLC:** R*_{f}* (EtOAc/MeOH, 95:5)= 0.26.

| | | |
|---|---|---|
| **HPLC-MS** (ESI+): | *m*/*z* (found) | 700.323 , *t*_{R} = 8.6 min |
| | *m*/*z* (calc.) | 700.30 (M+H)⁺ = (C₃₆H₄₇ClN₃O₉)⁺ |

**¹H NMR** (500 MHz, Chloroform-d): *δ* /*ppm* = 7.37 -7.30 (m, 5H, uA-C^{ar}**H**), 7.16 (d, ⁴*J* = 2.0 Hz, 1H, uB-C^{ar,2}**H**), 7.03 (dd, ³*J* = 8.5 Hz, ⁴*J* = 2.1 Hz, 1H, uB-C^{ar,6}**H**), 7.10 (dd, ³*J* = 9.0 Hz, ³*J* = 3.4 Hz, 1H, uC-N**H**), 6.83 (d, ³*J* = 8.4 Hz, 1H, uB-C^{Ar,5}**H**), 6.76 (dd, ³*J* = 15.1 Hz, ³*J* = 10.9 Hz, ³*J* = 4.1 Hz, 1H, uA-C^{β}**H**), 6.28 (d, ³*J* = 7.4 Hz, 1H, uD-N**H**), 5.74 (dddd, ³*J* = 17.4 Hz, ³*J* = 10.7 Hz, ³*J* = 5.4 Hz, ³*J* = 5.4 Hz, 1H, uD-C**H**=CH₂), 5.68 (d, ³*J* = 15.3 Hz, 1H, uA-C^{α}**H**), 5.58 (m, 1H, uB-N**H**) 5.22 (ddd, ³*J* = 11.9 Hz, ³*J* = 5.5 Hz, ³*J* = 2.3 Hz, 1H, uA-C^{δ}**H**), 5.18 (dd, ³*J* = 17.4Hz, ²*J* = 1.6 Hz, 1H, uD-CH=C**H**₂*^{trans}*), 5.16 (dd, ³*J* = 10.4, ²*J* = 1.4 Hz, 1H, uD-CH=C**H**₂^{cis}), 4.69 (dd, ³*J* = 7.7 Hz, ³*J* = 4.8 Hz, 1H, uB-C^{α}**H**), 4.46 (ddd, ³*J* = 8.0 Hz, ³*J* = 4.9 Hz, ³*J* = 4.8 Hz, 1H, uD-C^{α}**H**), 3.83 (s, 3H, uB-OC**H**₃), 3.78 (dd, ³*J* = 5.7 Hz, ⁴*J* = 1.4 Hz, 2H, uD-OC**H₂**CH=CH₂), 3.67 (d, ³*J* = 2.0 Hz, 1H, uA-C^{η}-**H**), 3.43 (dd, ³*J* = 4.8 Hz, ³*J* = 1.5 Hz, 2H, uD-C^{β}**H₂**), 3.39 (dd, ²*J* = 13.2 Hz, ³*J* = 8.7 Hz, 1H, uC-C^{β}**H^{A}**H^{B}), 3.18 (dd, ²*J* = 13.2 Hz, ³*J* = 3.3 Hz, 1H, uC-C^{β}H^{A}**H^{B}**), 3.09 (dd, ²*J* = 14.5, ³*J* =4.9 Hz, 1H, uB-C^{β}**H^{A}**H^{B}), 2.98 (ddd, ²*J* = 14.6 Hz, ³*J* = 7.9 Hz, ³*J* = 2.0 Hz, 1H, uB-C^{β}H^{A}**H^{B}**), 2.90 (dd, ³*J* = 7.0 Hz, ³*J* = 2.0 Hz, 1H, uA-C^{ζ}**H**), 2.57 (ddd, ²*J* = 12.2 Hz, ³*J* = 4.3, ³*J* = 2.2 Hz, 1H, uA-C^{γ}**H^{A}**H^{B}), 2.41 (ddd, ²*J* = 14.2 Hz, ³*J* = 11.2 Hz, ³*J* = 2.7 Hz, 1H, uA-C^{γ}H^{A}**H^{B}**), 1.77 (qdd, ³*J* = 7.2 Hz, ³*J* = 7.2 Hz, ³*J* = 7.0 Hz, 1H, uA-C^{ε}**H**), 1.19 (s, 3H, uC-C(C**H₃**)^{A}(CH₃)^{B}), 1.14 (s, 3H, uC-C(CH₃)^{A}(C**H₃**)^{B}), 1.14 (d, ³*J* = 6.9 Hz, 3H, uA-C^{ε}C**H**₃).

### Cryptophycin-[uD-Ser(All)] H7

To a solution of diol **H6** (129 mg, 0.18 mmol, 1.0 eq.) and pyridinium *p*-toluene sulfonate (113 mg, 0.45 mmol, 2.5 eq.) in dichloromethane (5 mL) trimethyl orthoformate (1.80 mL, 16.44 mmol, 91.3 eq.) was added. After stirring at RT for 16 h the reaction mixture was filtered over silica (1 x 5 cm) and eluted with dichloromethane/ethyl acetate (300 mL, 1:1 v/v), then dried under vacuum to yield a colorless foam.

The intermediate orthoester (0.18 mmol, 1.0 eq.) was dissolved in dichloromethane (2.5 mL) and an acetylbromide-solution (0.5 M in dry dichloromethane, 0.85 mL, 0.45 mmol, 2.5 eq.) was added. The reaction mixture was stirred at RT for 4.5 h and then added to dichloromethane (20 mL) and NaHCO₃-solution (50% sat., 50 mL). The organic layer was separated and the aqueous layer was extracted with dichloromethane (3x20 mL). The combined organic layers were dried over MgSO₄ and evaporated. The bromo formate was dried under vacuum to yield a colorless foam.

An emulsion of dry ethylene glycol (2.5 mL), dry 1,2-dimethoxypropane (5.0 mL) and potassium carbonate (209 mg) was freshly prepared over 3 Å molecular sieves (350 mg) and homogenized by an vortexer and ultrasonic bath.

The potassium carbonate emulsion (4.5 mL, 0.91 mmol, 5.0 eq. K₂CO₃) homogenized by constant shaking was added to (0.18 mmol, 1.0 eq.). The reaction mixture was vigorous stirred at RT for 6 min and diluted with dry dichloromethane (20 mL). The solution was given to a cold KHSO₄-solution (0.5 %, 20 mL), the organic layer was separated immediately, and the aqueous layer was extracted with dichloromethane (3x20 mL). The combined organic layers were dried over MgSO₄ and evaporated. The residue was further purified via column chromatography (2 x 20 cm, pure EtOAc) to give compound **H7** (45 mg, 0.066 mmol, 37% over 3 steps) as a colorless foam.

**TLC: R***_{f}*(EtOAc) = 0.26.

| | | |
|---|---|---|
| **HPLC-MS** (ESI+): | *m*/*z* (found) | 682.31, *t*_{R} = 9.9 min |
| | *m*/*z* (calc.) | 682.29 (M+H)⁺ = (C₃₆H₄₅ClN₃O₈)⁺ |

**¹H NMR** (500 MHz, Chloroform-d) δ /ppm = 7.35 (m, 3H, uA- C^{ar}-**H**), 7.24 (dd, ³*J* = 7.8 Hz, ⁴*J* = 1.7 Hz, 2H, uA-C^{ar}**H**), 7.17 (d, ³*J* = 2.2 Hz, 1H, uB-C^{ar,2}**H**), 7.03 (dd, ³*J* = 8.4 Hz, ⁴*J* = 2.2 Hz, 1H, uB-C^{ar,6}**H**), 6.98 (dd, ³*J* = 8.9 Hz, ³*J* = 3.4 Hz, 1H, uC-N**H**), 6.83 (d, ³*J* = 8.4 Hz, 1H, uB-C^{ar,5}**H**), 6.76 (ddd, ³*J* = 15.0 Hz, ³*J* = 10.9 Hz, ³*J* = 4.1 Hz, 1H, uA-C^{β}**H**), 6.28 (d, ³*J* = 7.9 Hz, 1H, uD-N**H**), 5.74 (ddt, ³*J* = 17.5 Hz, ³*J* = 10.8 Hz, ³*J* = 5.4 Hz, 1H, uD-H₂C=C**H**), 5.68 (dd, ³*J* = 15.0 Hz, ⁴*J* = 1.8 Hz, 1H, uA-C^{α}**H**), 5.58 (m, 1H, uB-N**H**), 5.22 (m, 1H, uA-C^{δ}**H**), 5.20 - 5.14 (m, 2H, uD-**H₂**C=CH), 4.69 (ddd, ³*J* = 7.7 Hz, ³*J* = 7.7 Hz, ³*J* = 4.8 Hz, 1H, uB-C^{α}**H**), 4.46 (ddd, ³*J* = 8.0 Hz, ³*J* = 4.9 Hz, ³*J* = 4.9 Hz, 1H, uD-C^{α}**H**), 3.87 (s, 3H, uB-OC**H₃**), 3.78 (dd, ³*J* = 5.7 Hz, ⁴*J* = 1.4 Hz, 2H, uD-H₂C=CHC**H₂**), 3.67 (d, ³*J* = 2.0 Hz, 1H, uA-C^{η}**H**), 3.43 (m, 2H, uD-C^{β}**H**₂), 3.39 (dd, ²*J* = 13.2 Hz, ³*J* = 8.7 Hz, 1H, uC-C^{β}**H^{A}**H^{B}), 3.18 (dd, ²*J* = 13.2 Hz, ³*J* = 3.5 Hz, 1H, uC-C^{β}H^{A}**H^{B}**), 3.09 (dd, ²*J* = 14.5 Hz, ³*J* = 4.9 Hz, 1H, uB-C^{β}**H^{A}**H^{B}), 2.98 (ddd, ²*J* = 14.5 Hz, ³*J* = 7.9 Hz, 1H, uB-C^{β}H^{A}**H^{B}**), 2.90 (dd, ³*J* = 7.7 Hz, ³*J* = 2.0 Hz, 1H, uA-C^{ζ}**H**), 2.57 (dddd, ²*J* = 14.2 Hz, ³*J* = 4.3 Hz, ³*J* = 2.1 Hz, ³*J* = 2.1 Hz, 1H, uA-C^{γ}**H^{A}**H^{B}),2.41 (ddd, ²*J* = 14.1 Hz, ³*J* = 11.2 Hz, ³*J* = 11.2 Hz, 1H, uA-C^{γ}**H^{A}**H^{B}), 1.77 (qdd, ³*J* = 7.1 Hz, ³*J* = 7.0 Hz, ³*J* = 5.4 Hz, 1H, uA-C^{ε}**H**), 1.19 (s, 3H, uC-C(C**H**₃)^{A}(CH₃)^{B}), 1.14 (d, ³*J* = 6.9 Hz, 3H, uA-C^{ε}C**H₃**), 1.12 (s, 3H, uC-C(CH₃)^{A}(C**H₃**)^{B}).
**¹³C NMR** (126 MHz, Chloroform-*d*) δ /ppm = 178.1 (uC-**C**(=O)), 170.5 (uD-**C**(=O)), 170.4 (uB-**C**(=O)), 164.8 (uA-**C**(=O)), 154.3 (uB-**C**^{ar,4}), 142.0 (uA-**C**^{β}H), 137.0 (uA-**C**^{ar,1}), 133.8 (uD-H₂C=**C**HCH₂) 131.0 (uB-**C**^{ar,2}), 129.5 (uB-**C**^{ar,1}), 128.8 (uA-**C**^{ar}), 128.6 (uA-**C**^{ar}), 128.3 (uB-**C**^{ar,6}), 125.7 (uA-**C**^{ar}), 124.8 (uA-**C**^{α}), 122.7 (uB-**C**^{ar,3}-Cl), 118.0 (uD-H₂**C**=CHCH₂) 112.6 (uB-**C**^{ar,5}), 75.8 (uA-**C**^{δ}), 72.1 (uD-H₂C=CH**C**H₂) 68.7 (uD-**C**^{β}), 63.4 (uA-**C**^{ζ}), 59.2 (uA-**C**^{η}), 56.3 (uB-O**C**H₃), 54.7 (uB-**C**^{α}), 52.5 (uD-**C**^{α}), 47.2 (uC-**C**^{β}H₂), 43.0 (uC-**C**^{α}(CH₃)₂), 40.9 (uA-**C**^{ε}CH₃), 37.3 (uA-**C**^{γ}), 35.7(uB-**C**^{β}), 24.7 (uC-C^{α}(**C**H₃)^{A}(CH₃)^{B}), 22.8 (UC-C^{α}(CH₃)^{A}(**C**H₃)^{B}), 13.8 (UA-C^{ε}**C**H₃).

### Cryptophycin [uD-Ser] H8

Under inert conditions epoxide **H7** (17.5 mg, 0.026 mmol, 1.0 eq.) and Pd(PPh₃)₄ (5.5 mg, 0.2 eq.) were dissolved in dry degassed dichloromethane. Phenyl silane (16 µL, 0.13 mmol, 5.0 eq.) was added to the yellow solution and stirred at RT for 19 h. Column chromatography (1 x 25cm, EtOAc/MeOH, 95:5) yielded compound **H8** (13.2 mg, 0.021 mmol, 79%) as a colorless foam.

**TLC:** R*_{f}* (EtOAc/MeOH, 95:5) = 0.18.

| | | |
|---|---|---|
| **HPLC-MS** (ESI+): | *m*/*z* (found) | 642.2735, *t*_{R} = 8.6 min |
| | *m*/*z* (calc.) | 642.2577 (M+H)⁺ = (C₃₃H₄₁ClN₃O₈)⁺. |
| **HRMS:** (ESI, +) | *m*/*z* (found) | 664.2386 |
| | *m*/*z* (calc.) | 664.2596 (M+Na)⁺ = (C₃₃H₄₀ClN₃O₈Na)⁺ |

**¹H NMR** (500 MHz, Chloroform-*d*) δ /ppm = 7.34 (m, 3H, uA-C^{ar}**H**), 7.23 (dd, ³*J* = 7.7, 1.8 Hz, 2H, uA-^{Car}**H**), 7.18 (d, ³*J* = 2.2 Hz, 1H, uB-C^{ar,2}**H**), 7.04 (dd, ³*J* = 8.4 Hz, ³*J* = 2.2 Hz, 1H, uB-C^{ar,6}**H**), 6.95 (dd, ³*J* = 8.2 Hz, ³*J* = 4.1 Hz, 1H, uC-N**H**), 6.84 (d, ³*J* = 8.4 Hz, 1H, uB-C^{ar,5}**H**), 6.74 (ddd, ³*J* = 15.2 Hz, ³*J* = 11.1 Hz, ³*J* = 4.2 Hz, 1H, uA-C^{β}**H**), 6.53 (d, ³*J* = 7.6 Hz, 1H, uD-N**H**), 5.81 (d, ³*J* = 7.5 Hz, 1H, uB-N**H**), 5.74 (dd, ³*J* = 15.0 Hz, ⁴*J* = 1.7 Hz, 1H, uA-C^{α}**H**), 5.18 (ddd, ³*J* = 11.5 Hz, ³*J* = 6.7 Hz, ³*J* = 2.2 Hz, 1H, uA-C^{δ}**H**), 4.67 (ddd, ³*J* = 7.8 Hz, ³*J* = 7.8 Hz, ³*J* = 4.6 Hz, 1H, uB-C^{β}**H**), 4.37 (ddd, ³*J* = 8.1 Hz, ³*J* = 4.3 Hz, ³*J* = 4.3 Hz, 1H, uD-C^{β}**H**), 3.87 (s, 3H, uB-OC**H**₃), 3.70 (d, ³*J* = 2.0 Hz, 1H, uA-C^{η}**H**), 3.60 (d, ³*J* = 4.3 Hz, 1H, uD-C^{β}**H₂**), 3.35 (dd, ²*J* = 13.2 Hz, ³*J* = 8.2 Hz, 1H, uC-C^{β}**H^{A}**H^{B}), 3.25 (dd, ²*J* = 13.3 Hz, ³*J* = 4.0 Hz, 1H, uC-C^{β}H^{A}**H^{B}**), 3.09 (dd, ²*J* = 14.5 Hz, ³*J* = 5.0 Hz, 1H, uB-C^{β}**H^{A}**H^{B}), 2.97 (dd, ²*J* = 14.7 Hz, ³*J* = 8.3 Hz, 1H, uB-C^{β}H^{A}**H^{B}**), 2.94 (dd, ³*J* = 7.3 Hz, ³*J* = 2.0 Hz, 1H, uA-C^{ζ}**H**), 2.60 (dddd, ²*J* = 14.1 Hz, ³*J* = 4.3 Hz, ³*J* = 2.2 Hz, ⁴*J* = 2.2 Hz, 1H, uA-C^{γ}**H^{A}**H^{B}), 2.37 (ddd, ³*J* = 14.1 Hz, ³*J* = 11.3 Hz, ³*J* = 11.3 Hz, 1H, uA-C^{γ}H^{A}**H^{B}**), 1.87 (dq, ³*J* = 6.9 Hz, ³*J* = 6.9 Hz, 1H, uA-C^{ε}**H**), 1.20 (s, 3H, uC-C(C**H**₃)^{A}(CH₃)^{B}), 1.15 (d, ³*J* = 5.5 Hz, 1H, uA-C^{ε}C**H**₃), 1.14 (s, 3H, uC-C(C**H**₃)^{A}(C**H**₃)^{B}).

**¹³C NMR** (126 MHz, Chloroform-d) δ/ppm = 178.4 (uC-**C**(=O)), 170.8 (uD-**C**(=O)), 170.7 (uB-**C**(=O)), 164.9 (uA-**C**(=O)), 154.3 (uB-**C**^{ar,4}), 141.9 (uA-**C**^{β}H), 136.8 (uA-**C**^{ar,1}), 131.0 (uB-**C**^{ar,2}), 129.5 (uB-**C**^{ar,1}), 128.9 (uA-**C**^{ar}), 128.8 (uA-**C**^{ar}), 128.3 (uB-**C**^{ar,6}), 125.7 (uA-**C**^{ar}), 125.2 (uA-**C**^{α}), 122.7 (uB-**C**^{ar,3}-Cl), 112.6 (uB-**C**^{ar,5}), 75.7 (uA-**C**^{δ}), 63.8 (uA-**C**^{ζ}, 62.7 (uD-**C**^{β}), 58.9 (uA-**C**^{η}), 56.3 (uB-O**C**H₃), 54.8 (uB-**C**^{α}), 54.5 (uD-**C**^{α}), 47.3 (uC-**C**^{β}H₂), 43.1 (uC-**C**^{α}(CH₃)₂), 40.3 (uA-**C**^{ε}CH₃), 37.1 (uA-**C**^{γ}), 35.7(uB-**C**^{β}), 24.8 (uC-C^{α}(**C**H₃)^{A}(CH₃)^{B}), 22.6 (uC-C^{α}(CH₃)^{A}(**C**H₃)^{B}), 13.8 (uA-C^{ε}**C**H₃).

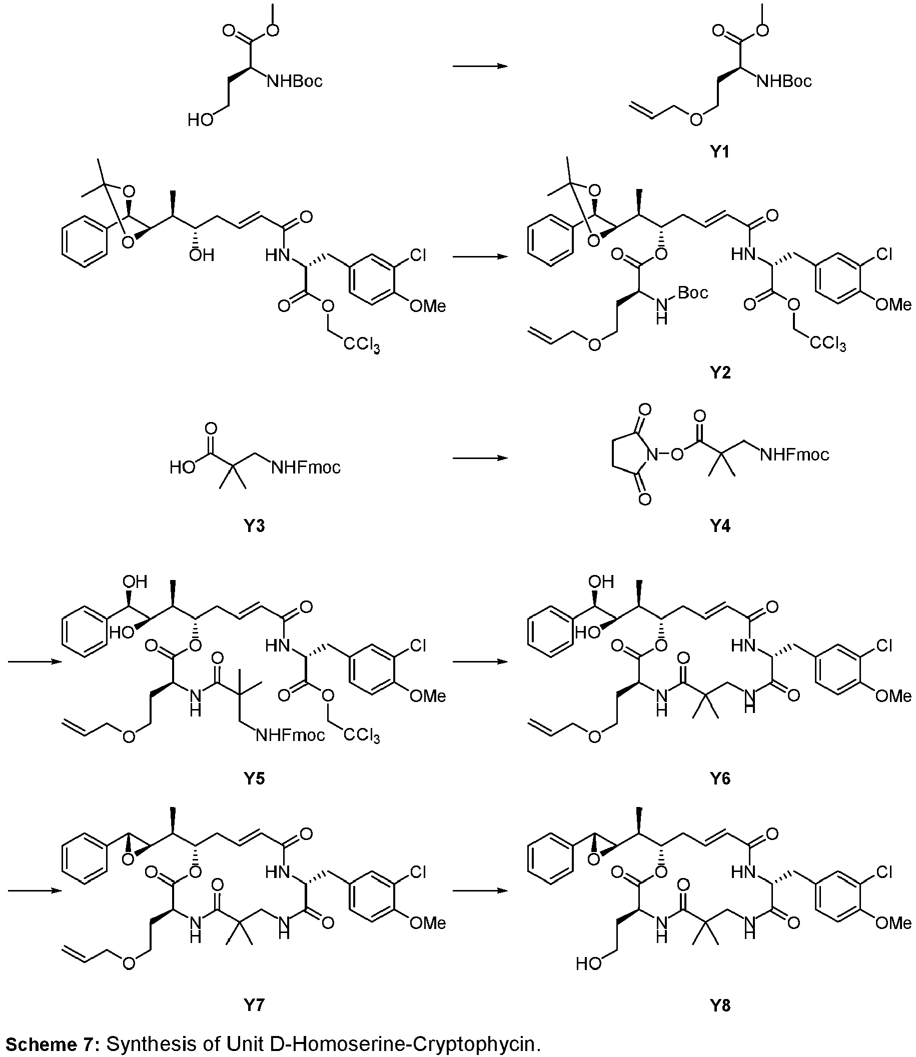

### Boc-L-HSe(All)-OH Y1

Boc-L-HSe-OMe was synthesized using literature known procedure (W.-J. Wu, Y. Wu, B. Liu, Tetrahedron 2017, 73, 1265-1274.).

Boc-L-HSe-OMe (204 mg, 0.870 mmol, 1 eq) was dissolved abs. DCM (2.6 mL) and benzyltrimethylammonium chloride (265 mg, 1.16 mmol, 1.3 eq), allyl bromide (0.15 mL, 1.72 mmol, 2 eq) and 40% NaOH solution (0.25 mL, 2.50 mmol, 2.9 eq) was added. The mixture was stirred for 16 h at rt and quenched with water (8 mL). The phases were separated, and the aqueous layer was acidified with citric acid (10 %, 6 mL) to pH = 2 and extracted with EtOAc (3 x 5 mL). The combined organic layers were washed with brine (2 x 5 mL) and dried over MgSO₄. The solvent was removed under reduced pressure and the crude was purified via column-chromatography (DCM/MeOH, 9:1) to yield Boc-L-HSe(All)-OH **Y1** (171 mg, 0.659 mmol, 75%) as colorless oil.
**R***_{f}* (DCM/MeOH 9:1) = 0.22,
**R***_{f}* (DCM/MeOH 8:2) = 0.40

**¹H NMR:** (500 MHz, Chloroform-d): δ / ppm = 5.88 (ddt, ³*J* = 16.4 Hz, ³*J* = 10.7 Hz, ³*J* = 5.5 Hz, 1H, H₂C=C**H**CH₂O), 5.67-5.58 (br s, 1H, N**H**), 5.25 (dd, ³*J* = 17.1 Hz, ²*J* = 1.2 Hz, 1H, **H**₂*^{trans}*C=CHCH₂O), 5.17 (dd, ³*J* = 10.5 Hz, ²*J* = 1.0 Hz, 1H, **H**₂*^{cis}*C=CHCH₂O), 4.41 (m, 1H, C^{α}**H**), 4.01-3.92 (m, 2H, Allyl: H₂C=CHC**H**₂O), 3.60-3.53 (m, 2H, C^{γ}**H**), 2.15 (m, 1H, C^{β}**H^{A}**H^{B}), 2.02 (m, 1H, C^{β}H^{A}**H^{B}**), 1.43 (s, 9H, C(C**H**₃)₃).

**¹³C NMR:** (126 MHz, Chloroform-*d*): δ / ppm = 176.6 (C^{α}**C**OOH), 155.9 (N**C**(=O)O), 134.3 (Allyl: H₂C=**C**H), 117.4 (H₂**C**=CH), 80.2 (C(**C**H₃)₃), 72.1 (**C**^{γ}), 66.9 (H₂C=CH**C**H₂O), 52.1 (**C**^{α}), 31.6 (**C**^{β}), 28.4 (C(**C**H₃)₃).

| | | |
|---|---|---|
| **HPLC-MS** (ESI +): | *m*/*z* (found) | 160.10, *t*_{R} = 7.4 min. |
| | *m*/*z* (calc.) | 160.10 (M-Boc+H⁺); (C₇H₁₄NO₃)⁺ |

### Boc-uD[HSe(All)]-uA[Acetonide]-uB-OTCE Y2

Building block A-B was synthesized according to SEWALD *et al.* (N. Sewald et al., J. Org. Chem. 2010, 75, 6953-6960).

A solution of Fmoc-HSe(All)-OH (**Y1**, 305 mg, 1.21 mmol, 1.0 eq.), building block A-B (799 mg, 1.21 mmol, 1.0 eq.) in abs THF (12 mL) was cooled to 0 °C and triethylamine (0.34 mL, 2.42 mmol, 2 eq), DMAP (0.037 g, 0.300 mmol, 0.25 eq) and 2,4,6-trichlorbenzoyl chloride (0.38 mL, 2.42 mmol, 2 eq) were added. The Solution was stirred for 3 h while warmed slowly to rt. A solution of citric acid (10 %, 30 mL) in water was added and the phases were separated. The aqueous layer was extracted with EtOAc (3 x 30 mL). The organic layers were washed with NaHCO₃ (30 mL) and brine (30 mL) and dried over MgSO₄, then concentrated in vacuo. Column chromatography (d = 4 cm, I = 20 cm, PE/EtOAc 2:1) yielded **Y2** a colorless oil (0.730 g, 0.810 mmol, 66 %).

**TLC:** R*_{f}* (PE/EtOAc 2:1) = 0.43.

**¹H-NMR:** (500 MHz, Chloroform-d): δ/ ppm = 7.41-7.27 (m, 5H, uA-C^{ar}**H**), 7.18 (m, 1H, uB-C^{ar,2}**H**), 7.04 (m, 1H, uB-C^{ar,5}**H**), 6.85 (m, 1H, uB-C^{ar,6}**H**), 6.60 (d, ³*J* = 7.6 Hz, 1H, uB-N**H**), 6.54 (m, 1H, uA-C^{β}**H**), 5.84 (m, 1H, uD-H₂C=C**H**CH₂O), 5.65 (d, ³*J* = 8.5 Hz, 1H, uD-N**H**), 5.55 (d, ³*J* = 15.5 Hz, 1H, uA- C^{α}**H**), 5.25 (dd, ³*J* = 17.3 Hz, ²*J* = 1.3 Hz, 1H, uD-**H**₂*^{trans}*C=CHCH₂O), 5.17 (d, ³*J* = 10.6Hz, 1H, uD-**H**₂^{cis}C=CHCH₂O), 4.98-4.85 (m, 2H, uA-C^{δ}**H**, uB-C^{α}**H**), 4.79 (d, ²*J* = 11.9 Hz, 1H, uB-C**H**₂CCl₃), 4.79-4.61 (m, 2H, uA-C^{η}**H**, uB-C**H**₂CCl₃), 4.23 (m, 1H, uD-C^{α}**H**), 3.94-3.90 (m, 2H, uD-CH₂CHC**H**₂O), 3.89-3.84 (m, 4H, uA-C^{ξ}**H**, uB-OC**H**₃), 3.82 (m, 1H, uA-C^{δ}**H**), 3.51-3.38 (m, 2H, uD-C^{γ}**H**₂), 3.18 (dd, ³*J* = 14.2 Hz, ³*J* = 5.6 Hz, 1H, uB-C^{β}**H₂**), 3.05 (m, 1H, uB-C^{β}**H**₂), 2.31 (m, 1H, uA-C^{γ}**H**), 2.27 (m, 1H, uA-C^{γ}**H**), 2.08-1.93 (m, 2H, uD-C^{β}**H**₂), 1.87 (m, 1H, uA-C^{ε}**H**), 1.51 (s, 3H, uA-C(C**H**₃)^{A}(C**H**₃)^{B}), 1.46 (s, 3H, uA-C(C**H**₃)^{A}(C**H**₃)₂), 1.40 (s, 9H, uD-C(C**H**₃)₃), 1.08 (d, ³*J* = 7.0 Hz, 3H, uA-C^{ε}C**H**₃).

**¹³C NMR:** (126 MHz, CDCl₃): δ /ppm = 171.8 (uD-C^{α}**C**OO), 170.2 (uB-**C**OO), 165.5 (uA-**C**ON), 155.8 (uD-N**C**OO), 154.2 (uB-**C**^{ar,4}), 139.2 (uA-**C**^{β}), 137.6 (uA-**C**^{ar,1}), 134.5 (uD-H₂C=**C**H), 131.1 (uB-**C**^{ar,2}), 129.2 (uB-**C**^{ar,1}), 128.9 (uA- **C**^{ar, meta}), 128.6 (uB-**C**^{ar,6}), 127.4 (uA-**C**^{α}), 127.1 (uA- **C**^{ar, ortho}), 122.4 (uB-**C**^{ar,3}), 117.3 (uD-H₂**C**=CH), 112.3 (uB-**C**^{ar,5}), 109.1 (uA-**C**(CH₃)₂), 94.5 (uD-**C**Cl₃), 82.6 (uA-**C**^{ξ}), 80.8 (uA-**C**^{η}), 80.0 (uD-**C**(CH₃)₃), 75.4 (uA-**C**^{δ}), 74.8 (uB-**C**H₂CCl₃), 72.1 (uD-**C**^{γ}), 66.9 (uD-CH**C**H₂O), 56.3 (uB-O**C**H₃), 53.5 (uD-**C**^{α}), 52.7 (uB-**C**^{α}), 36.5 (uA-**C**^{γ}), 35.8 (uA-**C**^{ε}), 32.0 (uB-**C**^{β}), 31.2 (uD-**C**^{β}), 28.5 (uD-C(**C**H₃)₃), 27.34 (uA-C(**C**H₃)₂), 27.2 (uA-C(**C**H₃)₂), 9.7 (uA-C^{ε}**C**H₃).

| | | |
|---|---|---|
| **HPLC-MS** (ESI +): | *m*/*z* (found) | 803.20, t_{R} = 12.8 min. |
| | *m*/*z* (calc.) | 803.20 (M-Boc+H)⁺ = (C₃₇H₄₇Cl₄N₂O₉)⁺ |

### Fmoc-uC-OSu Y4

Fmoc protected Unit C **Y3** was synthesized according to B. OSSWALD (B. Osswald, Universität Bielefeld, Dissertation, 2015).

DCC (0.608 g, 2.94 mmol, 1 eq) in THF was added at 0 °C to a solution of Fmoc-Unit C **Y3** (1.00 g, 2.96 mmol, 1 eq) and N-hydroxysuccinimide (0.34 g, 2.95 mmol, 1 eq) in THF. The reaction was stirred for 18 h at rt, filtered through a pad of silica and washed with THF. The solvent was removed in vacuo and the residue was purified via column chromatography (PE/EtOAc, 1:1) to yield **Y4** (1.07 g, 2.45 mmol, 83%) as a colorless solid.

**TLC:** R*_{f}*(PE/EtOAc, 1:1) = 0.30

| | | |
|---|---|---|
| **TLC-MS** (ESI+): | *m*/*z* (found) | 459.1, |
| | *m*/*z* (calc.) | 459.1 (M+Na⁺); (C₂₄H₂₄N₂NaO₆)⁺ |

**¹H NMR** (500 MHz, Chloroform-d) δ / ppm = 7.76 (d, ³*J* = 7.5 Hz, 2H, Fmoc^{ar}-**H**), 7.63 (d, ³*J* = 7.5 Hz, 2H, Fmoc^{ar}-**H**), 7.39 (t, ³*J* = 7.4 Hz, 2H, Fmoc^{ar}-**H**), 7.31 (t, ³*J* = 7.7 Hz, 2H, Fmoc^{ar}-**H**), 5.97 (t, ³*J* = 6.8 Hz, 1H, N**H**), 4.35 (d, ³*J* = 7.6 Hz, 2H, C**H**₂NH), 4.25 (t, ³*J* = 7.5 Hz, 1H, Fmoc-CH₂C**H**), 3.51 (d, ³*J* = 6.8 Hz, 2H, Fmoc-C**H**₂CH), 2.89 (d, ³*J* = 4.8 Hz, 4H, OSu-C**H**₂C**H**₂), 1.39 (s, 6H, C(C**H**₃)₂).

**¹³C NMR** (126 MHz, Chloroform-d) δ / ppm = 171.9 (**C**(=O)O), 169.5 (OSu, **C**(=O), 157.0 (NH**C**(=O)O), 144.1 (Fmoc^{ar}-**C**), 141.4 (Fmoc^{ar}-**C**), 127.8 (Fmoc^{ar}-**C**), 127.2 (Fmoc^{ar}-**C**), 125.4 (Fmoc^{ar}-**C**), 120.1 (Fmoc^{ar}-**C**), 67.2 (Fmoc, **C**H₂CH), 49.6 (**C**H₂NH), 47.3 (Fmoc, CH₂**C**H), 44.5 (**C**(CH₃)₂), 25.8 (OSu, **C**H₂**C**H₂), 22.6 (C(**C**H₃)₂).

### Fmoc-uC-uD[HSe(All)]-uA[diol]-uB-OTCE Y5

Unit DAB **Y2** (0.603 g, 0.667 mmol, 1 eq.) was dissolved in DCM (7.34 mL) and water (1.55 mL) was added. The solution was cooled to 0 °C and TFA (7.34 mL) was added. The reaction was stirred for 5 min at 0 °C and 10 min at rt. The solvent was removed under vacuo and the residue was redissolved in DCM (7.34 mL) and water (1.55 mL) was added. The solution was cooled to 0 °C and TFA (7.34 mL) was added. The reaction was stirred for 5 min at 0 °C and 10 min at rt. The solvent was removed, and the residue was taken up in EtOAc (50 mL) and washed with NaHCO₃ solution (sat., 50 mL). The aqueous layer was extracted with EtOAc (3 x 50 mL). The combined organic layers were washed with water (25 mL) and brine (25 mL) and dried over MgSO₄. Removing the solvent in vacuo yielded a yellow oil which was dissolved in DCM (3 mL) Fmoc-protected Unit C-OSu **Y4** (0.378 g, 0.867 mmol, 1.3 eq) and D*i*PEA (0.31 mL, 1.77 mmol, 2.7 eq) was added and the reaction mixture was stirred for 4 h at rt. It was diluted with DCM (20 mL) and ctric acid solution (10%, 20 mL) was added. The phases were separated, and the aqueous layer was extracted with DCM (5 x 15 mL). The combined organic layers were dried over MgSO₄ and the solvent was removed under vacuo. Flash-chromatography (PE/EtOAc, 2:1 to pure EtOAc) yielded seco-cryptophycin **Y5** (0.284 g, 0.262 mmol, 39%) as colorless foam.

**TLC:** R*_{f}* (PE/EtOAc 2:1) = 0.11.

**¹H NMR:** (500 MHz, Chloroform-d): δ /ppm = 7.76 (d, ³*J* = 7.5 Hz, 2H, Fmoc-ar-**H**), 7.63 (d, ³*J* = 7.5 Hz, 2H, Fmoc-ar-**H**), 7.42-7.26 (m, 9H, Fmoc-ar-**H**, uA-C^{ar}-**H**), 7.16 (m, 1H, uB-C^{ar,2}**H**), 7.04 (dd, ³*J* = 8.5 Hz, ⁴*J* = 2.0 Hz, 1H, uB-C^{ar,6}**H**), 6.80 (m, 1H, uB-C^{ar,5}**H**), 6.59 (m, 1H, uA-C^{β}**H**), 5.93-5.58 (m, 2H, uD-CH₂=C**H**, uA-C^{α}**H**), 5.30-5.14 (m, 2H, uD-**H₂**C=CH), 5.07-4.91 (m, 2H, uA-C^{δ}**H**, uB-**C**^{α}H), 4.78 (m, 1H, uB-C**H**₂CCl₃), 4.67 (m, 1H, uB-C**H**₂CCl3), 4.55 (m, 1H, uA-C^{η}**H**), 4.46 (ddd, ³*J* = 5.5 Hz, ³*J* = 5.5 Hz, ³*J* = 5.5 Hz, 1H, uD-C^{α}H), 4.43-4.26 (m, 2H, Fmoc-C**H**₂CH), 4.23 (t, ³*J* = 7.4 Hz, 1H, Fmoc-CH₂C**H**), 4.04-3.88 (m, 2H, uD-CHC**H₂**O), 3.83 (s, 3H, uB-OC**H**₃), 3.65-3.48 (m, 3H, uD-C^{γ}**H₂**, uA-C^{ζ}**H**), 3.39-3.26 (m, 2H, uC-C^{β}**H₂**), 3.21 (d, ³*J* = 3.7 Hz, uA-C^{ζ}O**H**), 3.18 (dd, ²*J* = 14.1 Hz, ³*J* = 5.7 Hz, 1H, uB-C^{β}**H^{A}**H^{B}), 3.07 (s, 1H, uA-C^{η}O**H**), 2.99 (dd, ²*J* = 14.0 Hz, ³*J* = 7.8 Hz, 1H, uB-C^{β}H^{A}**H^{B}**), 2.51-2.40 (m, 2H, uD-C^{β}**H₂**), 2.32 (m, 1H, uA-C^{γ}**H^{A}**H^{B}), 2.20 (m, 1H, uA-C^{γ}H^{A}**H^{B}**), 1.47 (m, 1H, uA: uA-C^{ε}**H**CH₃), 1.22(s, 3H, uC-C(C**H**₃)^{A}(CH₃)^{B})), 1.12 (s, 3H, uC-C(CH₃)^{A}(C**H**₃)**^{B}**), 1.04-0.90 (m, 3H, uA: uA-C^{ε}HC**H**₃).

**¹³C NMR:** (126 MHz, Chloroform-*d*): δ /ppm = 177.7 (uC-**C**(=O)), 170.3 (uB-**C**(=O)), 166.0 (uA-**C**(=O)), 157.2 (Fmoc-N**C**(=O)O), 154.2 (uB-**C**^{ar,4}), 144.1 (Fmoc-**C**^{ar}), 141.5 (Fmoc-Ar-**C**^{ar}), 138.6 (uA-**C**^{ar,1}), 133.9 (uA-**C**^{β}), 131.5 (uB-C^{ar,2}), 129.4 (uB-C^{ar,1}), 128.9 (uA-**C**^{ar,3,5}), 128.6 (uB-C^{ar,6}), 127.8 (Fmoc-**C**^{ar}), 127.2 (uA-C^{ar,2,6}), 127.0 (uA-**C**^{α}), 125.4 (Fmoc-**C**^{ar}), 125.3 (Fmoc-**C**^{ar}), 122.1 **(uB-C^{ar,3}),** 120.1 (Fmoc-**C**^{ar}), 120.0 **(uB-C^{ar,3}),** 118.6 (uD-H₂**C**=CH), 112.2 (uB-**C**^{ar,5}), 94.6 (uB-**C**Cl3), 76.3 (uA-**C**^{δ}), 75.7 (uA-**C**^{η}), 75.3 (uA-**C**^{ζ}), 74.6 (uB-**C**H₂CCl₃), 72.7 (uD-H₂C=CH**C**H₂), 67.7 (uD-**C**^{γ}), 67.1 (Fmoc-**C**H₂CH), 56.2 (uB-O**C**H₃), 53.4 (uD-**C**^{α}), 53.2 (uB: **C**^{α}), 50.1 (uC-**C**^{β}), 47.4 (Fmoc-CH₂**C**H), 43.5 (uC-C(**C**H₃)^{A}(CH₃)^{B}), 37.9 (uA-**C**^{ε}), 36.6 (uA-**C**^{γ}), 33.5 (uB-**C**^{β}), 29.8 (uD-**C**^{β}), 23.3 (uC-C(**C**H₃)^{A}(CH₃)^{B}), 22.4 (uC-C(**C**H₃)^{A}(CH₃)^{B}), 10.1 (uA-C^{ε}**C**H₃).

| | | |
|---|---|---|
| HPLC-MS (ESI+): | *m*/*z* (found) | 1084.31, t_{R} = 12.1 min. |
| | *m*/*z* (calc.) | 1084.31 (M+H)⁺ = (C₅₄H₆₂Cl₄N₃O₁₂)⁺ |

### Cryptophycin-[uA-diol]-[uD-HSe(All)] Y6

Seco-cryptophycin **Y5** (0.249 g, 0.232 mmol, 1 eq) was dissolved in DMF (7 mL) and piperidine (115 µL, 1.16 mmol, 5 eq) were added slowly at 0 °C. The reaction was stirred at rt for 25 h and the solvent was removed under vacuo. Column chromatography (DCM/MeOH, 20:1) yielded cryptophycin-diol **Y6** (71.8 mg, 0.10 mmol, 43%).

**TLC:** R*_{f}* (DCM/MeOH, 20:1) = 0.13.

| | | |
|---|---|---|
| **HPLC-MS** (ESI +): | *m*/*z* (found) | 714.32, t_{R} = 8.2 min. |
| | *m*/*z* (calc.) | 714.32 (M+H) ⁺ = (C₃₇H₄₉ClN₃O₉)⁺. |

**¹H NMR** (500 MHz, Chloroform-d) δ/ppm = 7.35 - 7.27 (m, 5H, uA-C^{ar}**H**), 7.22 (dd, ³*J* = 7.7, ³*J* = 4.5 Hz, 1H, uC-NH), 7.16 (d, ⁴*J* = 1.7 Hz, 1H, uB-C^{ar,2}**H**), 7.03 (d (broad), ³*J* = 7.7 Hz, 2H, uB-c^{ar,6}**H**, uD-N**H**), 6.82 (d, ³*J* = 8.3 Hz, 1H, uB-C^{ar,5}**H**), 6.69 (ddd, ³*J* = 15.1 Hz, ³*J* = 10.7 Hz, ³*J* = 4.4 Hz, 1H, uA-C^{β}**H**), 6.01 (d, ³*J* = 7.8 Hz, 1H, uB-N**H**), 5.86 (ddt, ³*J* = 16.4 Hz, ³*J* = 10.9 Hz, ³*J* = 6.0 Hz, 1H, uD-H₂C=C**H**), 5.69 (d, ³*J* = 15.1 Hz, 1H, uA-C^{α}**H**), 5.25 (d, ³*J* = 17.5 Hz, 1H, uD-**H**₂*^{trans}*BC=CH), 5.19 (d, ³*J* = 10.3 Hz, 1H, uD-**H₂***^{cis}*C=CH), 5.08 - 5.02 (m, 1H, uA-C^{γ}**H**), 4.69 (ddd, ³*J* = 7.9 Hz, ³*J* = 7.9 Hz, ³*J* = 4.8 Hz, 1H, uB-C^{α}**H**), 4.54 (d, ³*J* = 8.5 Hz, 1H, uA-C^{η}**H**), 4.37 (ddd, ³*J* = 6.2 Hz, ³*J* = 6.2 Hz, ³*J* = 6.2 Hz, 1H, uD-C^{α}**H**), 3.92 (d, ³*J* = 5.8 Hz, 2H, uD-H₂C=CHC**H₂**), 3.85 (s, 3H, uB-OC**H₃**), 3.82 (d, ³*J* = 8.9 Hz, 1H, uA-C^{ζ}**H**), 3.53 - 3.44 (m, 2H, uD-C^{γ}**H₂**), 3.42 (s (broad), 1H, C^{η}O**H**), 3.35 (s (broad), 1H, uA-C^{ζ}O**H**), 3.27 (dd, ²*J* = 13.2 Hz, ³*J* = 7.6 Hz, 1H, uC-C^{β}**H**^{A}H^{B}), 3.21 (dd, ²*J* = 13.3 Hz, ³*J* = 4.2 Hz, 1H, uC-C^{β}H^{A}**H**^{B}), 3.07 (dd, ²*J* = 14.5 Hz, ³*J* =4.8 Hz, 1H, uB-C^{β}**H**^{A}H^{B}), 2.91 (dd, ²*J* = 14.5 Hz, ³*J* = 8.2 Hz, 1H, uB-C^{β}H^{A}**H^{B}**), 2.39 (ddd, ²*J* = 13.8 Hz, ³*J* = 3.4 Hz, ³*J* = 3.4 Hz, 1H, uA-C^{γ}**H**^{A}H^{B}), 2.19 - 2.11 (m, 1H, uA-C^{γ}H^{A}**H^{B}**), 2.03 (dddd, ²*J* = 13.2, ³*J* = 8.5 Hz, ³*J* = 4.3 Hz, ³*J* = 4.3 Hz 1H, uD-C^{β}**H**^{A}H^{B}), 1.88 (dddd, ²*J* =15.9 Hz, ³*J* = 5.4 Hz, ³*J* = 5.4 Hz, 1H, uD-C^{β}H^{A}**H**^{B}), 1.50 - 1.38 (m, 1H, uA-C^{ε}**H**), 1.14 (s, 3H, uC-C(C**H**₃)^{A}(CH₃)^{B}), 1.08 (s, 3H, uC-C(CH₃)^{A}(C**H**₃)^{B}), 0.97 (d, ³*J* = 6.8 Hz, 3H, uA-C^{ε}C**H**₃).

**¹³C NMR** (126 MHz, Chloroform-d) δ /ppm = 178.0 (uC-**C**(=O)), 172.0 (uD-**C**(=O)), 170.8 (uB-**C**(=O)), 165.4 (uA-**C**(=O)), 154.1 (uB-**C**^{ar,4}), 142.6 (uA-**C**^{β}H), 140.6 (uA-**C**^{ar,1}), 134.3 (uD-H₂C=**C**H), 131.0 (uB-**C**^{ar,2}), 129.8 (uB-**C**^{ar,1}), 128.8 (uA-**C**^{ar}), 128.5 (uA-**C**^{ar}), 128.4 (uB-**C**^{ar,6}), 127.1 (uA-**C**^{ar}), 124.6 (uA-**C**^{α}), 122.5 (uB-**C**^{ar,2-}Cl), 118.0 (uD-H₂**C**=CH), 112.5 (uB-**C**^{ar,5}), 76.1 (uA-**C**^{δ}), 75.8 (uA-**C**^{η}), 74.7 (uA-**C^{ζ}**, 72.4 (uD-H₂C=CH-**C**H₂), 66.8 (uD-**C**^{γ}), 56.3 (uB-O**C**H₃), 54.7 (uB-**C**^{α}), 51.6 (uD-**C**^{α}), 47.3 (uC-**C**^{β}H₂), 42.6 (uC-**C**^{α}(CH₃)₂), 38.1 (uA-**C**^{ε}CH3), 36.6 (uA-**C**^{γ}), 35.7 (uB-**C**^{β}), 31.0 (uD-**C**^{β}), 24.5 (uC-C^{α}(**C**H₃)^{A}(CH₃)^{B}), 22.9 (uC-C^{α}(CH₃)^{A}(**C**H₃)^{B}), 10.0 (uA-C^{ε}**C**H₃).

### Cryptophycin-[uD-HSe(All)] Y7

Cryptophycin-Diol **Y6** (71.8 mg, 0.1 mmol, 1 eq.) and PPTS (63 mg, 0.25 mmol, 2.5 eq.) were dried in HV for 10 min and dissolved in abs. DCM (3 mL) under argon. Trimethyl orthoformate (1 mL, excess) was added and the reaction was stirred at rt for 2.5 h, before filtered through a pad of silica and eluted with EtOAc/DCM (1:1, 300 mL). The solvent was removed under reduced pressure and dried in HV overnight. The intermediate orthoester was dissolved in abs. DCM (1.5 mL) and a freshly prepared AcBr-solution (0.5 M in abs. DCM, 0.5 mL, 0.25 mmol, 2.5 eq.) was added. The reaction was stirred for 5 h at rt and poured into NaHCO₃-solution (half sat., 50 mL) and extracted with DCM (4 × 20 mL). The combined organic layers were dried over MgSO₄ and the dissolved was removed under reduced pressure and dried in HV overnight.

An emulsion of abs. ethylene glycol (2.5 mL), abs. 1,2-dimethoxyethane (5.0 mL) and potassium carbonate (209 mg, 1.51 mmol) was freshly prepared over 3 Å molecular sieves (350 mg) and homogenized by vortexer and ultrasonic bath.

The potassium carbonate emulsion (2.5 mL, 0.50 mmol K₂CO₃, 5 eq.) homogenized by constant shaking was added to the bromo-formate intermediate. The mixture was stirred for 5.5 min at rt then diluted with abs. dichloromethane (20 mL). The solution was given to KHSO₄ solution (0.5 %, 20 mL), phases were separated immediately, and the aqueous phase was further extracted with dichloromethane (3 × 20 mL). The combined organic phases were dried over MgSO₄ and concentrated in vacuo. Column chromatography (pure EtOAc) yielded cryptophycin **Y7** as colorless solid (46.6 mg, 66.9 µmol, 67 %).
**TLC:** R*_{f}* (pure EtOAc) = 0.20

| | | |
|---|---|---|
| **HPLC-MS** (ESI+): | *m*/*z* (found) | 696.31, t_{R} = 10.0 min. |
| | *m*/*z* (calc.) | 696.30 (M+H)⁺ = (C₃₇H₄₇ClN₃O₈)⁺ |

**¹H NMR** (500 MHz, Chloroform-d) δ /ppm = 7.51 (dd, ³*J* = 7.9 Hz, ³*J* = 3.9 Hz, 1H, uC-NH), 7.38 - 7.30 (m, 3H, uA-C^{ar}**H**), 7.22 (d, ²*J* = 1.8 Hz, 1H, uB-C^{ar,2}**H**), 7.21 - 7.17 (m, 3H, uA-C^{ar}**H**), 7.03 (dd, ³*J* = 8.4 Hz, ⁴*J* = 2.2 Hz, 1H, uB-C^{ar,6}**H**), 6.83 (d, ³*J* = 8.4 Hz, 1H, uB-C^{ar,5}**H**), 6.76 (ddd, ³*J* = 15.1 Hz, ³*J* = 10.5 Hz, ³*J* = 4.6 Hz, 1H, uA-C^{β}**H**), 5.84 (ddt, ³*J* = 17.3 Hz, ³*J* = 10.3 Hz, ³*J* = 5.8 Hz, 1H, uD-H₂C=C**H**), 5.75 (dd, ³*J* = 15.1 Hz, ³*J* = 1.7 Hz, 1H, uA-C^{α}**H**), 5.65 (d, ³*J* = 8.0 Hz, 1H, uB-N**H**), 5.24 (ddt, ³*J* = 17.3 Hz, ²*J* = 1.6 Hz, 1H, uD-**H₂***^{trans}*C=CHCH₂), 5.19 (dd, ³*J* = 10.3 Hz, ²*J* = 1.4 Hz, 1H, uD-H₂*^{cis}*C=CHCH₂), 5.15 (ddd, ³*J* = 11.3 Hz, ³*J* = 5.9 Hz, ³*J* = 1.9 Hz, 1H, uA-C^{δ}**H**), 4.75 (ddd, ³*J* = 8.1, Hz, ³*J* = 6.2 Hz, ³*J* = 6.2 Hz, 1H, uB-C^{α}**H**), 4.26 (ddd, ³*J* = 6.7 Hz, ³*J* = 4.2 Hz, 1H uD-C^{α}**H**), 3.88 (dd, ³*J* = 5.8 Hz, ³*J* = 1.5 Hz, 2H, uD-H₂C=CHC**H**₂), 3.87 (s, 3H, uD-OC**H**₃), 3.66 (d, ³*J* = 1.9 Hz, 1H, uA-C^{η}**H**), 3.35 - 3.32 (m, 1H, uD-C^{γ}**H**^{A}H^{B}), 3.30 (m, 1H, uC-C^{β}**H**^{A}H^{B}), 3.24 (ddd, ²*J* = 9.5 Hz, ³*J* = 5.5 Hz, ³*J* = 3.4 Hz, 1H, uD-C^{γ}**H**^{A}H^{B}), 3.14 (dd, ²*J* = 13.2 Hz, ³*J* = 4.0 Hz, 1H uC-C^{β}H^{A}**H^{B}**), 3.06 (d, ³*J* = 6.1 Hz, 2H, uB-C^{β}**H₂**), 2.86 (dd, ³*J* = 7.9 Hz, ³*J* = 2.0 Hz, 1H, uA-C^{ζ}**H**), 2.57 (ddd, ²*J* = 12.6 Hz, ³*J* = 4.8 Hz, ³*J* = 2.4 Hz, 1H, uA-C^{γ}**H^{A}**H^{B}), 2.42 (ddd, ²*J* = 14.5 Hz, ³*J* = 10.9 Hz, ³*J* = 10.9 Hz, 1H, uA-C^{γ}H^{A}**H**^{B}), 1.80 (m, 1H, uD-C^{β}**H**^{A}H^{B}), 1.75 (m, 1H, uA-C^{ε}**H**), 1.60 (m, 1H, uD-C^{β}H^{A}**H^{B}**), 1.14 (d, 3H, uA-C^{ε}**H**), 1.13 (s, 3H, uC-C(C**H₃**)^{A}(CH₃)^{B}), 1.06 (s, 3H, uC-C(CH₃)^{A}(C**H**₃)**^{B}**).

**¹³C NMR** (126 MHz, Chloroform-d) δ /ppm = 178.4 (uC-**C**(=O)), 171.7 (uD-**C**(=O)), 170.4 (uB-**C**(=O), 165.1 (uA-**C**(=O)), 154.2 (uB-**C**^{ar,4}), 141.5 (uA-**C**^{β}H), 136.9 (uA-**C**^{ar,1}), 134.0 (uD-H₂**C**=CH), 131.1 (uB-**C**^{ar,2}), 129.6 (uB-**C**^{ar,1}), 128.8 (uA-**C**^{ar}), 128.6 (uA-**C**^{ar}), 128.4 (uB-**C**^{ar,6}), 125.7 (uA-**C**^{ar}), 125.1 (uA-**C**^{α}), 122.7 (uB-**C**^{ar,3-}Cl), 118.0 (uD-H₂**C**=CH), 112.5 (uB-**C**^{ar,5}), 75.8 (uA-**C**^{δ}), 72.4 (uD-H₂C=CH-**C**H₂), 67.3 (uD-**C**^{γ}), 63.7 (uA-**C^{ζ}**, 59.5 (uA-**C**^{η}), 56.3 (uB-O**C**H₃), 54.4 (uB-**C**^{α}), 52.3 (uD-**C**^{α}), 47.2 (uC-**C**^{β}H₂), 42.0 (uC-**C**^{α}(CH₃)₂), 41.0 (uA-**C**^{ε}CH3), 37.3 (uA-C^{γ}), 35.6 (uB-C^{β}), 30.3 (uD-C^{β}), 24.2 (uC-C^{α}(**C**H₃)^{A}(CH₃)^{B}), 23.2 (uC-C^{α}(CH₃)^{A}(**C**H₃)^{B}), 14.1 (uA-C^{ε}**C**H₃).

### Cryptophycin-[uD-HSe] Y8

Allyl protected cryptophycin **Y7** (16.5 mg, 23.7 µmol, 1 eq) and Pd(PPh₃)₄ (5 mg, 4.3 µmol, 0.2 eq) was dissolved in degassed abs. DCM (0.5 mL) and phenyl silane (14.6 µL, 118.7 µmol, 5 eq) was added and the reaction was stirred for 24 h at rt. Purification via column chromatography (EtOAc/MeOH, 100:5) by directly injecting the reaction mixture on the column, yielded cryptophycin **Y8** (13.7 mg, 20.0 µmol, 84%). **TLC:** R*_{f}* (EtOAc/MeOH, 100:5) = 0.17

| | | |
|---|---|---|
| **HRMS:** (ESI, +) | *m*/*z* (found) | 678.25 |
| | *m*/*z* (calc.) | 678.25 (M+Na)⁺ = (C₃₄H₄₂ClN₃NaO₈)⁺ |

**¹H NMR** (600 MHz, Chloroform-d) δ = 7.38 - 7.28 (m, 3H, uA-C^{ar}**H**), 7.22 (dd, ³*J* = 6.5 Hz, ⁴*J* = 1.4 Hz, 2H, uA-C^{ar}**H**), 7.19 (d, ⁴*J* = 2.1 Hz, 1H, uB-C^{ar,2}**H**), 7.04 (dd, ³*J* = 8.4 Hz, ⁴*J* = 2.2 Hz, 1H, uB-C^{ar,6}**H**), 6.84 (d, ³*J* = 8.4 Hz, 1H, uB-C^{ar,5}**H**), 6.77 (d, ³*J* = 6.9 Hz, 1H, uD-N**H**), 6.74 (m, 1H, uA-C^{β}**H**), 6.67 (dd, ³*J* = 8.2 Hz, ³*J* = 4.1 Hz, 1H, uC-N**H**), 5.75 (s (broad), 1H, uB-NH), 5.71 (dd, ³*J* = 14.9 Hz, ³*J* = 1.7 Hz, 1H, uA-C^{α}**H**), 5.20 (ddd, ³*J* = 11.5 Hz, ³*J* = 6.7 Hz, ³*J* = 2.2 Hz, 1H, uA-C^{δ}**H**), 4.63 (ddd, ³*J* = 7.8 Hz, ³*J* = 5.1 Hz, 1H, uB-C^{α}**H**), 4.40 (ddd, ³*J* = 10.5 Hz, ³*J* = 7.2 Hz, ³*J* = 3.6 Hz, 1H, uD-C^{α}**H**), 3.87 (s, 3H, uB-OC**H**₃), 3.67 (d, ³*J* = 1.9 Hz, 1H, uA-C^{η}**H**), 3.49 (m, 1H, uD-C^{γ}**H**^{A}H^{B}), 3.40 (m, 1H, uD-C^{γ}**H**^{A}H^{B}), 3.37 (m, 1H, uC-C^{β}**H**^{A}H^{B}), 3.30 (dd, ²*J* = 13.2 Hz, ³*J* = 4.0 Hz, 1H, uC-C^{β}H^{A}**H^{B}**), 3.12 (dd, ²*J* = 14.6 Hz, ³*J* = 5.0 Hz, 1H, uB-C^{β}**H**^{A}H^{B}), 2.94 (dd, ²*J* = 14.5 Hz, ³*J* = 8.3 Hz, 1H, uB-C^{β}H^{A}**H^{B}**), 2.89 (dd, ³*J* = 7.8 Hz, ³*J* = 2.0 Hz, 1H, uA-C^{ζ}**H**), 2.61 (dddd, ³*J* = 14.0 Hz, ³*J* = 4.3 Hz, ³*J* = 2.1 Hz, ³*J* = 2.1 Hz, 1H, uA-C^{γ}**H**^{A}H^{B}), 2.37 (m, 1H, uA-C^{γ}H^{A}**H^{B}**), 1.82 - 1.74 (m, 2H, uA-C^{ε}**H**, uD-C^{β}**H^{A}**H^{B}), 1.33 (dddd, ²*J* = 14.1 Hz, ³*J* = 6.8 Hz, ³*J* = 6.8 Hz, ³*J* = 3.3 Hz, 1H, uD-C^{β}H^{A}**H^{B}**), 1.20 (s, 3H, uC-C(C**H₃**)^{A}(CH₃)^{B}), 1.16 (d, ³*J* = 6.9 Hz, 1H, uA-C^{ε}C**H**₃), 1.13 (s, 3H, uC-C(CH₃)^{A}(C**H**₃)^{B}).

**¹³C NMR** (151 MHz, Chloroform-d) δ /ppm = 178.7 (uC-**C**(=O)), 172.5 (uD-**C**(=O)), 170.7 (uB-**C**(=O), 164.9 (uA-**C**(=O)), 154.3 (uB-**C**^{ar,4}), 142.0 (uA-**C**^{β}H), 136.8 (uA-**C**^{ar,1}), 131.0 (uB-**C**^{ar,2}), 129.4 (uB-**C**^{ar,1}), 129.2 (uA-**C**^{ar}), 128.8 (uA-**C**^{ar}), 128.4 (uB-**C**^{ar,6}), 125.9 (uA-**C**^{ar}), 125.1 (uA-**C**^{α}), 122.7 (uB-**C**^{ar,3-}Cl), 112.6 (uB-**C**^{ar,5}), 75.7 (uA-**C**^{δ}), 63.7 (uA-**C^{ζ}**, 59.5 (uA-**C**^{η}), 58.4 (uD-**C**^{γ}), 56.3 (uB-O**C**H₃), 54.9 (uB-**C**^{α}), 50.6 (uD-**C**^{α}), 47.2 (uC-**C**^{β}H₂), 43.4 (uC-**C^{α}**(CH₃)₂), 40.8 (uA-**C**^{ε}CH3), 37.3 (uA-C^{γ}), 35.7 (uB-**C**^{β}), 34.6 (uD-C^{β}), 25.2 (uC-C^{α}(**C**H₃)^{A}(CH₃)^{B}), 22.1 (uC-C^{α}(CH₃)^{A}(**C**H₃)^{B}), 13.9 (uA-C^{ε}**C**H₃).

### Example 2: Biological tests

The KB-3-1 and KB-V1 cells were cultivated as a monolayer in DMEM (Dulbecco's modified Eagle medium) with glucose (4.5 g L⁻¹), L-glutamine, sodium pyruvate and phenol red, supplemented with 10 % (KB-3-1) and 15 % (KB-V1) fetal bovine serum. 50 µg mL⁻¹ gentamycin is added for the KB-V1 cells. The cells were maintained at 37 °C and 5.3 % CO₂/humidified air. KB-V1 cells were continuously selected during cultivation with vinblastine sulfate (150 nM). On the day before the test, the cells (70 % confluence) were detached with trypsin/ethylenediaminetetraacetic acid (EDTA) solution (0.05 %/0.02 % in DPBS) and plated in sterile 96-well plates in a density of 10,000 cells in 100 µL medium per well. The dilution series of the compounds were prepared from stock solutions in DMSO of concentrations of 1 mM or 10 mM. The stock solutions were diluted with culture medium (15 % FBS [KB-V1]; 10 % FBS [KB-3-1]) at least 50 times. Some culture medium was added to the wells to adjust the volume of the wells to the wanted dilution factor. The dilution prepared from stock solution was added to the wells. Each concentration was tested in six replicates. Dilution series were prepared by pipetting liquid from well to well. The control contained the same concentration of DMSO as the first dilution. After incubation for 72 h at 37 °C and 5.3 % CO₂/humidified air, 30 µL of an aqueous resazurin solution (175 µM) was added to each well. The cells were incubated at the same conditions for 6 h. Subsequently, the fluorescence was measured. The excitation was effected at a wavelength of 530 nm, whereas the emission was recorded at a wavelength of 588 nm. The IC₅₀ values were calculated as a sigmoidal dose response curve using GraphPad Prism 4.03. The IC₅₀ values equal the drug concentrations, at which vitality is 50 %.

Results of the biological tests in form of IC₅₀ values of compounds **C5, G5, C6, G6,** and **C7** are shown in Table 1:

**Table 1: IC50 values of compounds C5, G5, C6, G6, and C7**

| Compound | KB-3-1 cells | KB-V1 cells |
|---|---|---|
| **C5** | 2.2e⁻¹⁰ mol/L | 4.0e⁻⁷ mol/L |
| **C6** | 1.0e⁻⁹ mol/L | >5e⁻⁵ mol/L |
| **C7** | 3.8e⁻¹¹ mol/L | 2.1e⁻⁷ mol/L |
| **T7** | 2.0e⁻¹¹ mol/L | |
| **G5** | 4.2e⁻⁶ mol/L | |
| **G6** | 6.0e⁻⁵ mol/L | |
| **Y7** | 1.7e⁻¹¹ mol/L | |

## Claims

1. Cryptophycin compound of formula (I) 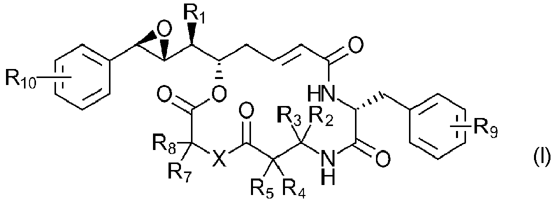 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, R₇ and R₈ represent, independently of each other, a hydrogen atom or a (C₁-C₆)alkyl group or a (C₁-C₆)alkylene-N(R₁₁)₂ group or a (C₁-C₆)alkylene-N⁺(R₁₁)₃ group or a (C₁-C₆)alkylene-OR₁₁ group or a (C₁-C₆)alkylene-SR₁₁ group or a (C₁-C₆)alkylene-S⁺(R₁₁)₂ group or a (C₁-C₆)alkylene-S(=O)R₁₁ group or a (C₁-C₆)alkylene-S⁺(=O)(R₁₁)₂ group or a (C₁-C₆)alkylene-S-SR₁₁ group or a (C₁-C₆)alkylene-COOR₁₁ group; or alternatively R₄ and R₅ or R₇ and R₈ form together with the carbon atom to which they are attached a (C₃-C₆)cycloalkyl or a (C₃-C₆)heterocycloalkyl group; with the proviso that one of R₄, R₅, R₆, R₇ and R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)alkylene-SR₁₁, (C₁-C₆)alkylene-S⁺(R₁₁)₂, (C₁-C₆)alkylene-S(=O)R₁₁, (C₁-C₆)alkylene-S⁺(=O)(R₁₁)₂, (C₁-C₆)alkyleneS-SR₁₁, and (C₁-C₆)alkylene-COOR₁₁, and the others represent a hydrogen atom or a (C₁-C₆)alkyl group, preferably a hydrogen or (C₁-C₄)alkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₁ independently represents a hydrogen atom or a (C₁-C₆)alk(en)yl group; and
each R₁₂ independently represents a hydrogen atom, a (C₁-C₆)alkyl group, a (C₃-C₆)cycloalkyl group or a (C₃-C₆)heterocycloalkyl group.

2. The compound of claim 1, wherein the compound is a compound of formula (1.1)

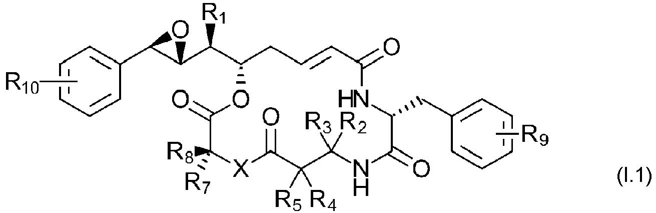

3. The compound of claim 1 or 2, wherein
(1) R₁ is methyl; and/or
(2) each of R₂ and R₃ represents a hydrogen atom or one of R₂ and R₃ represents a hydrogen atom and the other one represents a methyl group or R₂ and R₃ form together with the carbon atom to which they are attached a cyclopropyl group; and/or
(3) each of R₄ and R₅ represents a methyl or ethyl group, preferably methyl group, or one represents hydrogen and the other represents methyl or ethyl or both represent hydrogen or both combine to form together with the carbon atom to which they are attached a C₃-cycloalkyl group; and/or
(4) X is O or NR₆, wherein R₆ represents a hydrogen atom; and/or
(5) R₇ represents a hydrogen atom; and/or
(6) R₈ represents a group selected from (C₁-C₆)alkylene-N(R₁₁)₂, (C₁-C₆)alkylene-N⁺(R₁₁)₃, (C₁-C₆)alkylene-OR₁₁, (C₁-C₆)alkylene-SR₁₁, (C₁-C₆)alkylene-S⁺(R₁₁)₂, (C₁-C₆)alkyleneS(=O)R₁₁, (C₁-C₆)alkylene-S⁺(=O)(R₁₁)₂, (C₁-C₆)alkylene-S-SR₁₁, and (C₁-C₆)alkylene-COOR₁₁; and/or
(7) R₉ represents at least two substituents, one being selected from a methoxy group or a NH(C₁-C₆)alkyl, N((C₁-C₆)alkyl)₂ or -N⁺((C₁-C₆)alkyl)₃ group, preferably being in the 4-position, and the other being selected from a halogen, preferably chlorine, atom, preferably being in the 3-position; and/or
(8) R₁₀ represents a hydrogen atom.

4. The compound of any one of the preceding claims, wherein R₁ is methyl, each of R₂ and R₃ represents a hydrogen atom, R₆ represents a hydrogen atom, R₇ represents a hydrogen atom, R₉ represents two substituents selected from a methoxy group and a halogen, preferably chlorine, atom, more preferably 3-chloro-4-methoxy, and R₁₀ represents a hydrogen atom.

5. Cryptophycin derivative of formula (II) 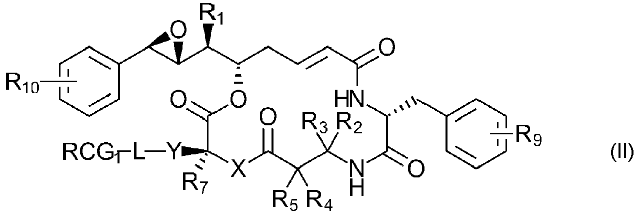 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, and R₇ represent, independently of each other, a hydrogen atom or a (C₁-C₆)alkyl group, preferably a hydrogen or (C₁-C₄)alkyl group; or alternatively R₄ and R₅ form together with the carbon atom to which they are attached a (C₃-C₆)cycloalkyl or a (C₃-C₆)heterocycloalkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-RCG₁ represents a group of formula: -(C₁-C₆)alkylene-NR₁₃-L-RCG₁, -(C₁-C₆)alkylene-N⁺(R₁₃)₂-L-RCG₁, -(C₁-C₆)alkylene-O-L-RCG₁, -(C₁-C₆)alkylene-S(=O)-L-RCG₁, -(C₁-C₆)alkylene-S⁺(=O)(R₁₃)-L-RCG₁, -(C₁-C₆)alkylene-S-L-RCG₁, -(C₁-C₆)alkylene-S⁺(R₁₃)-L-RCG₁, or -(C₁-C₆)alkylene-S-S-L-RCG₁;
R₁₃ represents a (C₁-C₆)alkyl group;
L represents a linker group; and
RCG₁ represents a reactive group.

6. The cryptophycin derivative of claim 5, wherein
(i) RCG₁ is alkenyl, such as ethenyl, alkynyl, such as ethynyl, -N₃ or N-maleinimide; and/or
(ii) the group RCG1-L-Y- is a group of formula (IV.1) or (IV.2):

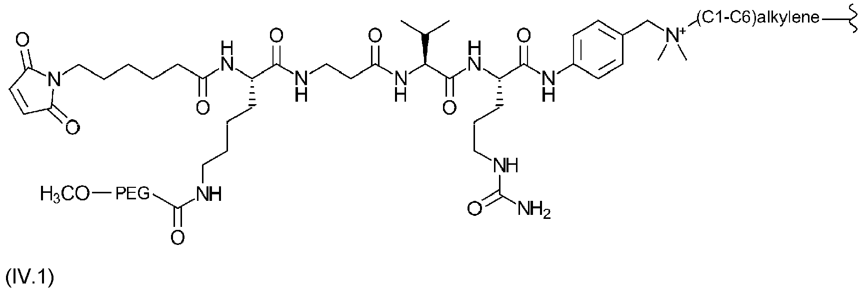

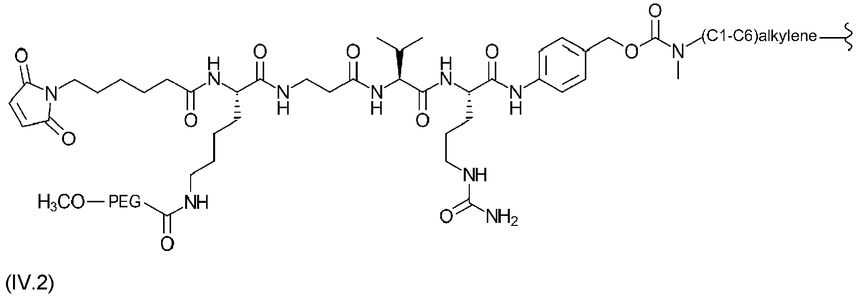

7. Cryptophycin conjugate of formula (III) 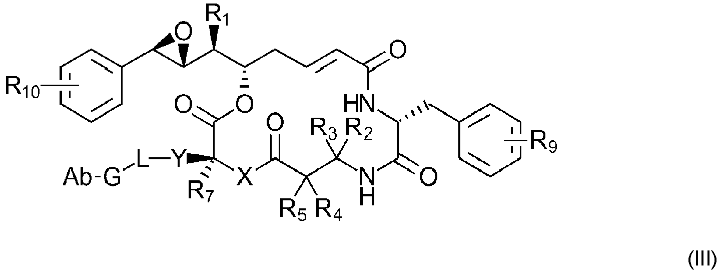 or stereoisomer or a pharmaceutically acceptable salt thereof,
wherein
X represents O or NR₆;
R₁ represents a (C₁-C₆)alkyl group, preferably methyl;
R₂ and R₃ represent, independently of each other, a hydrogen atom or a (C₁-C₆)alkyl group; or alternatively R₂ and R₃ form together with the carbon atom to which they are attached a (C₃-C₆)cycloalkyl or a (C₃-C₆)heterocycloalkyl group;
R₄, R₅, R₆, and R₇ represent, independently of each other, a hydrogen atom or a (C₁-C₆)alkyl group, preferably a hydrogen or (C₁-C₄)alkyl group; or alternatively R₄ and R₅ form together with the carbon atom to which they are attached a (C₃-C₆)cycloalkyl or a (C₃-C₆)heterocycloalkyl group;
R₉ represents one or more substituents of the phenyl nucleus selected, independently from each other, from: a hydrogen atom, -OH, (C₁-C₄)alkoxy, halogen, -N(R₁₂)₂, or -N⁺(R₁₂)₃;
R₁₀ represents one or more substituents of the phenyl nucleus selected, independently from each other, from: a hydrogen atom, -OH, (C₁-C₄)alkylene-OH, (C₁-C₄)alkoxy and (C₁-C₄)alkyl;
each R₁₂ independently represents a hydrogen atom, a (C₁-C₆)alkyl group, a (C₃-C₆)cycloalkyl group or a (C₃-C₆)heterocycloalkyl group;
Y-L-G-Ab represents a group of formula: -(C₁-C₆)alkylene-NR₁₃-L-G-Ab, -(C₁-C₆)alkylene-N⁺(R₁₃ )₂-L-G-Ab, -(C₁-C₆)alkylene-O-L-G-Ab, -(C₁-C₆)alkylene-S(=O)-L-G-Ab, -(C₁-C₆)alkyleneS⁺(=O)(R₁₃)-L-G-Ab, -(C₁-C₆)alkylene-S-L-G-Ab, -(C₁-C₆)alkylene-S⁺(R₁₃)-L-G-Ab, or -(C₁-C₆)alkylene-S-S-L-G-Ab;
R₁₃ represents a (C₁-C₆)alkyl group;
L represents a linker group;
G represents the product of reaction between RCG1, a reactive chemical group present at the end of the linker and RCG2, an orthogonal reactive chemical group present on Ab; and
Ab represents a peptide moiety, preferably an oligopeptide or polypeptide moiety, preferably an antibody or antibody-like molecule, or a small molecule, preferably folic acid or DUPA (Glu-urea-Glu), as homing device.

8. The cryptophycin conjugate of claim 7, wherein
(i) G is a residue of reactive coupling group RCG1 after the coupling reaction with RCG2 of Ab, preferably selected from: 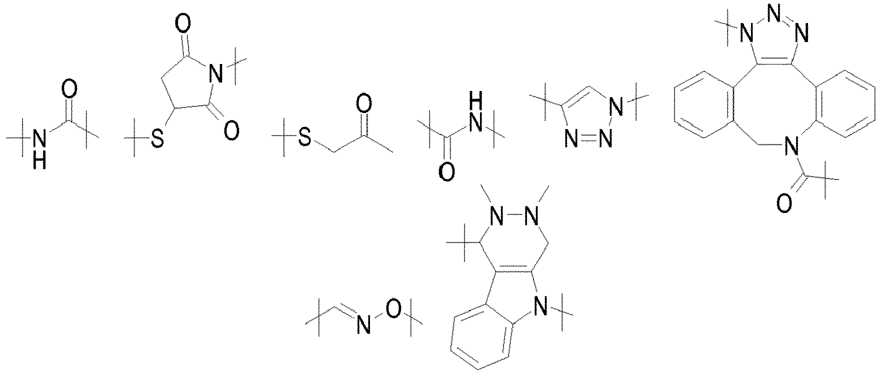 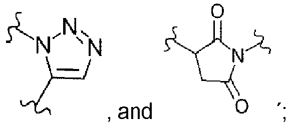 and/or
(ii) Ab-G-L-Y- is selected from the groups of formula (V.1) and (V.2):

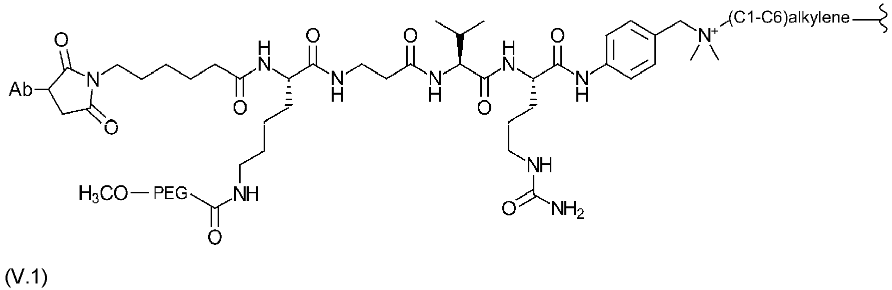

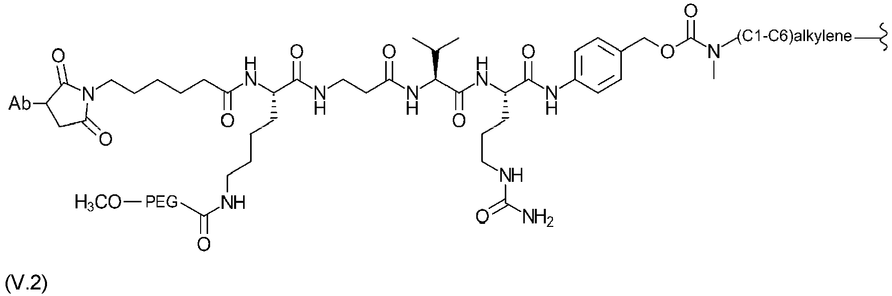

9. The cryptophycin derivative or conjugate of any one of claims 5-8, wherein L is a linker of the formula Str-Pep-Sp, wherein Str is a stretcher unit, Pep is a peptide or non-peptide linker unit, and Sp is a spacer unit.

10. The cryptophycin derivative or conjugate of claim 9, wherein Str is a -(C₁-C₁₀)alkylene-C(=O)-group, a -(CH₂)ₐ-(O-CH₂CH₂)ₙ-(CH2)_{b}-C(=O)- group, or a -(CH₂)ₐ-(CH₂CH₂-O)ₙ-(CH₂)_{b}-C(=O)-group, wherein a and b are independently 0 or an integer of 1 to 4, n is an integer of 1 to 20.

11. The cryptophycin derivative or conjugate of claim 9 or 10, wherein Sp is a spacer unit of formula

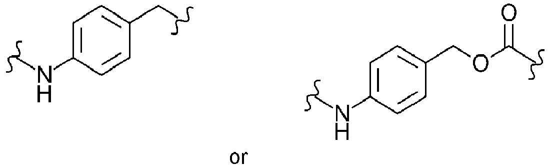

12. The cryptophycin derivative or conjugate of any one of claims 9 to 11, wherein Pep is a bond, a peptidyl moiety, or a non-peptide chemical moiety selected from the group consisting of: 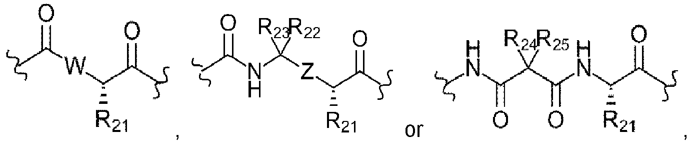 wherein
W is -NH-heterocycloalkylene- or heterocycloalkylene;
Z is bivalent heteroaryl, aryl, -C(=O)(C₁-C₆)alkylene, (C₂-C₆)alkenyl, (C₁-C₆)alkylenyl or (C₁-C₆)alkylene-NH-;
each R₂₁ is independently (C₁-C₁₀)alkyl, (C₂-C₁₀)alkenyl, (C₁-C₁₀)alkylNHC(=NH)NH₂, (C₁-C₁₀)alkylNHC(=O)NH₂ or (OCH₂CH₂)ₙ-OH or (CH₂CH₂O)ₙ-H with n = 3 to 50;
R₂₂ and R₂₃ are each independently H, (C₁-C₁₀)alkyl, (C₂-C₁₀)alkenyl, arylalkyl or heteroarylalkyl, or (OCH₂CH₂)ₙ-OH or (CH₂CH₂O)ₙ-H with n = 3 to 20, or R₂₂ and R₂₃ together with the carbon atom to which they are attached form (C₃-C₇)cycloalkyl; and
R₂₄ and R₂₅ are each independently (C₁-C₁₀)alkyl, (C₂-C₁₀)alkenyl, arylalkyl, or heteroarylalkyl, - CH₂-O-(C₁-C₁₀)alkyl, or R₂₂ and R₂₃ together with the carbon atom to which they are attached form (C3-C7)cycloalkyl;
wherein, if Pep is a peptidyl moiety, it optionally comprises or consists of a Val-Cit moiety, a Lys-β-Ala-Val-Cit moiety, a Phe-Lys moiety, or a Val-Ala moiety, wherein the side chain of lysine is optionally PEGylated.

13. The cryptophycin derivatives or conjugates of any one of claims 5 to 12 for use as a pharmaceutical.

14. The cryptophycin derivatives or conjugates of any one of claims 5 to 12 for use as a pharmaceutical for treating cancer.

15. Pharmaceutical composition comprising any one or more of the cryptophycin conjugates of claims 7-12; and
a pharmaceutically acceptable excipient, diluent, stabilizer and/or carrier.
